(19) Europäisches Patentamt
European Patent Office
Office européen des brevets

(11) EP 4 357 338 A1

(12) EUROPEAN PATENT APPLICATION
published in accordance with Art. 153(4) EPC

(43) Date of publication:
**24.04.2024 Bulletin 2024/17**

(21) Application number: **22824137.8**

(22) Date of filing: **10.06.2022**

(51) International Patent Classification (IPC):
***C07D 307/52*** (2006.01) ***C07D 307/79*** (2006.01)
***C07D 417/12*** (2006.01) ***C07D 405/12*** (2006.01)
***C07D 409/12*** (2006.01) ***C07D 333/20*** (2006.01)
***C07D 333/34*** (2006.01) ***C07D 207/335*** (2006.01)
***C07D 277/28*** (2006.01) ***C07D 231/12*** (2006.01)
***C07D 261/08*** (2006.01) ***C07D 233/64*** (2006.01)
***C07D 307/54*** (2006.01) ***C07D 409/14*** (2006.01)
***C07D 249/06*** (2006.01) ***A61P 29/00*** (2006.01)
***A61P 1/00*** (2006.01) ***A61P 1/04*** (2006.01)
***A61P 25/00*** (2006.01) ***A61K 31/341*** (2006.01)
***A61K 31/428*** (2006.01)

(52) Cooperative Patent Classification (CPC):
**A61K 31/341; A61K 31/343; A61K 31/381; A61K 31/40; A61K 31/402; A61K 31/415; A61K 31/4155; A61K 31/4164; A61K 31/4178; A61K 31/4192; A61K 31/42; A61K 31/426; A61K 31/428; A61K 31/443; A61P 1/00;** (Cont.)

(86) International application number:
**PCT/CN2022/098183**

(87) International publication number:
**WO 2022/262657 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.06.2021 CN 202110666168**

(71) Applicant: **Shanghai Leado Pharmatech Co. Ltd.**
**Shanghai 201114 (CN)**

(72) Inventors:
- **WANG, Youxin**
**Shanghai 201114 (CN)**
- **ZHANG, Lingling**
**Shanghai 201114 (CN)**
- **DING, Qiang**
**Shanghai 201114 (CN)**

(74) Representative: **Mewburn Ellis LLP**
**Aurora Building**
**Counterslip**
**Bristol BS1 6BX (GB)**

# (54) N-SUBSTITUTED PHENYLSULFONAMIDE COMPOUND AND USE THEREOF

(57) The present invention relates to an N-substituted phenylsulfonamide compound and a use thereof. Specifically, the present invention provides a compound represented by formula I, or an optical isomer thereof, or a racemate thereof, or a pharmaceutically acceptable salt thereof. The compound described in the present invention has an excellent inhibitory effect on transient receptor potential channel proteins, and has an excellent therapeutic effect on transient receptor potential channel protein-related diseases, such as inflammatory bowel disease, irritable bowel syndrome, pain, and inflammation.

EP 4 357 338 A1

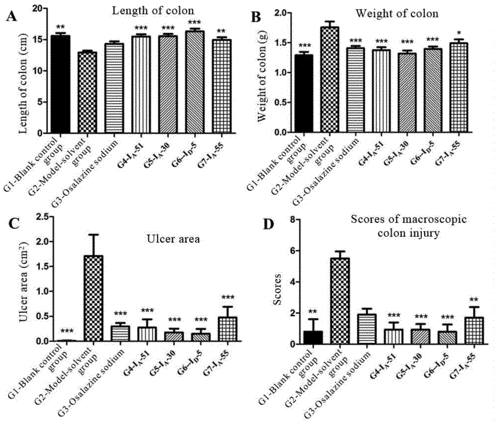


Figure 1

(52) Cooperative Patent Classification (CPC): (Cont.)
**A61P 1/04; A61P 25/00; A61P 25/04; A61P 29/00; C07D 207/335; C07D 231/12; C07D 233/64; C07D 249/06; C07D 261/08; C07D 277/28; C07D 307/52; C07D 307/54; C07D 307/79; C07D 333/20; C07D 333/34; C07D 405/12; C07D 409/12; C07D 409/14; C07D 417/12**

## Description

## TECHNICAL FIELD

The present invention relates to the fields of pharmaceutical chemistry and pharmacotherapy, specifically to N-substituted phenyl sulfonamide compounds and the use thereof.

## BACKGROUND OF THE INVENTION

Transient receptor potential ankyrin 1 (TRPA1), also known as ANKTM1, is a member of channel family of the transient receptor potential (TRP). TRPA1 is mainly distributed in the primary sensory neurons of the dorsal root nerve (DRG), trigeminal nerve (TG), and vagus nerve (VG), and is expressed in peptidergic neurons (rich in neuropeptides CGRP and SP, as well as neurotrophin receptor TrkA) and non peptidogenic neurons (co expressing purine receptor P2X3, Neuroturin, Artemin, G protein-coupled receptor of Mrg family, as well as $GFR_{\alpha 1}$ and $GFR_{\alpha 2}$ in the GDNF receptor family). From the distribution of human systems, TRPA1 is highly expressed in the gastrointestinal system, peripheral nervous system, respiratory system, and urinary system. When functional abnormality occur in these organ tissues, the abnormal expression and function of TRPA1 channels usually also occur synchronously.

Inflammatory bowel disease (IBD) is a chronic inflammatory disease that forms erosion or ulcer in the intestinal mucosa. The main types are divided into two categories: ulcerative colitis (UC) and Crohn's disease (CD). The former is mainly limited to the colon, while the latter can affect any segment of the gastrointestinal tract, with the most common symptoms being diarrhea and abdominal pain. At present, the pathogenesis of IBD is not entirely clear, and common treatment includes four categories: 5-aminosalicylic acids, glucocorticoids, antibiotics, and immunosuppressants. These drugs have drawbacks such as tolerance, limited applicability, and poor therapeutic effect. Research has shown that TRPA1 plays an important role in gastrointestinal regulation. TRPA1 immune response was detected in inhibitory motor neurons, descending interneurons, cholinergic and intrinsic sensory neurons in the cecum and colon of mice. More and more evidence suggests that TRPA1 is expressed in the intrinsic sensory neurons of the intestinal and submucosal nervous plexus, as well as in the surface epithelial cells of the colon mucosa. In the study of biopsy materials from active and inactive CD and UC patients, it was found that mRNA of TRPA1 was significantly upregulated. In addition, studies have also reported an increase in TRPA1 expression in the colon stenosis area of CD patients, including surgery and endoscopic examination. In DNBS, TNBS, or DSS induced colitis *in vivo* models, administration of selective inhibitor of TRPA1, HC-030031, or knocking out the TRPA1 gene, significantly alleviated the symptom of colitis in animals. Therefore, inhibitor of TRPA1 can be used for the treatment of IBD.

In the animal models study of irritable bowel syndrome (IBS), pancreatitis, and gastric mucosal injury, etc, it was found that the expression of TRPA1 was also significantly upregulated. In rodent model, TRPA1 and TRPV1 together promoted stress-induced visceral hyperalgesia, also known as irritable bowel syndrome. In the acute pancreatitis model, TRPA1 and TRPV1 have a synergistic effect, jointly regulating the transition from acute inflammation and hyperalgesia phenotype to chronic inflammation and hyperalgesia phenotype. In the rat model of acute gastric mucosal injury, the TRPA1 antagonist HC-030031 also showed positive result.

Visceral pain, as a major visceral sensation, is often caused by stimuli of the internal organs such as mechanical traction, spasms, ischemia, or inflammation. Through different animal models of visceral hypersensitivity, such as colitis, colorectal distension, or stress, it has been confirmed that TRPA1 is involved in the regulation of visceral hypersensitivity. Neurogenic pain is a pain syndrome caused by damage or disease of the central or peripheral nervous system, mainly manifested as hyperalgesia, abnormal hyperalgesia, and spontaneous pain. Unlike inflammatory pain, neurogenic pain is not related to the key link of inflammation vascular response, but depends on the damage and dysfunction of the nervous system, often caused by damage of peripheral nerves. In recent years, more and more studies have shown that TRPA1 channel plays an important role in different neurogenic pain, such as diabetic neuropathy and neuropathy caused by chemotherapy drugs. Recent studies have also shown that TRPA1 plays a mediating role in pain such as toothache and migraine, and the administration of TRPA1 antagonist can significantly alleviate the onset of pain symptom.

Inflammation is a defense response of living tissue with vascular system to injury factors, and the stimulation of inflammatory mediators such as prostaglandin, 5-hydroxytryptamine, and bradykinin is the main cause of local pain in inflammation. Inflammatory pain is a common problem of certain chronic diseases, and there is still a lack of effective treatment in clinical practice. Animal experimental study has shown that TRPA1 is involved in inflammatory response and plays an important role in inflammatory pain. By using TRA1 specific blocker, the inflammatory pain response in experimental rats can be significantly reduced. The pathogenesis of asthma and cough is becoming increasingly clear with the deepening of research. Based on current research, TRPA1 plays an important role in the occurrence of asthma and cough. Compounds that induce asthma and cough, whether endogenous cytokines in cells or exogenous factors, can activate TRPA1. Antagonist of TRPA1 can alleviate asthma symptom and block airway hyperresponsiveness.

Due to the widespread distribution and expression of TRPA1 in the human system, importance of its function

is self-evident. In addition to the above physiological functions involved by TRPA1, the development of the reported indications of TRPA1 inhibitor also involves chronic obstructive pulmonary disease, antitussive, antipruritic, allergic rhinitis, ear disease, anti-diabetes, urinary incontinence, etc. TRPA1 is a newly confirmed therapeutic target for inflammatory bowel disease, irritable bowel syndrome, pain, and inflammation, and there is currently no marketed drug targeting this target.

Inflammatory bowel disease, irritable bowel syndrome, and pain belong to refractory diseases. Therefore, there is an urgent need in this field to develop a therapeutic drug targeting TRPA1 target in order to improve the therapeutic effect of the disease.

## SUMMARY OF THE INVENTION

The purpose of the present invention is to provide a structurally novel compound targeting TRPA1 and the use thereof.

The first aspect of the present invention provides a compound of formula I, or an optical isomer thereof, or a racemate thereof, or a pharmaceutically acceptable salt thereof, or a prodrug thereof;

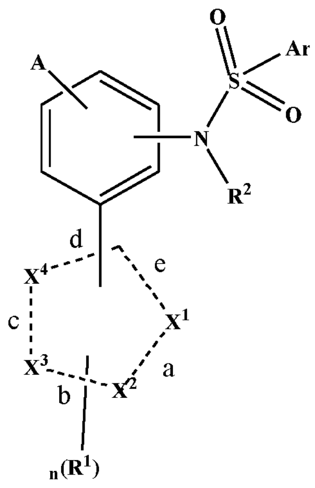

I

wherein,

Ar is substituted or unsubstituted C6-C12 aryl, substituted or unsubstituted 3-12 membered heteroaryl, 3-12 membered heterocycloalkyl ring fused C6-C12 aryl, substituted or unsubstituted C6-C12 aryl-substituted or unsubstituted C1-C8 alkyl-, or substituted or unsubstituted 3-12 membered heteroaryl-substituted or unsubstituted C1-C8 alkyl-;
$X^1$, $X^2$, $X^3$, and $X^4$ are each independently C, O, S, or N;
"------" labeled as a, b, c, d, and e are single bond or double bond;
$R^1$ is hydrogen, substituted or unsubstituted C1-C6 alkyl, substituted or unsubstituted C3-C7 cycloalkyl, or halogen;
$R^2$ is hydrogen, substituted or unsubstituted C1-C10 alkyl, or substituted or unsubstituted C3-C10 cycloalkyl;
A is

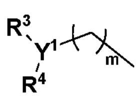 , 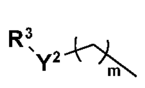 or, 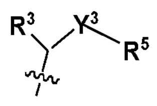 , 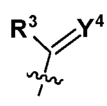 , 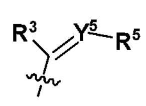 ,

substituted or unsubstituted C2-C6 ester group, substituted or unsubstituted C2-C6 carboxyl, substituted or unsubstituted C2-C6 amido, substituted or unsubstituted 3-8 membered heterocycloalkyl, or substituted or unsubstituted $H_2N\text{-}HN\text{-}C(O)\text{-}$;
m is 0, 1, 2, or 3;
$Y^1$ is N;
$Y^2$ is O or S;
$Y^3$ is NH, O, or S;

$Y^4$ is O or S;
$Y^5$ is N;
$R^3$ and $R^4$ are each independently hydrogen, substituted or unsubstituted C1-C6 alkyl, substituted or unsubstituted C3-C8 cycloalkyl, substituted or unsubstituted C6-C12 aryl, substituted or unsubstituted C6-C12 aryl-substituted or unsubstituted C1-C3 alkyl-, substituted or unsubstituted C2-C6 acyl, or $R^3$ and $R^4$ with adjacent $Y^1$ to which they are attached form a substituted or unsubstituted 3-8 membered heterocycloalkyl;
$R^5$ is hydrogen, substituted or unsubstituted C1-C6 alkyl, hydroxyl, thiol, or substituted or unsubstituted C1-C6 alkoxy;
n is 0, 1, 2, 3, 4, or 5;
wherein, any one of "substituted" refers to one or more (preferably 1, 2, 3, 4, 5, or 6) hydrogen atoms on the ring or group being substituted by substituents selected from the group consisting of C1-C8 alkyl, C3-C8 cycloalkyl, C1-C8 haloalkyl, C3-C8 halocycloalkyl, halogen, nitro, -CN, hydroxyl, thiol, amino, C1-C4 carboxyl, C2-C4 ester group, C2-C4 amido, C1-C8 alkoxy, C1-C8 alkylthio, C1-C8 haloalkoxy, C1-C8 haloalkylthio, C6-C12 aryl, 5-10 membered heteroaryl, and 5-10 membered heterocycloalkyl; and
the heterocycles in the heteroaryl, heterocycloalkyl ring, and heterocycloalkyl each independently contain 1-4 (preferably 1, 2, 3, or 4) heteroatoms selected from N, O, and S.

In another preferred embodiment, Ar is substituted or unsubstituted C6-C10 aryl, substituted or unsubstituted 3-10 membered heteroaryl, 3-10 membered heterocycloalkyl ring fused C6-C10 aryl, substituted or unsubstituted C6-C10 aryl-substituted or unsubstituted C1-C6 alkyl-, or substituted or unsubstituted 3-10 membered heteroaryl-substituted or unsubstituted C1-C6 alkyl-.
In another preferred embodiment, Ar is substituted or unsubstituted C6-C10 aryl, substituted or unsubstituted 3-8 membered heteroaryl, 3-8 membered heterocycloalkyl ring fused C6-C10 aryl, substituted or unsubstituted C6-C10 aryl-substituted or unsubstituted C1-C4 alkyl-, or substituted or unsubstituted 3-8 membered heteroaryl-substituted or unsubstituted C1-C4 alkyl-.
In another preferred embodiment, Ar is substituted or unsubstituted C6-C8 aryl, substituted or unsubstituted 5-8 membered heteroaryl, 5-8 membered heterocycloalkyl ring fused C6-C8 aryl, substituted or unsubstituted C6-C8 aryl-substituted or unsubstituted C1-C3 alkyl-, or substituted or unsubstituted 5-8 membered heteroaryl-substituted or unsubstituted C1-C3 alkyl-.
In another preferred embodiment, Ar is substituted or unsubstituted C6-C10 aryl, substituted or unsubstituted 3-8 membered (preferably 5-8 membered) heteroaryl.
In another preferred embodiment, Ar is phenyl, halophenyl, methoxyphenyl, trifluoromethoxyphenyl, trifluoromethylphenyl, methylphenyl, naphthyl, dihydrofuran fused phenyl, benzothiazolyl, pyridinyl, halopyridinyl, imidazolyl, methylimidazolyl, thienyl, halo thienyl, or benzyl.
In another preferred embodiment, Ar is phenyl, or thienyl.
In another preferred embodiment, the halophenyl is monohalogenated phenyl or dihalogenated phenyl.
In another preferred embodiment, dihydrofuran fused phenyl is

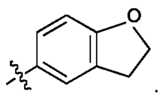

In another preferred embodiment, the substituents in Ar is selected from the group consisting of halogen (preferably fluorine), C1-C4 alkyl, C1-C4 haloalkyl, C1-C4 alkoxy, and C1-C4 haloalkoxy.
In another preferred embodiment, $R^1$ is hydrogen, substituted or unsubstituted C1-C4 alkyl, substituted or unsubstituted C3-C7 cycloalkyl, or halogen.
In another preferred embodiment, $R^1$ is hydrogen, substituted or unsubstituted C1-C2 alkyl, substituted or unsubstituted C3-C7 cycloalkyl, or halogen.
In another preferred embodiment, $R^1$ is hydrogen, or substituted or unsubstituted C1-C4 alkyl, preferably hydrogen.
In another preferred embodiment, $R^1$ is hydrogen, or methyl.
In another preferred embodiment, $R^2$ is hydrogen, substituted or unsubstituted C1-C6 alkyl, or substituted or unsubstituted C3-C7 cycloalkyl.
In another preferred embodiment, $R^2$ is hydrogen, or substituted or unsubstituted C1-C4 alkyl, preferably hydrogen.
In another preferred embodiment, $R^2$ is hydrogen, or methyl.
In another preferred embodiment, A is

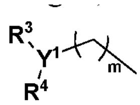, 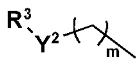 or, 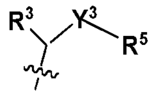, 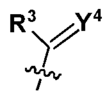,

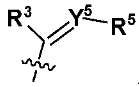,

substituted or unsubstituted C2-C4 ester group, substituted or unsubstituted C1-C4 carboxyl, substituted or unsubstituted C1-C4 amido, or substituted or unsubstituted $H_2N$-HN-C(O)-.

In another preferred embodiment, A is

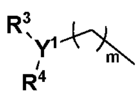, 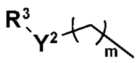 or, 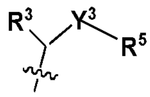, 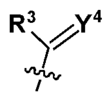,

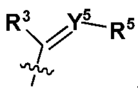,

methyl ester group, ethyl ester group, formyl, acetamido, formamido, or $H_2N$-HN-C (O)-.

In another preferred embodiment, A is

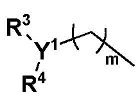 or 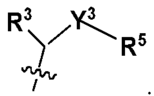.

In another preferred embodiment, A is

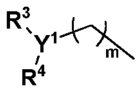.

In another preferred embodiment, $Y^3$ is N.

In another preferred embodiment, m is 0, 1, or 2.

In another preferred embodiment, $R^3$ and $R^4$ are each independently hydrogen, substituted or unsubstituted C1-C4 alkyl, substituted or unsubstituted C3-C6 cycloalkyl, substituted or unsubstituted C2-C4 acyl, or $R^3$ and $R^4$ with adjacent $Y^1$ to which they are attached form substituted or unsubstituted 3-6 membered heterocycloalkyl.

In another preferred embodiment, $R^3$ and $R^4$ are each independently hydrogen, substituted or unsubstituted C1-C2 alkyl, substituted or unsubstituted C3-C6 cycloalkyl, substituted or unsubstituted C2-C4 acyl, or $R^3$ and $R^4$ with adjacent $Y^1$ to which they are attached form substituted or unsubstituted 3-6 membered heterocycloalkyl.

In another preferred embodiment, $R^3$ and $R^4$ are each independently hydrogen, methyl, trifluoromethyl-methyl-, ethyl, cyclopropyl, cyclobutyl, or acetyl, or $R^3$ and $R^4$ with adjacent $Y^1$ to which they are attached form substituted or unsubstituted azetidinyl.

In another preferred embodiment, when A is

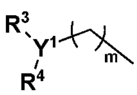,

$R^3$ is hydrogen, substituted or unsubstituted C1-C6 alkyl, substituted or unsubstituted C3-C6 cycloalkyl, substituted or unsubstituted C2-C4 acyl, $R^4$ is hydrogen, preferably $R^3$ and $R^4$ are both hydrogen.

In another preferred embodiment, when A is

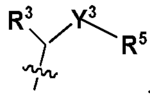

,

$R^3$ is hydrogen, substituted or unsubstituted C1-C6 alkyl, substituted or unsubstituted C3-C6 cycloalkyl, substituted or unsubstituted C6-C12 aryl, or substituted or unsubstituted C6-C12 aryl-substituted or unsubstituted C1-C3 alkyl-, $R^5$ is hydrogen, or substituted or unsubstituted C1-C6 alkyl.

In another preferred embodiment, when A is

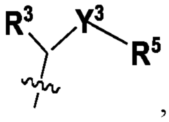

,

$R^3$ is hydrogen, substituted or unsubstituted C1-C6 alkyl, substituted or unsubstituted C3-C6 cycloalkyl, $R^5$ is hydrogen, or substituted or unsubstituted C1-C6 alkyl.

In another preferred embodiment, $R^5$ is hydrogen, hydroxyl, thiol, or substituted or unsubstituted C1-C4 alkoxy.

In another preferred embodiment, $R^5$ is hydrogen, hydroxyl, thiol, or methoxy.

In another preferred embodiment, $R^3$ and $R^4$ with adjacent $Y^1$ to which they are attached form haloazetidinyl

In another preferred embodiment, $X^1$, $X^2$, $X^3$, and $X^4$ are each independently C, O, S, or N.

In another preferred embodiment, $X^1$ is C, O, S, or N.

In another preferred embodiment, $X^2$ is C, O, S, or N.

In another preferred embodiment, $X^3$ is C, O, S, or N.

In another preferred embodiment, $X^4$ is C, O, S, or N.

In another preferred embodiment, one or more (2 or 3) of $X^1$, $X^2$, $X^3$, and $X^4$ is O, S, or N, and the rest are C.

In another preferred embodiment, "_____" labeled as a, b, c, d, and e are each independently single bond or double bond.

In another preferred embodiment, "------" labeled as a is single bond or double bond.

In another preferred embodiment, "------" labeled as b is single bond or double bond.

In another preferred embodiment, "------" labeled as c is single bond or double bond.

In another preferred embodiment, "------" labeled as d is single bond or double bond.

In another preferred embodiment, "------" labeled as e is single bond or double bond.

In another preferred embodiment, $X^1$, $X^2$, $X^3$, $X^4$ and "------" labeled as a, b, c, d, and e form an aromatic ring or a heteroaromatic ring.

In another preferred embodiment, the heterocycles in the heteroaromatic ring each independently contains 1-4 (preferably 1, 2, 3, or 4) heteroatoms selected from N, O, and S.

In another preferred embodiment, $X^1$, $X^2$, $X^3$, $X^4$ and "------" labeled as a, b, c, d, and e form furan ring, thiophene ring, pyrrole ring, thiazole ring, pyrazole ring, isoxazole ring, oxazole ring, imidazole ring, or triazole ring.

In another preferred embodiment, n is 0, 1, 2, 3, or 4.

In another preferred embodiment, any one of "substituted" refers to one or more (preferably 1, 2, 3, 4, 5, or 6) hydrogen atoms on the ring or group being substituted by substituents selected from the group consisting of C1-C6 alkyl, C3-C8 cycloalkyl, C1-C6 haloalkyl, C3-C8 halocycloalkyl, halogen, nitro, -CN, hydroxyl, thiol, amino, C1-C4 carboxyl, C2-C4 ester group, C2-C4 amido, C1-C6 alkoxy, C1-C6 alkylthio, C1-C6 haloalkoxy, C1-C6 haloalkylthio, C6-C12 aryl, 5-10 membered heteroaryl, and 5-10 membered heterocycloalkyl.

In another preferred embodiment, any one of "substituted" refers to one or more (preferably 1, 2, 3, 4, 5, or 6) hydrogen atoms on the ring or group being substituted by substituents selected from the group consisting of C1-C4 alkyl, C3-C8 cycloalkyl, C1-C4 haloalkyl, C3-C8 halocycloalkyl, halogen, nitro, -CN, hydroxyl, thiol, amino, C1-C4 carboxyl, C2-C4 ester group, C2-C4 amido, C1-C4 alkoxy, C1-C4 alkylthio, C1-C4 haloalkoxy, C1-C4 haloalkylthio, C6-C12 aryl, 5-10 membered heteroaryl, and 5-10 membered heterocycloalkyl.

In another preferred embodiment, the heterocycles in the heteroaryl, heterocycloalkyl ring, and heterocycloalkyl each independently contain 1-4 (preferably 1, 2, 3, or 4) heteroatoms selected from N, O, and S.

In another preferred embodiment, the compound has the structure of formula **I-1**:

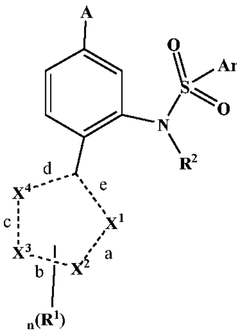

**I-1**.

In another preferred embodiment, the compound has the structure of formula **I-2**:

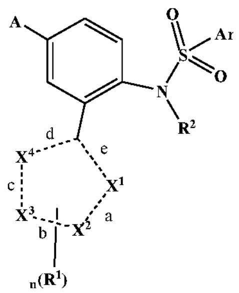

**I-2**.

In another preferred embodiment, the compound has the structure of formula **I-3**:

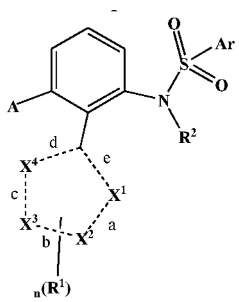

**I-3**.

In another preferred embodiment, the compound has the structure of formula **I-4**:

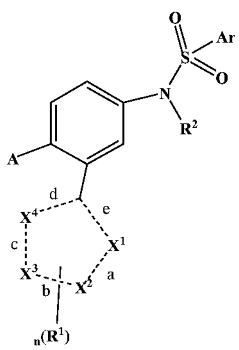

**I-4**.

In another preferred embodiment, the compound has the structure of formula **I-5**:

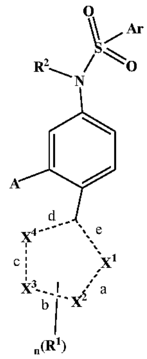

**I-5**.

In another preferred embodiment, the compound has the structure of formula **I-6**:

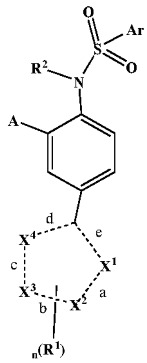

**I-6**.

In another preferred embodiment, the compound has the structure of formula **I-7**:

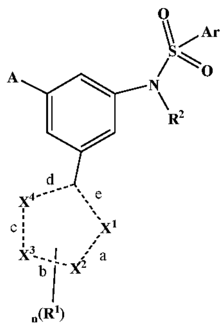

**I-7**.

In another preferred embodiment, the compound has the structure of formula **I-8**:

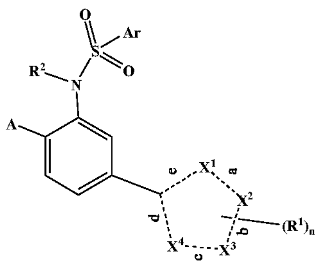

**I-8**.

In another preferred embodiment, the compound has the structure of formula Z:

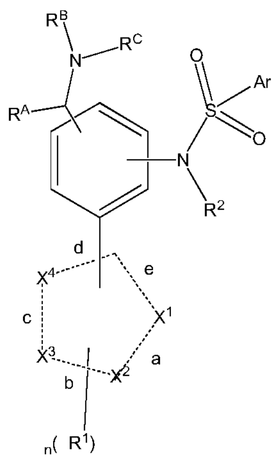

Z

wherein, $R^1$, $R^2$, $X^1$, $X^2$, $X^3$, $X^4$, Ar, a, b, c, d, e, and n are described above; $R^A$, $R^B$, and $R^C$ are each independently hydrogen, substituted or unsubstituted C1-C6 alkyl.

In another preferred embodiment, $R^1$, $R^2$, $X^1$, $X^2$, $X^3$, $X^4$, Ar, A, a, b, c, d, e, and n are each independently corresponding groups in the compounds prepared in the examples.

In another preferred embodiment, the compound is selected from the group consisting of:

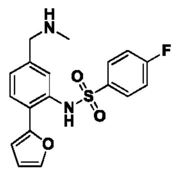 , 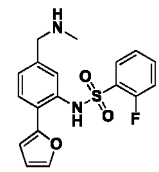 , 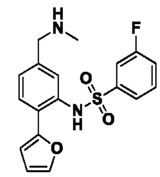 , 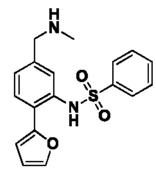 , 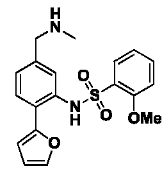 ,

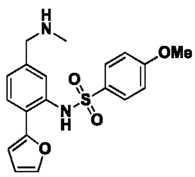 , 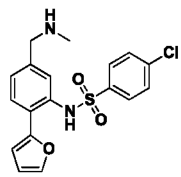 , 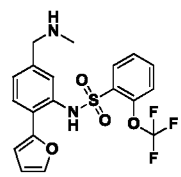 , 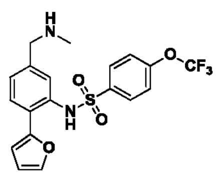 , 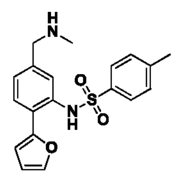 ,

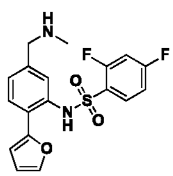 , 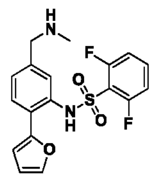 , 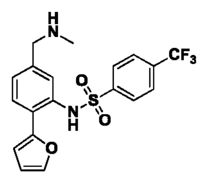 , 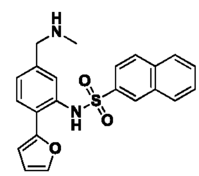 , 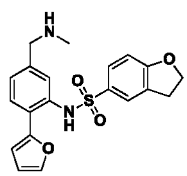 ,

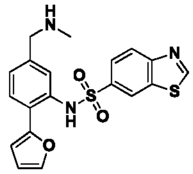 , 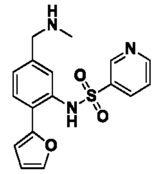 , 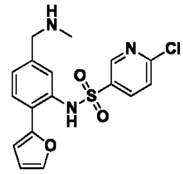 , 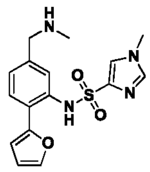 , 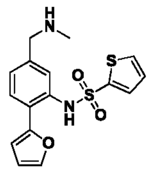 ,

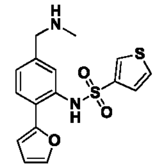 , 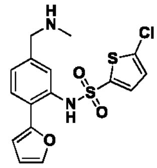 , 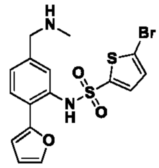 , 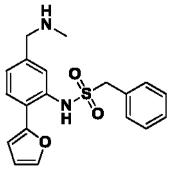 , 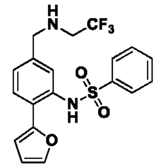 ,

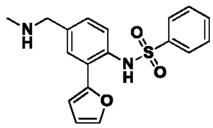 , 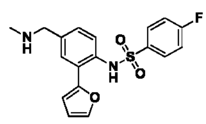 , 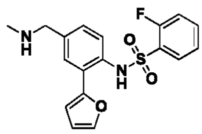 , 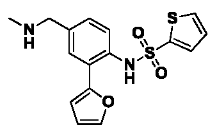 , 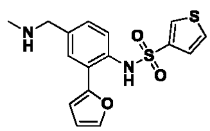 ,

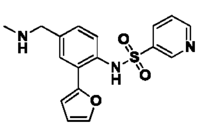 , 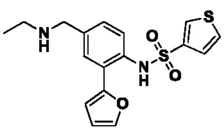 , 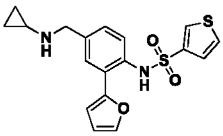 , 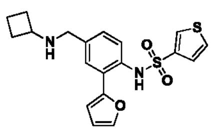 , 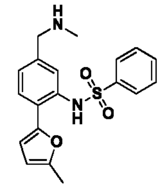 ,

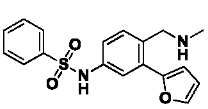 , 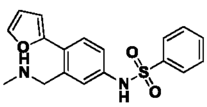 , 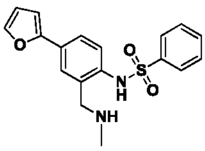 , 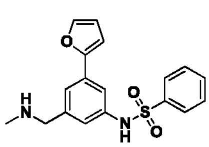 , 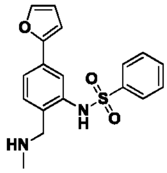 ,

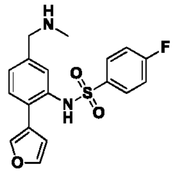 , 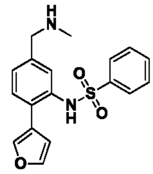 , 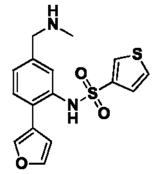 , 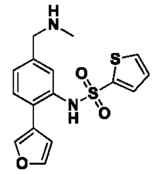 , 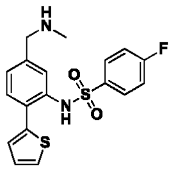 ,

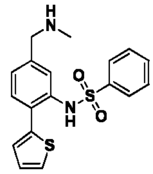 , 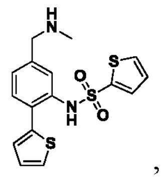 , 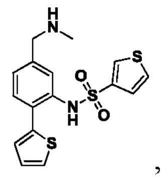 , 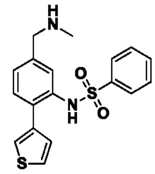 , 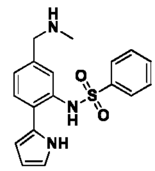 ,

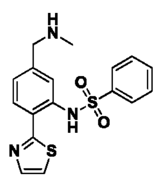 , 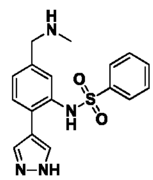 , 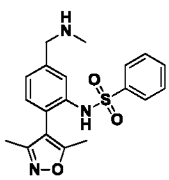 , 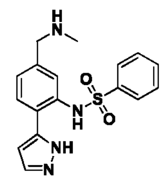 , 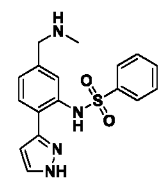 ,

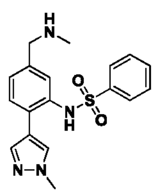 , 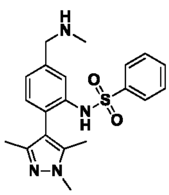 , 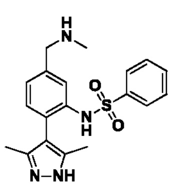 , 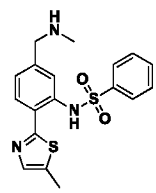 , 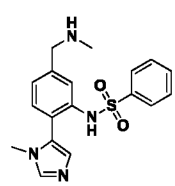 ,

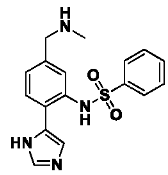 , 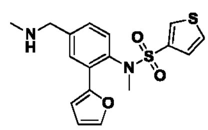 , 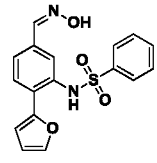 , 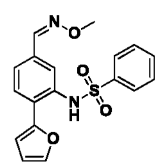 , 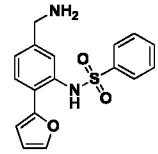 ,

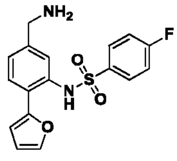 , 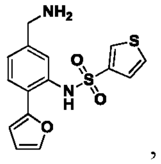 , 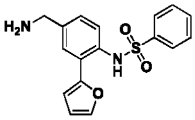 , 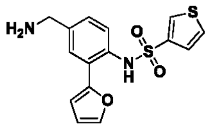 , 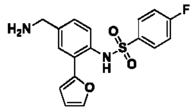 ,

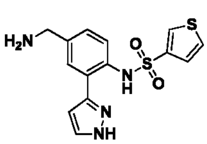 , 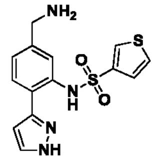 , 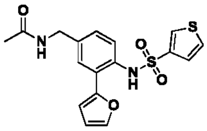 , 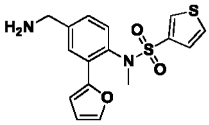 , 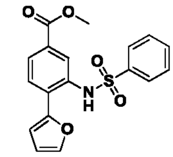 ,

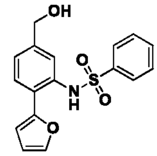 , 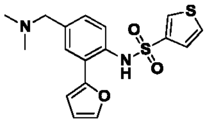 , 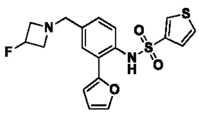 , 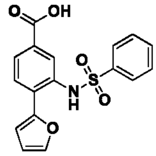 , 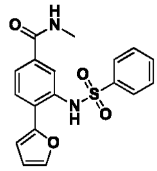 ,

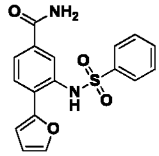 , 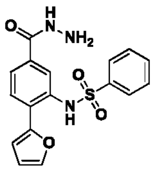 , 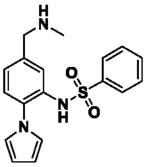 , 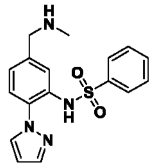 , 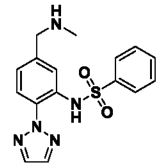 ,

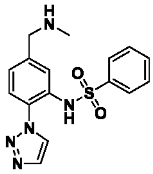 , 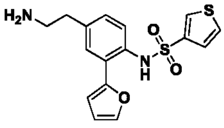 , 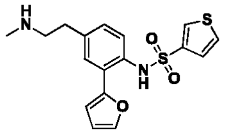 , 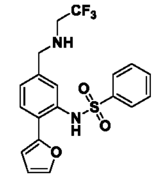 , 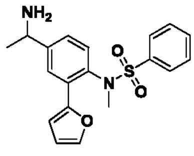

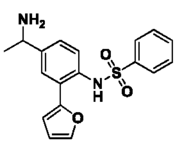 , 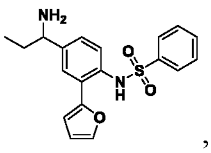 , 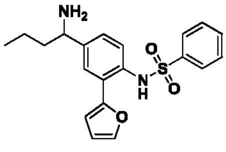 , 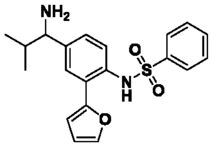 , 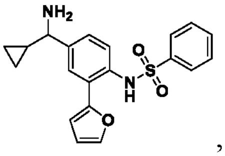 ,

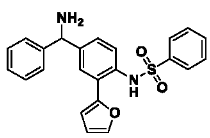 , 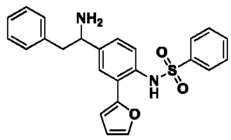 , 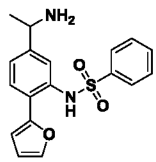 , 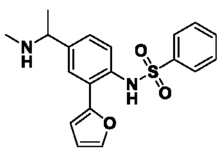 , 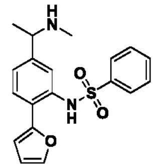 ,

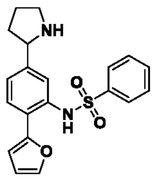

.

The second aspect of the present invention provides a pharmaceutical composition comprising a compound of formula I as described in the first aspect of the present invention, or an optical isomer thereof, or a racemate thereof,or a pharmaceutically acceptable salt thereof, or a prodrug thereof; and pharmaceutically acceptable carriers.

In another preferred embodiment, the dosage form of the pharmaceutical composition is oral formulation, injectable formulation, or topical formulation.

In another preferred embodiment, the dosage form of the pharmaceutical composition is solid formulation, liquid formulation, or semi-solid formulation.

In another preferred embodiment, the dosage form of the pharmaceutical composition is tablet, injection, infusion solution, paste, gel, solution, microsphere or film.

The third aspect of the present invention provides a method for preparing a compound of formula I as described in the first aspect of the present invention, or an optical isomer thereof, or a racemate thereof, or a pharmaceutically acceptable salt thereof, or a prodrug thereof, wherein the method comprises:

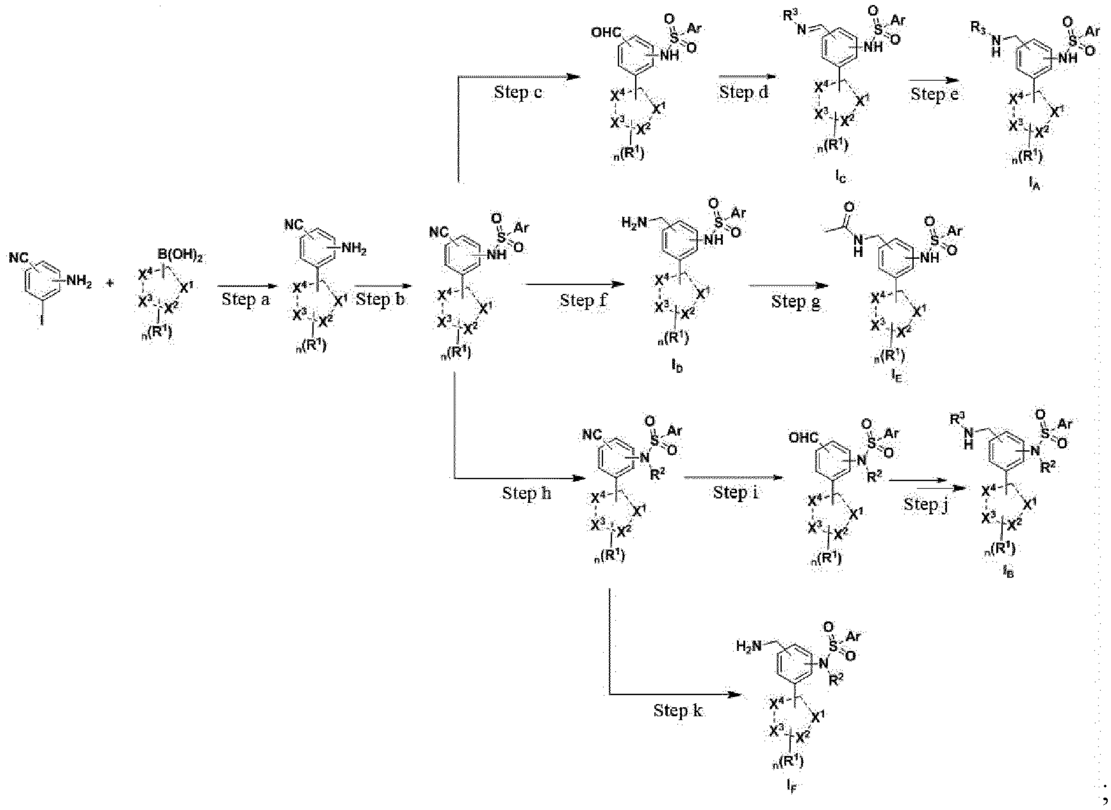


;

wherein, the definitions of $X^1$, $X^2$, $X^3$, $X^4$, $R^1$, $R^2$, n, Ar, and $R^3$ are defined as above. In another preferred embodiment, the method comprises:

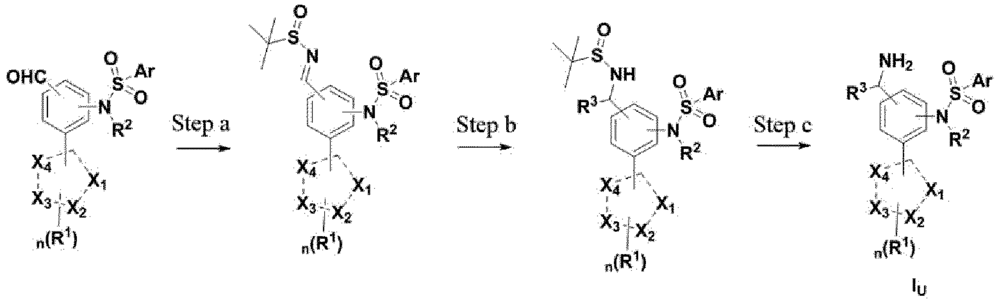


$I_U$ ;

wherein, the definitions of $X^1$, $X^2$, $X^3$, $X^4$, $R^1$, $R^2$, n, Ar, and $R^3$ are defined as above. In another preferred embodiment, the method comprises:

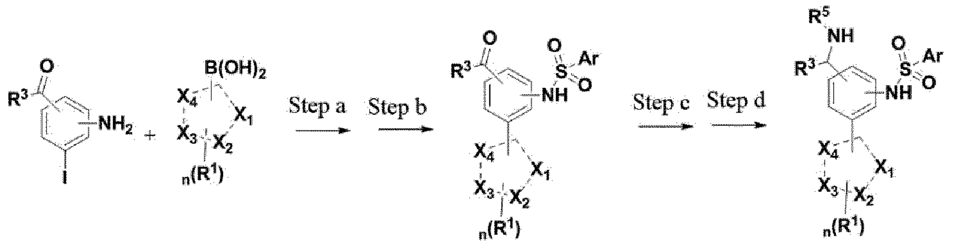


$I_V$ ;

wherein, the definitions of $X^1$, $X^2$, $X^3$, $X^4$, $R^1$, $R^2$, n, Ar, and $R^3$ are defined as above. In another preferred embodiment, the method comprises:

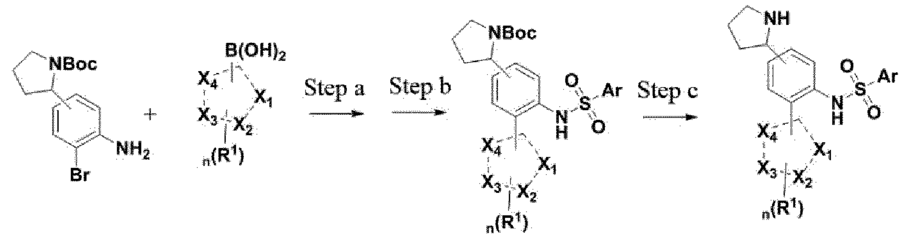


$I_W$ ;

wherein, the definitions of $X^1$, $X^2$, $X^3$, $X^4$, $R^1$, $R^2$, n, Ar, and $R^3$ are defined as above. In another preferred embodiment, the method comprises:

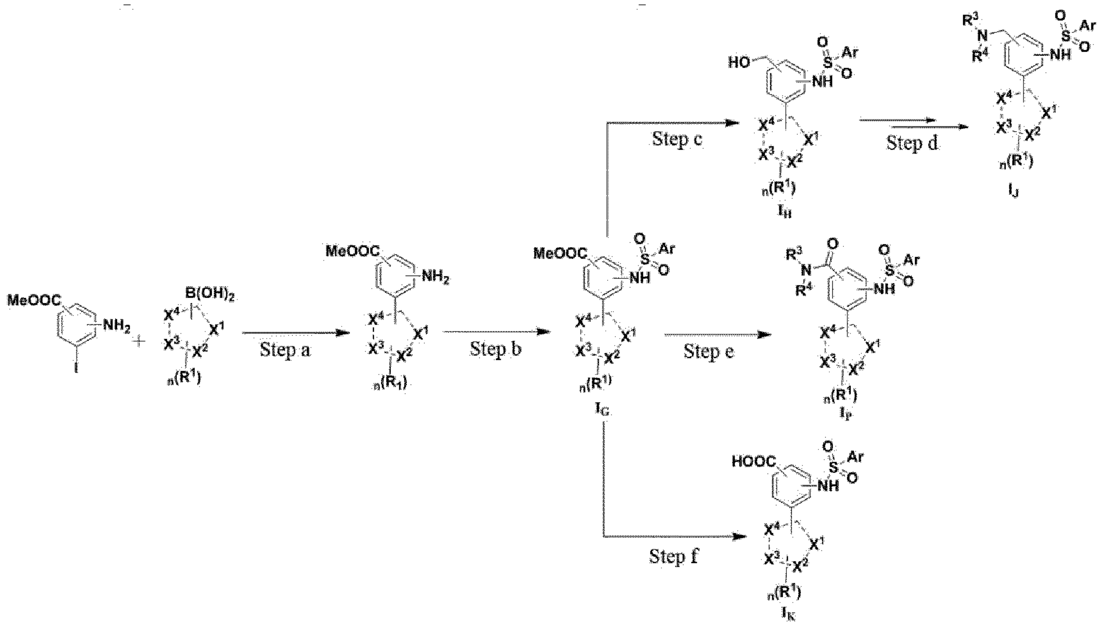


wherein, the definitions of $X^1$, $X^2$, $X^3$, $X^4$, $R^1$, n, Ar, $R^3$, and $R^4$ are defined as above. In another preferred embodiment, the method comprises:

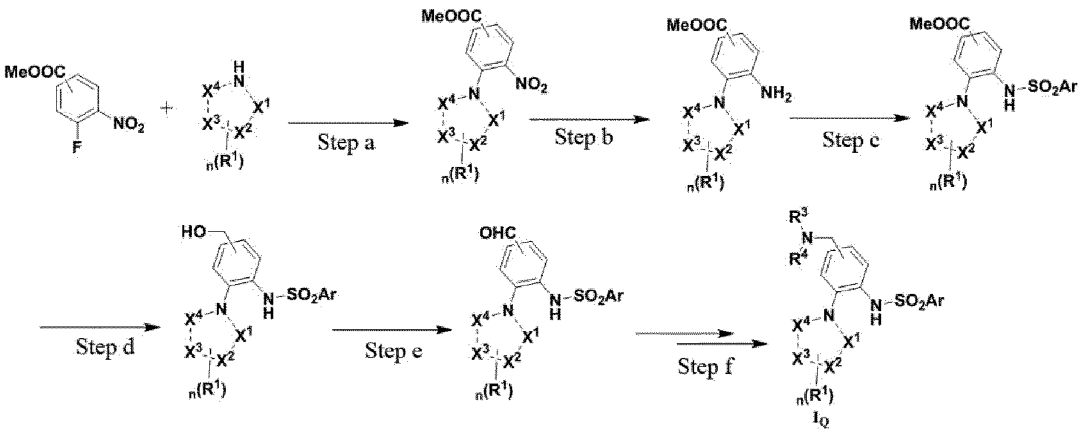


wherein, the definitions of $X^1$, $X^2$, $X^3$, $X^4$, $R^1$, n, Ar, $R^3$, and $R^4$ are defined as above. In another preferred embodiment, the method comprises:

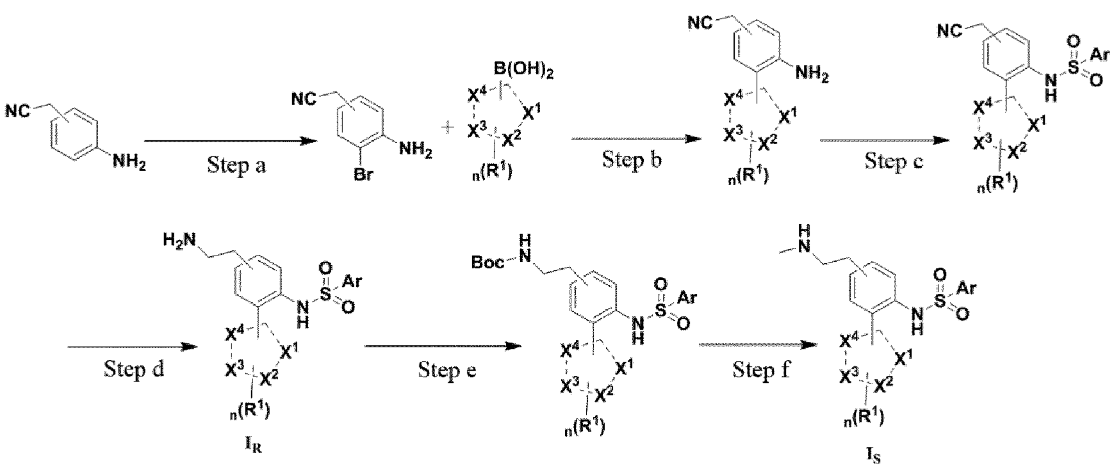

wherein, the definitions of $X^1$, $X^2$, $X^3$, $X^4$, $R^1$, n, and Ar are defined as above.

The fourth aspect of the present invention provides a use of a compound of formula I as described in the first aspect of the present invention, or an optical isomer thereof, or a racemate thereof, or a pharmaceutically acceptable salt thereof, or a prodrug thereof, or a pharmaceutical composition as described in the second aspect of the present invention in (a) preparing an inhibitor of transient receptor potential channel protein TRPA1; and/or (b) preparing drugs for preventing and/or treating disease related to transient receptor potential channel protein TRPA1.

In another preferred embodiment, the disease related to transient receptor potential channel protein TRPA1 is selected from the group consisting of inflammatory bowel disease, irritable bowel syndrome, pain, inflammation, and the combination thereof.

In another preferred embodiment, the inflammatory bowel disease includes Crohn's disease and/or ulcerative colitis.

In another preferred embodiment, the pain includes visceral pain, acute inflammatory pain, chronic inflammatory pain, neurogenic pain, fibromyalgia, headache, neuralgia, and pain caused by cancer.

In another preferred embodiment, the prevention and/or treatment of inflammatory bowel disease comprises one or more manners selected from the group consisting of:

(i) improving ulcer;
(ii) improving intestinal infarction;
(iii) improving intestinal adhesion;
(iv) increasing thickness of intestinal wall; and/or
(v) reducing the level of intestinal inflammatory factors.

In another preferred embodiment, the prevention and/or treatment of Crohn's disease and/or ulcerative colitis comprises one or more manners selected from the group consisting of:

(i) improving ulcer;
(ii) improving intestinal infarction;
(iii) improving intestinal adhesion;
(iv) increasing thickness of intestinal wall; and/or
(v) reducing the level of intestinal inflammatory factors.

In another preferred embodiment, the inflammatory factors are selected from the group consisting of TNF-$\alpha$, IL-10, and the combinations thereof.

The fifth aspect of the present invention provides a method for *in vitro* non-therapeutically and non-diagnostically inhibiting transient receptor potential channel protein activity, comprising the steps of contacting the transient receptor potential channel protein or cells expressing the protein with a compound of formula I as described in the first aspect of the present invention, or an optical isomer thereof, or a racemate thereof, or a pharmaceutically acceptable salt thereof, or a prodrug thereof, thereby inhibiting the activity of the transient receptor potential channel proteins.

The sixth aspect of the present invention provides a method for inhibiting transient receptor potential channel protein, or preventing and/or treating disease related to transient receptor potential channel protein TRPA1, comprising the steps of administering to a subject in need thereof a compound of formula I as described in the first aspect of the present invention, or an optical isomer thereof, or a racemate thereof, or a pharmaceutically acceptable salt thereof, or a prodrug thereof, or a pharmaceutical composition as described in the second aspect of the present invention.

The seventh aspect of the present invention provides a compound shown in one of the following formulas II-1 to II-6:

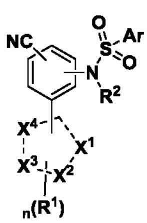
II-1

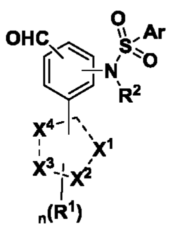
II-2

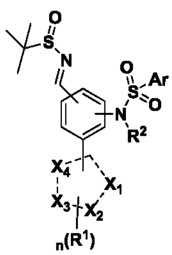
II-3

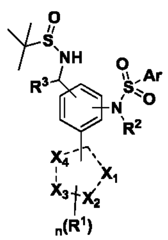
II-4

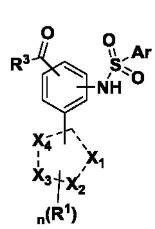
II-5

, or

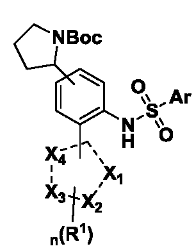
II-6

wherein,

Ar is substituted or unsubstituted C6-C12 aryl, substituted or unsubstituted 3-12 membered heteroaryl, 3-12 membered heterocycloalkyl ring fused C6-C12 aryl, substituted or unsubstituted C6-C12 aryl-substituted or unsubstituted C1-C8 alkyl-, or substituted or unsubstituted 3-12 membered heteroaryl-substituted or unsubstituted C1-C8 alkyl-;
$X^1$, $X^2$, $X^3$, and $X^4$ are each independently C, O, S, or N;
"- - - - -" labeled as a, b, c, d, and e are single bond or double bond;
$R^1$ is hydrogen, substituted or unsubstituted C1-C6 alkyl, substituted or unsubstituted C3-C7 cycloalkyl, or halogen;
$R^2$ is hydrogen, substituted or unsubstituted C1-C10 alkyl, or substituted or unsubstituted C3-C10 cycloalkyl;
n is 0, 1, 2, 3, 4, or 5;
wherein, any one of "substituted" refers to one or more (preferably 1, 2, 3, 4, 5, or 6) hydrogen atoms on the ring or group being substituted by substituents selected from the group consisting of C1-C8 alkyl, C3-C8 cycloalkyl, C1-C8 haloalkyl, C3-C8 halocycloalkyl, halogen, nitro, -CN, hydroxyl, thiol, amino, C1-C4 carboxyl, C2-C4 ester group, C2-C4 amido, C1-C8 alkoxy, C1-C8 alkylthio, C1-C8 haloalkoxy, C1-C8 haloalkylthio, C6-C12 aryl, 5-10 membered heteroaryl, 5-10 membered heterocycloalkyl; and
the heterocycles in the heteroaryl, heterocycloalkyl ring, and heterocycloalkyl each independently contain 1-4 (preferably 1, 2, 3, or 4) heteroatoms selected from N, O, and S.

In another preferred embodiment, the intermediate is selected from the group consisting of:

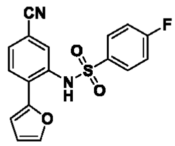 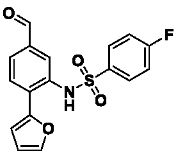 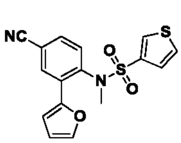 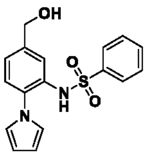 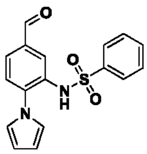 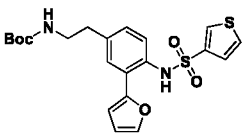

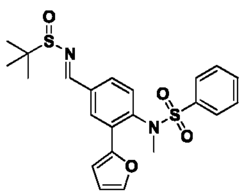 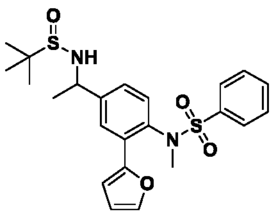 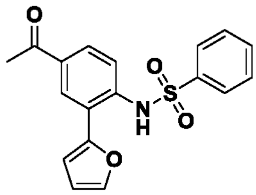 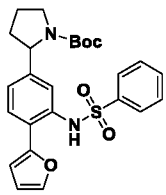 .

It should be understood that within the scope of the present invention, the above-described technical features of the present invention and the technical features described in detail below (e.g., examples) may be combined with each other to constitute a new or preferred technical solution. Limited by space, it will not be repeated here.

## DESCRIPTION OF THE DRAWINGS

Figure 1 is the results of the colon length (A), colon weight (B), ulcer area (C), and macroscopic colon injury score (D) of the compounds $I_A$-51, $I_A$-30, $I_D$-5, and $I_A$-55 of the present invention in DNBS induced colitis model in rats.
Figure 2 is representative colorectal photos of the compounds $I_A$-51, $I_A$-30, $I_D$-5, and $I_A$-55 of the present invention in DNBS induced colitis model in rats (A is blank control group, B is model-solvent group, C is osalazine sodium group, D is compound $I_A$-51 group, E is compound $I_A$-30 group, F is compound $I_D$-5 group, and G is compound $I_A$-55 group).
Figure 3 is the DAI score results of compounds $I_A$-51, $I_A$-30, $I_D$-5, and $I_A$-55 of the present invention in DSS induced inflammatory colitis model in C57BL/6 mice.
Figure 4 is the effect results of compounds $I_A$-51, $I_A$-30, $I_D$-5, and $I_A$-55 of the present invention on the weight and length of the colorectum in DSS induced inflammatory colitis model in C57BL/6 mice.
Figure 5 is the ELISA analysis results of colorectal inflammatory factors TNF-$\alpha$ and IL-10 of compounds $I_A$-51, $I_A$-30, $I_D$-5, and $I_A$-55 of the present invention in DSS induced inflammatory colitis model in C57BL/6 mice.
Figure 6 is the results of writhing frequency of compounds $I_A$-51, $I_A$-30, $I_D$-5, and $I_A$-55 of the present invention in the acetic acid induced writhing pain model in ICR mice.

## DETAILED DESCRIPTION OF THE INVENTION

Through extensive and in-depth research, the inventor unexpectedly developed a compound of formula **I**, or

an optical isomer thereof, or a racemate thereof, or a pharmaceutically acceptable salt thereof for the first time. The experiment shows that compound of formula **I** of the present invention has a significant inhibitory effect on TRPA1. The compound of formula **I** of the present invention can effectively treat inflammatory bowel disease, irritable bowel syndrome, pain, and inflammation and so on related to the TRPA1 target. The present invention has been completed on this basis.

## Terms

As used herein, the terms "include" "comprise" and "contain" can be used interchangeably, include not only closed-type definitions, but also semi-closed -type, and open-type definitions. In other words, the terms include "consist of" and "substantially consist of".

As used herein, the terms "transient receptor potential channel protein TRPA1", "TRPA1", and "transient receptor potential channel protein A1" can be used interchangeably, known as transient receptor potential ankyrin 1 in English.

It should be understood that one of ordinary skill in the art can select the substituents and substitution patterns on the compounds of the present invention to obtain chemically stable compounds, which can be synthesized by techniques known in the art and method as described below. If it is substituted by more than one (or more) substituent groups, it should be understood that the multiple groups can be on the same carbon or on different carbons, as long as a stable structure is produced.

As used herein, the term "substitution" or "substituted" refers to hydrogen atoms on a group being substituted by nonhydrogen groups, but it needs to meet its valence requirement and generate a chemically stable compound through substitution, that is, a compound that does not undergo spontaneous transformations such as cyclization or elimination.

As used herein, "R1", "$R_1$" and "$R^1$" have the same meaning and can be replaced with each other, and other similar definitions have the same meaning.

As used herein, the term "alkyl" refers to a saturated hydrocarbyl with straight (ie, unbranched) or branched chain containing only carbon atoms , or a group with a combination of straight and branched chains. When the alkyl is preceded by a carbon number limitation (e.g., C1-C10 alkyl), it means that the alkyl has 1 to 10 carbon atoms. For example, C1-C4 alkyl refers to an alkyl containing 1 to 4 carbon atoms, and representative examples include methyl, ethyl, propyl, isopropyl, butyl, isobutyl, sec-butyl, tert-butyl, or the like.

In the present invention, the term "halogen" refers to F, Cl, Br or I.

In the present invention, the term "halo (halogenated)" means being substituted by halogen.

As used herein, the term "haloalkyl" refers to one or more (preferably 1, 2, 3, or 4) hydrogen atoms of an alkyl being substituted by halogens. The alkyl and halogen are defined above, and when the alkyl is preceded by a carbon number limitation (such as C1-C6 haloalkyl), it means that the alkyl has 1 to 6 carbon atoms. For example, C1-C6 haloalkyl refers to a haloalkyl containing 1-6 carbon atoms, and representative examples include but are not limited to $-CF_3$, $-CHF_2$, monofluoroisopropyl, difluorobutyl or the like.

As used herein, the term "cycloalkyl" refers to a ring system group having a saturated or partially saturated monocyclic ring, bicyclic ring or polycyclic ring (fused, bridged or spiro ring). When a certain cycloalkyl is preceded by a carbon number limitation (e.g., C3-C12), it means that the cycloalkyl has 3 to 12 ring carbon atoms. In some preferred examples, the term "C3-C8 cycloalkyl" refers to a saturated or partially saturated monocyclic or bicyclic alkyl group having 3 to 8 carbon atoms, including cyclopropyl, cyclobutyl, cyclopentyl, cycloheptyl, or the like. "Spiro cycloalkyl" refers to a bicyclic or polycyclic group that shares a carbon atom (called a spiro atom) between the monocyclic rings. These may contain one or more double bonds, but none of the rings have a fully conjugated $\pi$ electron system. "Fused cycloalkyl" refers to an all-carbon bicyclic or polycyclic group in which each ring share a pair of neighboring carbon atoms with other ring(s) in the ring system, one or more rings of which may contain one or more double bonds, but none of the rings have a fully conjugated $\pi$ electron system. "Bridged cycloalkyl" refers to an all-carbon polycyclic group in which any two rings share two carbon atoms that are not directly bonded, these rings may contain one or more double bonds, but none of the rings have a fully conjugated $\pi$ electron system. Some representative examples of cycloalkyl groups are as follows, including but not limited to:

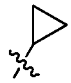 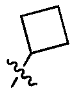 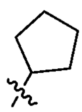 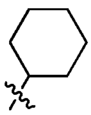 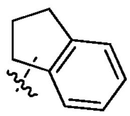 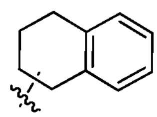 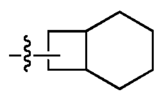

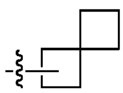 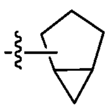 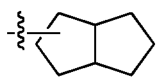 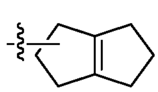 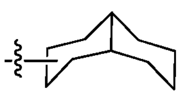 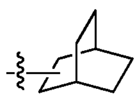

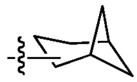 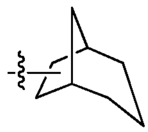

As used herein, the term "halocycloalkyl" refers to one or more (preferably 1, 2, 3, or 4) hydrogens of a cycloalkyl being substituted by halogens. The cycloalkyl and halogen are defined above, and when the cycloalkyl is preceded by a carbon number limitation (such as C3-C8 haloalkyl), it means that the cycloalkyl has 3 to 8 cyclic carbon atoms. For example, C3-C8 haloalkyl refers to a halocycloalkyl containing 3-6 carbon atoms, and representative examples include but are not limited to monofluorocyclopropyl, monochlorocyclobutyl, monofluorocyclopentyl, difluorocycloheptyl, or the like.

The term "alkoxy" refers to R-O- group, wherein R is an alkyl, and the alkyl is defined as above. When the alkoxy is preceded by a carbon number limitation, for example, C1-C8 alkoxy, it means that the alkyl in the alkoxy group has 1-8 carbon atoms. Representative examples of alkoxy include (but are not limited to): methoxy, ethoxy, n-propoxy, isopropoxy, tert-butoxy, or the like.

As used herein, the term "alkylthio" refers to the R-O- group, wherein R is an alkyl, and the alkyl is defined as defined above. When the alkylthio is preceded by a carbon number limitation, for example, C1-C8 alkylthio, it means that the alkyl in the alkylthio has 1-8 carbon atoms. Representative examples of alkylthio groups include but are not limited to: methylthio, ethylthio, n-propylthio, isopropylthio, tert-butyl thio, or the like.

As used herein, the term "haloalkoxy" refers to haloalkyl-O-, the haloalkyl is as defined above. For example, C1-C6 haloalkoxy refers to haloalkoxy containing 1-6 carbon atoms, and representative examples include but are not limited to monofluoromethoxy, monofluoroethoxy, difluorobutoxy, or the like.

As used herein, the term "haloalkylthio" refers to haloalkyl-S-, the haloalkyl is as defined above. For example, C1-C6 haloalkylthio refers to haloalkylthio containing 1-6 carbon atoms, and representative examples include but are not limited to monofluoromethylthio, monofluoroethylthio, difluorobutylthio, or similar groups.

The term "heterocycloalkyl ring" refers to a completely saturated or partially unsaturated cycle (including but not limited to, for example, 3-7 membered monocyclic, 7-11 membered bicyclic, or 8-16 membered tricyclic ring system) containing at least one heteroatom in a ring having at least one carbon atom. When the heterocycle is preceded by a member limitation, it means the number of ring atoms on theheterocycle, for example, 3-16 membered heterocycle refers to a heterocycle having 3 to 16 ring atoms. Each heteroatom-containing heterocycle can contain one or more (such as 1, 2, 3, or 4) heteroatoms, these heteroatoms are each independently selected from the group consisting of nitrogen atom, oxygen atom, and sulfur atom, wherein the nitrogen atom or sulfur atom may be oxidized, and the nitrogen atom also may be quaternized. Heterocycle can be attached to the residue of any heteroatom or carbon atom of a ring or molecule having ring system. Typical monocyclic heterocycloalkyl rings include, but are not limited to azetidine ring, oxetane ring, imidazoline ring, imidazolidine ring, tetrahydrofuran ring, piperidine ring, piperazine ring, 2-oxypiperazine ring, 2-oxypiperidine ring, 4-piperidone ring, tetrahydropyran ring, morpholine ring, thiomorpholine ring, thiomorpholine sulfoxide ring, thiomorpholine sulfone ring, 1,3-dioxane ring, and tetrahydro-1,1-dioxythiophene ring, etc.. Polycyclic heterocycloalkyl ring include spiro, fused, and bridged heterocyclic ring, wherein the spiro, fused, and bridged heterocycles involved are optionally attached to other rings by single bond, or are further fused with other cycloalkyl rings, or heterocyclyl rings by any two or more atoms on the ring.

The term "heterocycloalkyl" refers to a completely saturated or partially unsaturated cyclic group (including but not limited to, for example, 3-7 membered monocyclic, 7-11 membered bicyclic, or 8-16 membered tricyclic ring system) containing at least one heteroatom in a ring having at least one carbon atom. When the heterocycloalkyl is preceded by a member limitation, it means the number of ring atoms on the heterocycloalkyl, for example, 3-16 membered heterocycloalkyl refers to a heterocycloalkyl having 3 to 16 ring atoms. Each heteroatom-containing heterocycle can contain one or more (such as 1, 2, 3, or 4) heteroatoms, these heteroatoms are each independently selected from the group consisting of nitrogen atom, oxygen atom, and sulfur atom, wherein the nitrogen atom or sulfur atom may be oxidized, and the nitrogen atom also may be quaternized. Heterocycloalkyl can be attached to the residue of any heteroatom or carbon atom of a ring or molecule having ring system. Typical monocyclic heterocycloalkyls include, but are not limited to azetidinyl, oxetanyl, imidazolinyl, imidazolidinyl, tetrahydrofuranyl, piperidinyl, piperazinyl, 2-oxypiperazinyl, 2-oxypiperidinyl, 4-piperidonyl, tetrahydropyranyl, morpholinyl, thiomorpholinyl, thiomorpholine sulfinyl, thiomorpholine sulfonyl, 1,3-dioxanyl, and tetrahydro-1,1-dioxythiophenyl, etc.. Polycyclic heterocycloalkyls include spiro, fused, and bridged heterocyclyl, wherein the spiro, fused, and bridged heterocycloalkyls involved are optionally attached to other groups by single bond, or are further fused with other cycloalkyl rings, or heterocycles by any two or more atoms on the ring.

The term "aromatic ring" refers to an all carbon monocyclic ring or fused polycyclic ring (i.e. a ring that shares adjacent carbon atom pair) with conjugated $\pi$ electron system, which is an aromatic cyclic hydrocarbon compound. When the aromatic ring is preceded by a carbon number limitation, such as C6-C12 aromatic ring, it means that the

aromatic ring has 6 to 12 ring carbon atoms, such as benzene and naphthalene rings. The aromatic ring can fuse with other carbon rings (including saturated or unsaturated rings), but cannot contain heteroatoms such as nitrogen, oxygen, or sulfur, and the site connecting to the parent structure must be on the carbon atom on the ring with conjugated $\pi$ electron system. Representative aromatic rings are benzene ring, naphthalene ring, or the like.

The term "aryl" refers to an all carbon monocyclic or fused polycyclic (i.e., a ring that shares adjacent carbon atom pair) group with conjugated $\pi$ electron system, which is an aromatic cyclic hydrocarbon compound group. When the aryl is preceded by a carbon atom limitation, such as C6-C12 aryl, it means that the aryl has 6 to 12 cyclic carbon atoms, such as phenyl and naphthyl. The aromatic ring can fuse with other cyclic groups (including saturated or unsaturated rings), but cannot contain heteroatoms such as nitrogen, oxygen, or sulfur, and the site connecting to the parent structure must be on the carbon atom on the ring with conjugated $\pi$ electron system. The following are representative aryl examples, including but not limited to:

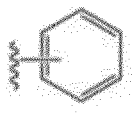 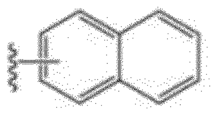 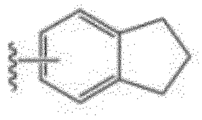 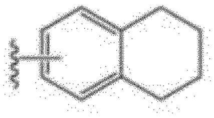 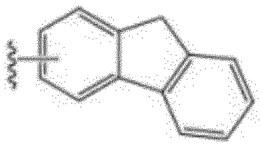.

The term "heteroaromatic ring" refers to an aromatic heterocycle with one to more (preferably 1, 2, 3, or 4) heteroatoms, which may be a monocyclic ring (monocyclic) or a fused or covalently connected polycyclic ring (bicyclic, tricyclic, or polycyclic). Each heteroatom-containing heterocycle can have one or more (such as 1, 2, 3, or 4) heteroatoms each independently selected from the group consisting of oxygen, sulfur, and nitrogen. When the heteroaromatic ring is preceded by a member limitation, it means the number of ring atoms on the heteroaromatic ring. For example, a 5-12 membered heteroaromatic ring refers to a heteroaromatic ring with 5 to 12 ring atoms. Representative examples include but are not limited to: pyrrole ring, pyrazole ring, imidazole ring, oxazole ring, isoxazole ring, thiazole ring, thiadiazole ring, isothiazole ring, furan ring, pyridine ring, pyrazine ring, pyrimidine ring, pyridazine ring, triazine ring, triazole ring, and tetrazole ring, etc..

The term "heteroaryl" refers to an aromatic heterocyclic system group with one to more (preferably 1, 2, 3, or 4) heteroatoms, which can be a monocyclic ring (monocyclic) or a fused or covalently connected polycyclic ring (bicyclic, tricyclic, or polycyclic). Each heteroatom-containing heterocycle can have one or more (such as 1, 2, 3, or 4) heteroatoms each independently selected from the group consisting of oxygen, sulfur, and nitrogen. When the heteroaryl is preceded by a member limitation, it means the number of ring atoms on the heteroaryl. For example, a 5-12 membered heteroaryl refers to a heteroaryl with 5 to 12 ring atoms. Representative examples include but are not limited to: pyrrolyl, pyrazolyl, imidazolyl, oxazolyl, isoxazolyl, thiazolyl, thiadiazolyl, isothiazolyl, furanyl, pyridinyl, pyrazinyl, pyrimidinyl, pyridazinyl, triazinyl, triazolyl, and tetrazolyl, etc..

As used herein, the term "carboxyl" refers to a -COOH group or an alkyl-COOH group, alkyl is defined as above. For example, "$C_2$-$C_4$ carboxyl" refers to a group with the -C1-C3 alkyl-COOH structure, and representative examples of carboxyl include (but are not limited to) - COOH, $-CH_2COOH$, $-C_2H_4COOH$, or the like.

As used herein, the term "ester group" refers to a R-CO-O- group or a -CO-O-R group, wherein R is alkyl, and the alkyl is defined as above. For example, "$C_2$-$C_4$ ester group" refers to a group with $C_1$-$C_3$ alkyl-CO-O- structure or -CO-O-$C_1$-$C_3$ alkyl structure. Representative examples of ester group include but are not limited to: $CH_3COO$-, $C_2H_5COO$-, $C_3H_8COO$-, $(CH_3)_2CHCOO$-, $-COOCH_3$, $-COOC_2H_5$, $-COOC_3H_8$, or the like.

As used herein, the term "amide " refers to an R-CO-N- group or a -CO-N-R group, wherein R is alky, and the alkyl is defined as above. For example, "$C_2$-$C_4$ amide" refers to a group with $C_1$-$C_3$ alkyl-CO-N- structure or -CO-N-$C_1$-$C_3$ alkyl structure. Representative examples of amide include but are not limited to: $CH_3CO$-N-, $C_2H_5CO$-N-, $C_3H_8CO$-N-, $(CH_3)_2CHCO$-N-, -CO-N-$CH_3$, -CO-N-$C_2H_5$, -CO-N-$C_3H_8$, or the like.

As used herein, when used alone or as part of other substituents, the term "amino" refers to $-NH_2$.

As used herein, when used alone or as part of other substituents, the term "nitro" refers to - $NO_2$.

As used herein, when used alone or as part of other substituents, the term "hydroxyl" refers to -OH.

As used herein, when used alone or as part of other substituents, the term "thiol" refers to - SH.

In this specification, it should be interpreted that all substituents are unsubstituted, unless explicitly described as "substituted" herein. The term "substituted" refers to one or more hydrogen atoms on a specific group being substituted by a specific substituent. The specific substituent is the substituent described previously, or the substituent appearing in each example. Preferably, the substituted refers to one or more (preferably 1, 2, 3, 4, 5, or 6) hydrogen atoms on the ring or group being substituted by substituents selected from the group consisting of C1-C8 alkyl, C3-C8 cycloalkyl, C1-C8 haloalkyl, C3-C8 halocycloalkyl, halogen, nitro, -CN, hydroxyl, thiol, amino, C1-C4 carboxyl, C2-C4 ester group, C2-C4 amide, C1-C8 alkoxy, C1-C8 alkylthio, C1-C8 haloalkoxy, C1-C8 haloalkylthio, C6-C12 aryl, 5-10 membered heteroaryl, 5-10 membered heterocycloalkyl.

In the present invention, the term "prevent" refers to a method of preventing the onset of a disease and/or its accompanying symptoms or protecting the protected subject from acquiring the disease. The term "prevent" used herein also includes delaying the onset of disease and/or its accompanying symptoms and reducing the risk of disease from the subject.

The "treat" described in the present invention includes delaying and terminating the progression of the disease, or eliminating the disease, and does not require 100% inhibition, elimination, and reversal. In some embodiments, compared to the levels observed in the absent of the composition, kit, food box or health product box, or active ingredient combination of the present invention, the composition or pharmaceutical composition of the present invention alleviates, inhibits, and/or reverses for example, at least about 10%, at least about 30%, at least about 50%, or at least about 80% by inhibiting diseases related to transient receptor potential channel protein TRPA1.

**Active ingredient**

As used herein, "compound of the present invention", "N-substituted phenylsulfonamide compound of the present invention", or "compound of formula **I**" can be used interchangeably and refers to a compound of formula I, or an optical isomer thereof, or a racemate thereof, or a pharmaceutically acceptable salt thereof, or a prodrug thereof. It should be understood that this term also includes mixtures of the aforementioned components.

The present invention provides a compound of formula **I**, or an optical isomer thereof, or a racemate thereof, or a pharmaceutically acceptable salt thereof, or a prodrug thereof;

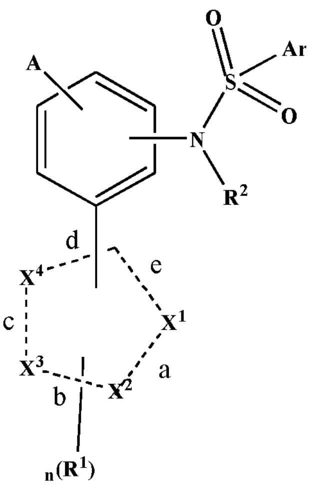

**I**

wherein,

Ar is substituted or unsubstituted C6-C12 aryl, substituted or unsubstituted 3-12 membered heteroaryl, 3-12 membered heterocycloalkyl ring fused C6-C12 aryl, substituted or unsubstituted C6-C12 aryl-substituted or unsubstituted C1-C8 alkyl-, or substituted or unsubstituted 3-12 membered heteroaryl-substituted or unsubstituted C1-C8 alkyl-;
$X^1$, $X^2$, $X^3$, and $X^4$ are each independently C, O, S, or N;
"- - - - - -" labeled as a, b, c, d, and e are single bond or double bond;
$R^1$ is hydrogen, substituted or unsubstituted C1-C6 alkyl, substituted or unsubstituted C3-C7 cycloalkyl, or halogen;
$R^2$ is hydrogen, substituted or unsubstituted C1-C10 alkyl, or substituted or unsubstituted C3-C10 cycloalkyl;
A is

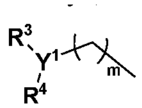 , 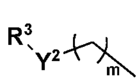 or, 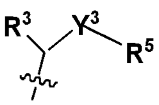 , 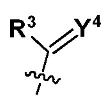 , 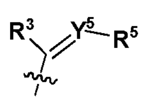 ,

substituted or unsubstituted C2-C6 ester group, substituted or unsubstituted C2-C6 carboxyl, substituted or unsubstituted C2-C6 amido, or substituted or unsubstituted $H_2N\text{-}HN\text{-}C(O)\text{-}$;

m is 0, 1, 2, or 3;
$Y^1$ is N;
$Y^2$ is O or S;
$Y^3$ is NH, O, or S;
$Y^4$ is O or S;
$Y^5$ is N;
$R^3$ and $R^4$ are each independently hydrogen, substituted or unsubstituted C1-C6 alkyl, substituted or unsubstituted C3-C8 cycloalkyl, substituted or unsubstituted C2-C6 acyl, or $R^3$ and $R^4$ together with adjacent $Y^1$ form a substituted or unsubstituted 3-8 membered heterocycloalkyl;
$R^5$ is hydrogen, hydroxyl, thiol, or substituted or unsubstituted C1-C6 alkoxy;
n is 0, 1, 2, 3, 4, or 5;
wherein, any one of "substituted" refers to one or more (preferably 1, 2, 3, 4, 5, or 6) hydrogen atoms on the ring or group being substituted by substituents selected from the group consisting of C1-C8 alkyl, C3-C8 cycloalkyl, C1-C8 haloalkyl, C3-C8 halocycloalkyl, halogen, nitro, -CN, hydroxyl, thiol, amino, C1-C4 carboxyl, C2-C4 ester group, C2-C4 amido, C1-C8 alkoxy, C1-C8 alkylthio, C1-C8 haloalkoxy, C1-C8 haloalkylthio, C6-C12 aryl, 5-10 membered heteroaryl, and 5-10 membered heterocycloalkyl; and
the heterocycles in the heteroaryl, heterocycloalkyl ring, and heterocycloalkyl each independently contain 1-4 (preferably 1, 2, 3, or 4) heteroatoms selected from N, O, and S.

Preferably, the compound of formula **I** is as described in the first aspect of the present invention.

The term "pharmaceutically acceptable salt" refers to a salt suitable for use as a drug formed by the compound of the present invention with an acid or base. Pharmaceutically acceptable salts include inorganic and organic salts. A preferred type of salt is salts formed by the compound of the present invention and an acid. The acids suitable for forming salts include (but are not limited to) inorganic acids such as hydrochloric acid, hydrobromic acid, hydrofluoric acid, sulfuric acid, nitric acid, phosphoric acid and organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenesulfonic acid; and acidic amino acids such as aspartic acid and glutamic acid. A preferred type of salt is metal salts formed by the compound of the present invention and a base. The bases suitable for forming salts include (but are not limited to) inorganic bases such as sodium hydroxide, potassium hydroxide, sodium carbonate, sodium bicarbonate, sodium phosphate, and organic bases such as ammonia, triethylamine, and diethylamine.

The compound shown in formula **I** of the present invention can be converted into a pharmaceutically acceptable salt by conventional methods. For example, a solution of the corresponding acid can be added to the solution of the compound described above, and after salt formation is completed, the corresponding salt of the compound of the present invention can be obtained by removing the solvent.

The preferred compounds of the present invention are the specific compounds prepared in the examples of the present application.

Representatively, the compounds described in the present invention are selected from Table 1 below:

Table 1

| | | | | |
|---|---|---|---|---|
| 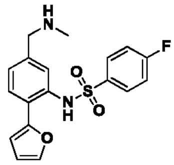 , | 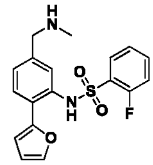 , | 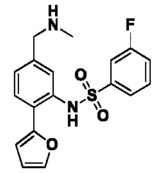 , | 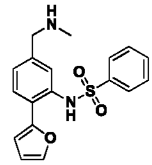 , | 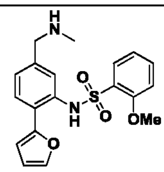 , |
| 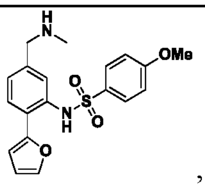 , | 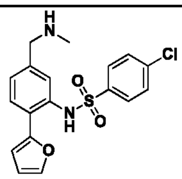 , | 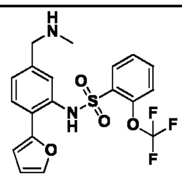 , | 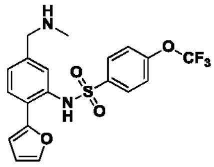 , | 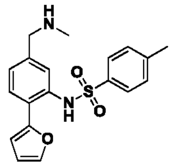 , |
| 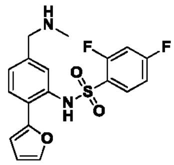 , | 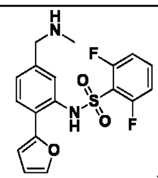 , | 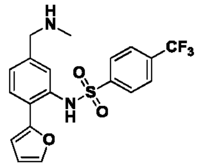 , | 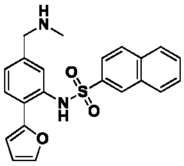 , | 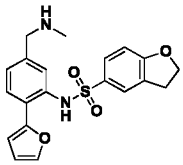 , |

| | | | | |
|---|---|---|---|---|
| 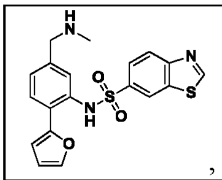, | 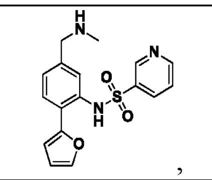, | 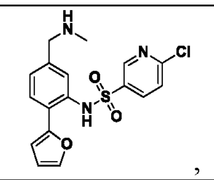, | 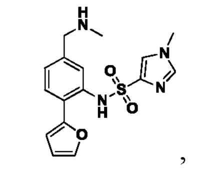, | 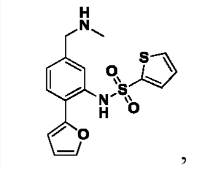, |
| 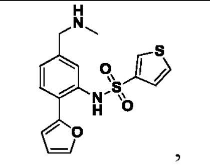, | 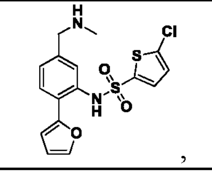, | 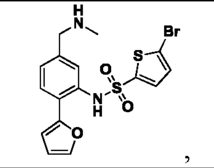, | 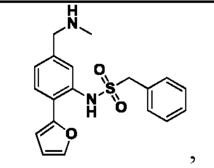, | 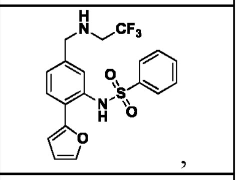, |
| 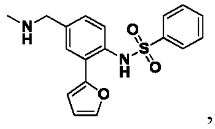, | 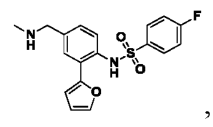, | 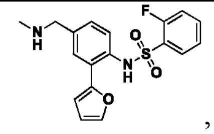, | 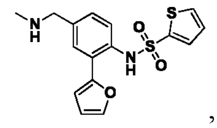, | 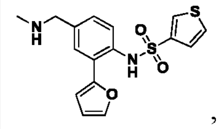, |
| 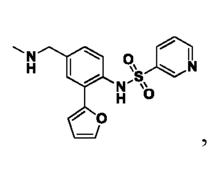, | 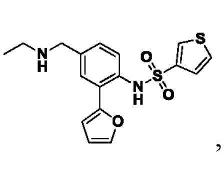, | 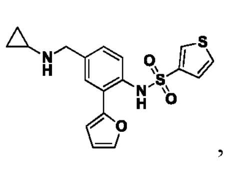, | 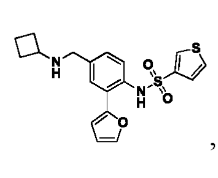, | 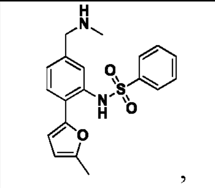, |
| 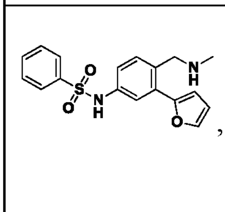, | 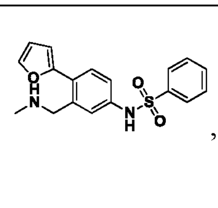, | 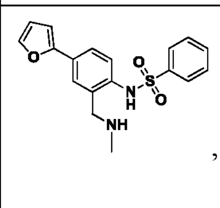, | 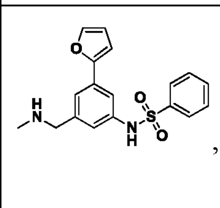, | 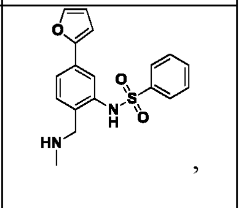, |
| 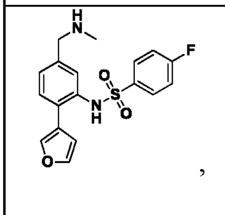, | 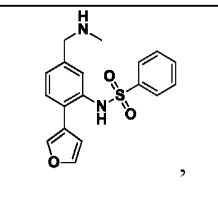, | 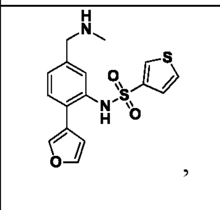, | 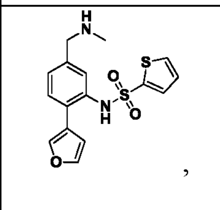, | 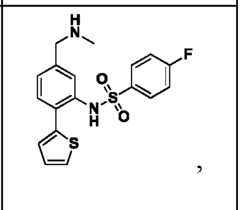, |
| 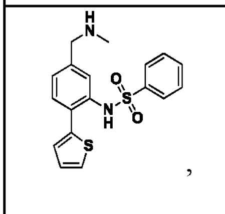, | 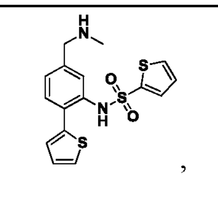, | 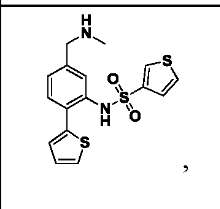, | 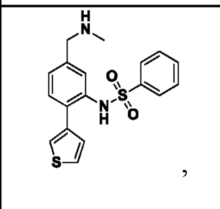, | 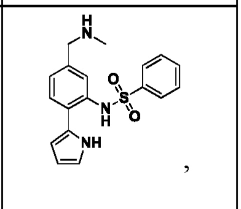, |
| 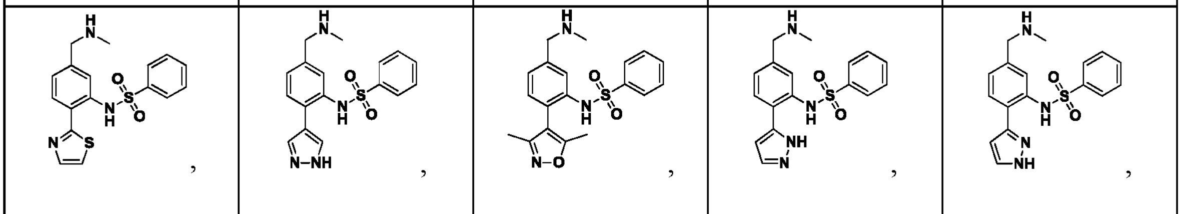, | , | , | , | , |
| 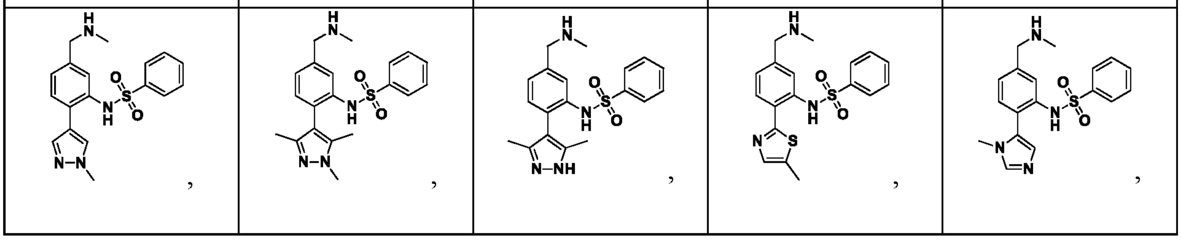, | , | , | , | , |

| | | | | |
|---|---|---|---|---|
| 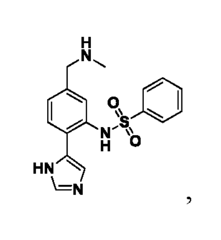 , | 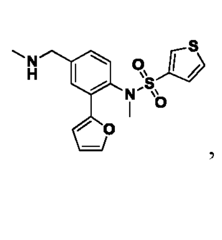 , | 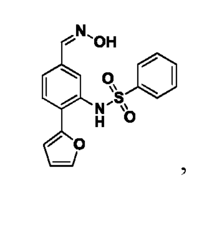 , | 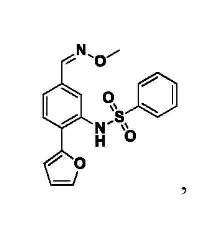 , | 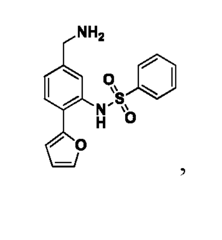 , |
| 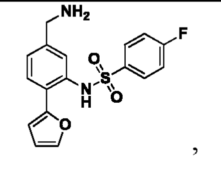 , | 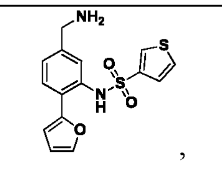 , | 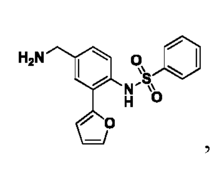 , | 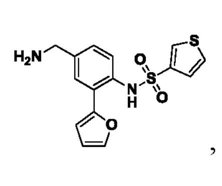 , | 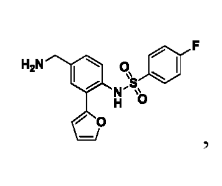 , |
| 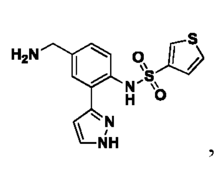 , | 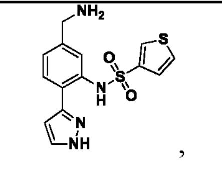 , | 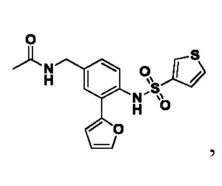 , | 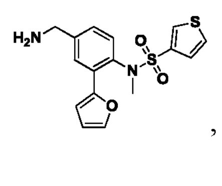 , | 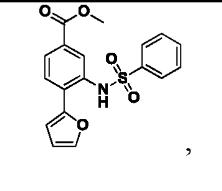 , |
| 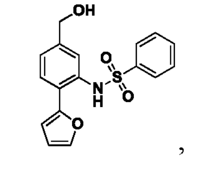 , | 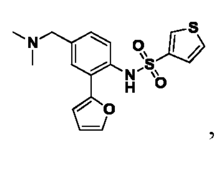 , | 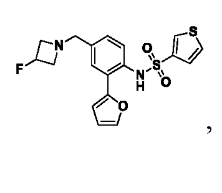 , | 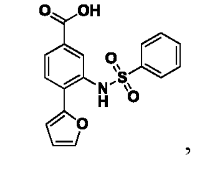 , | 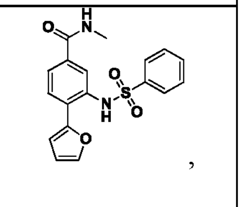 , |
| 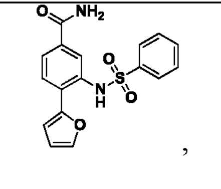 , | 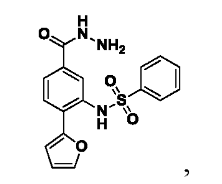 , | 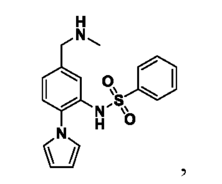 , | 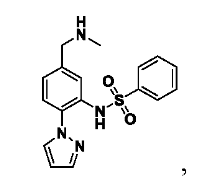 , | 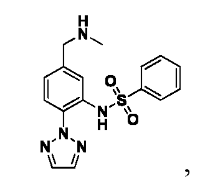 , |
| 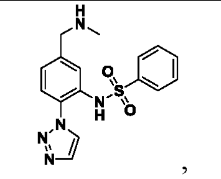 , | 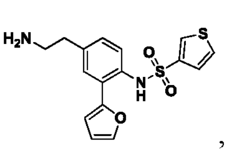 , | 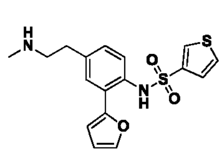 , | 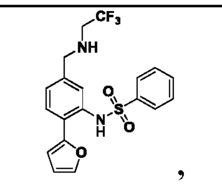 , | 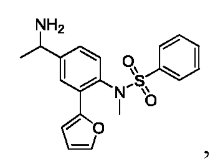 , |
| 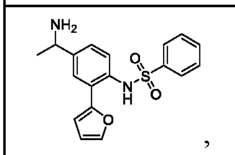 , | 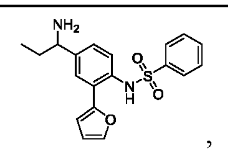 , | 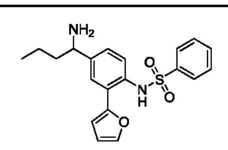 , | 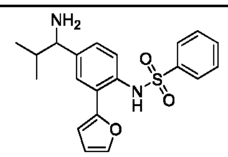 , | 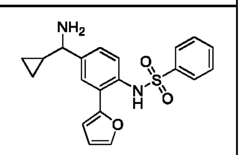 , |
| 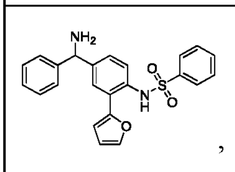 , | 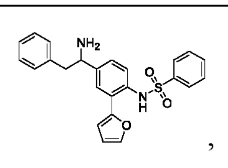 , | 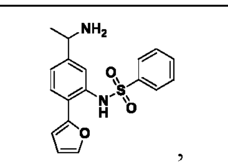 , | 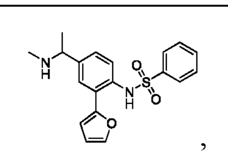 , | 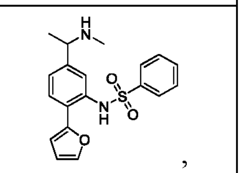 , |
| . | | | | |

## Preparation method

The present invention also provides a method for preparing compounds $\mathbf{I_A}$ to $\mathbf{I_W}$ as shown in formula I of the present invention.

The present invention also provides a method for preparing intermediates **IV** to **XXVII** for preparing the compounds described above.

The specific synthesis strategies are as follows:

Synthesis of $\mathbf{I_A}$ to $\mathbf{I_F}$:

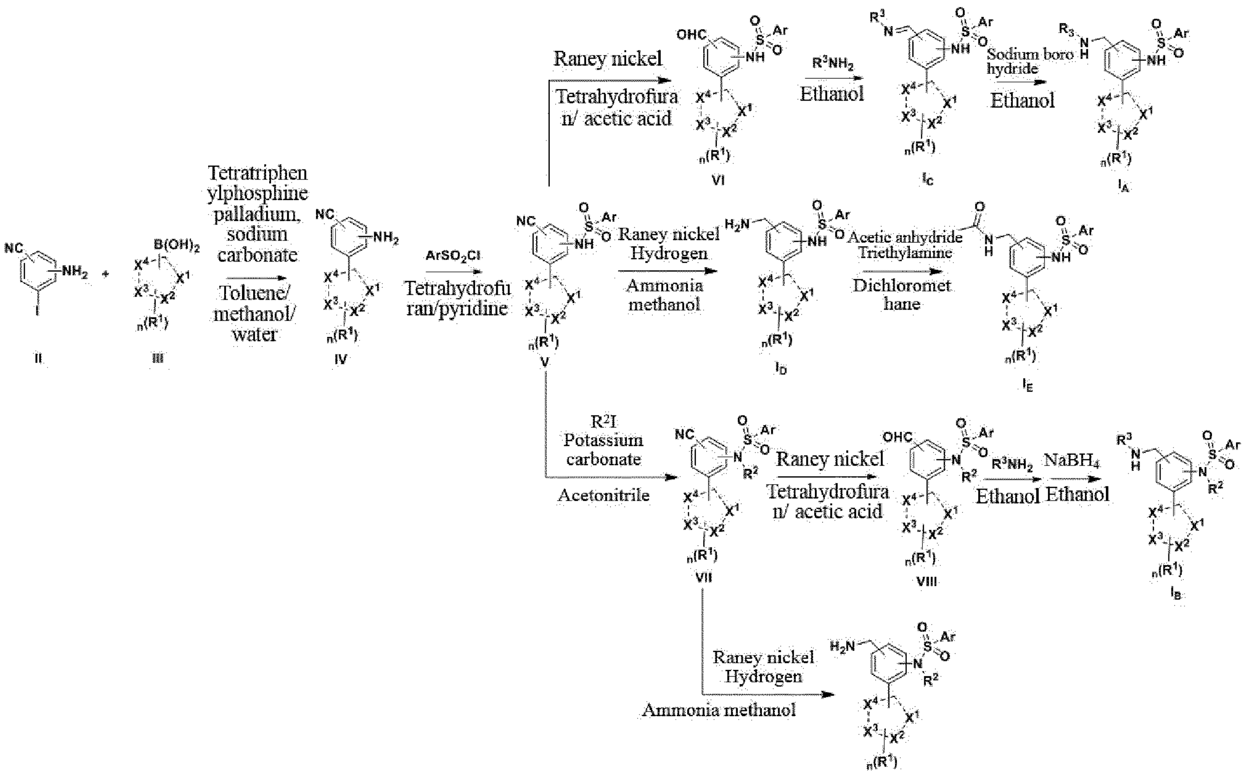


II III IV V VI $I_C$ $I_A$ $I_D$ $I_E$ VII VIII $I_B$

wherein, $X^1$, $X^2$, $X^3$, $X^4$, $R^1$, n, Ar, $R^2$, and $R^3$ are as defined in the first aspect of the present invention.

Substituted iodobenzene **II**, substituted or unsubstituted five-membered heteroaryl boronic acid **III**, tetratriphenylphosphine palladium, and sodium carbonate are dissolved in a mixed solution of toluene, methanol, and water. The mixture is heated to reflux overnight under nitrogen protection. After the reaction is completed, the solvent is evaporated to dryness, water is added to the system, the reaction solution is extracted three times with ethyl acetate, washed with water, washed with saturated saline, dried with anhydrous sodium sulfate, filtered, concentrated, and the residue was separated by column chromatography to obtain intermediate **IV**.

Intermediate **IV**, substituted or unsubstituted sulfonyl chloride are dissloved in a mixed solution of pyridine and tetrahydrofuran, the reaction is carried out overnight at 50-100 °C in a sealed tube. After the reaction is completed, the solvent is evaporated to dryness, and the residue is separated by column chromatography to obtain intermediate **V**.

Intermediate **V** is dissloved in tetrahydrofuran solution, an appropriate amount of acetic acid and Raney nickel are added, and the mixture is reacted at 50-100 °C for 1-2 hours. After the reaction is completed, Raney nickel is removed by suction filtration, the filtrate is evaporated to dryness, and the residue is separated by column chromatography to obtain intermediate **VI**.

Intermediate **VI** is dissloved in ethanol solution, substituted amine is added, and the mixture is reacted overnight at room temperature. After the reaction is completed, the solvent is evaporated to dryness, and the residue is separated by column chromatography to obtain compound $\mathbf{I_C}$.

Intermediate **VI** is dissloved in ethanol solution, substituted amine is added, and the mixture is reacted overnight at room temperature, and sodium borohydride is added in batches to the system the next day and the reaction is carried out for 1 hour. After the reaction is completed, the solvent is evaporated to dryness, and the residue is separated by

column chromatography to obtain compound $I_A$.

Intermediate **V** is dissloved in ammonia methanol solution, Raney nickel is added, hydrogen is introduced to the system, and the reaction is carried out overnight at room temperature. After the reaction is completed, Raney nickel is removed by suction filtration, the filtrate is evaporated to dryness, and the residue is separated by column chromatography to obtain compound $I_D$.

Compound $I_D$ is dissloved in dichloromethane solution, triethylamine and acetic anhydride are added, and the mixture is reacted overnight at room temperature. After the reaction is completed, the solvent is evaporated to dryness, and the residue is separated by column chromatography to obtain compound $I_E$.

Intermediate **V** is dissloved in acetonitrile solution, potassium carbonate and substituted alkyl iodine are added, and the mixture is reacted at 60-80 °C for three hours. After the reaction is completed, the solvent is evaporated and the residue is separated by column chromatography to obtain intermediate **VII**.

Intermediate **VII** is dissloved in tetrahydrofuran solution, an appropriate amount of acetic acid and Raney nickel are added, and the mixture is reacted at 50-100 °C for 1-2 hours. After the reaction is completed, Raney nickel is removed by suction filtration, the filtrate is evaporated to dryness, and the residue is separated by column chromatography to obtain intermediate **VIII**.

Intermediate **VIII** is dissloved in ethanol solution, substituted amine is added, and the mixture is reacted overnight at room temperature, and sodium borohydride is added in batches to the system the next day and the reaction is carried out for 1 hour. After the reaction is completed, the solvent is evaporated to dryness, and the residue is separated by column chromatography to obtain compound $I_B$.

Intermediate **VII** is dissloved in an ammonia methanol solution, Raney nickel is added, and hydrogen gas is introduced to the system, and the reaction is carried out overnight at room temperature. After the reaction is completed, Raney nickel is removed by filtration, the filtrate is evaporated to dryness, and the residue is separated by column chromatography to obtain compound $I_F$.

Synthesis of $I_G$, $I_H$, $I_J$, $I_K$, and $I_P$:

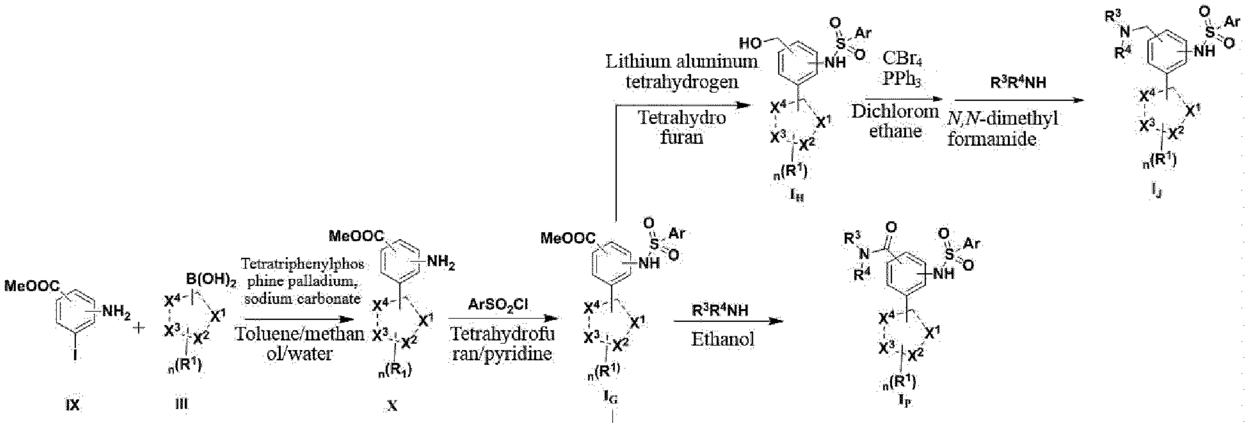


wherein, $X^1$, $X^2$, $X^3$, $X^4$, $R^1$, n, Ar, $R^3$, and $R^4$ are as defined in the first aspect of the present invention.

Substituted iodobenzene **IX**, substituted or unsubstituted five-membered heteroaryl boronic acid **III**, tetratriphenylphosphine palladium, and sodium carbonate are dissolved in a mixed solution of toluene, methanol, and water. The mixture is heated to reflux overnight under nitrogen protection. After the reaction is completed, the solvent is evaporated to dryness, water is added to the system, the reaction solution is extracted three times with ethyl acetate, washed with water, washed with saturated saline, dried with anhydrous sodium sulfate, filtered, concentrated, and the residue was separated by column chromatography to obtain intermediate **X**.

Intermediate **X**, substituted or unsubstituted sulfonyl chloride are dissloved in a mixed solution of pyridine and tetrahydrofuran, the reaction is carried out overnight at 50-100 °C in a sealed tube. After the reaction is completed, the solvent is evaporated to dryness, and the residue is separated by column chromatography to obtain compound $I_G$.

Compound $I_G$ is dissloved in tetrahydrofuran solution, the atmosphere is protected with nitrogen, and lithium aluminum tetrahydrogen is added in batches under ice bath condition. The mixture is reacted at room temperature for

1-4 hours. Post treatment: water, sodium hydroxide aqueous solution, and water are sequentially added to the system, the obtained mixture is suction filtered, and the filtrate is evaporated to dryness, and the residue is separated by column chromatography to obtain compound $\mathbf{I_H}$.

Compound $\mathbf{I_H}$ is dissloved in a dichloromethane solution, triphenylphosphine and carbon tetrabromide are added under ice bath condition, and the mixture is reacted at room temperature for 1-4 hours. After the reaction is completed, the solvent is evaporated to dryness. The residue is dissolved in *N,N*-dimethylformamide solution, substituted amine and potassium carbonate are added, and the reaction is carried out overnight at 60-90 °C. After the reaction is completed, water is added to the system, the reaction solution is extracted three times with ethyl acetate, washed with water, washed with saturated saline, dried with anhydrous sodium sulfate, filtered, concentrated, and the residue is separated by column chromatography to obtain compound $\mathbf{I_J}$.

Compound $\mathbf{I_G}$ is dissloved in ethanol solution, substituted amine is added, and the mixture is reacted overnight at 60-100 °C. After the reaction is completed, the solvent is evaporated to dryness, and the residue is separated by column chromatography to obtain compound $\mathbf{I_P}$.

Compound $\mathbf{I_G}$ is dissloved in tetrahydrofuran solution, lithium hydroxide aqueous solution is added, and the mixture is reacted overnight at 40-60 °C. After the reaction is completed, the solvent is evaporated to dryness, an appropriate amount of dilute hydrochloric acid is added to the system to adjust the pH to 3-4, then the reaction solution is extracted three times with ethyl acetate, washed with water, washed with saturated saline, dried with anhydrous sodium sulfate, filtered, concentrated, and the residue is separated by column chromatography to obtain compound $\mathbf{I_K}$.

Synthesis of $\mathbf{I_Q}$:

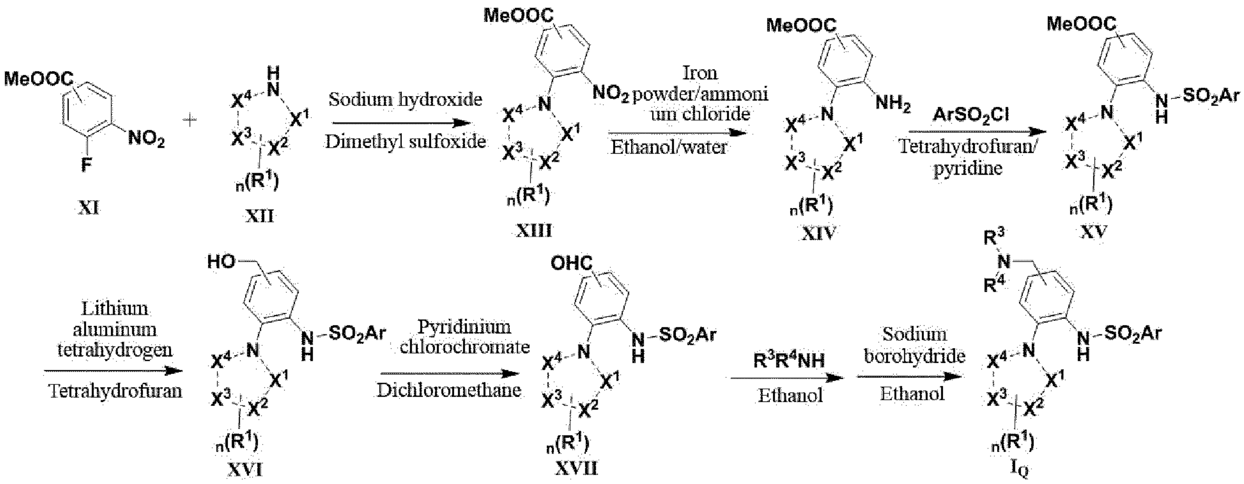


XI XII XIII XIV XV XVI XVII $I_Q$

wherein, $X^1$, $X^2$, $X^3$, $X^4$, $R^1$, n, Ar, $R^3$, and $R^4$ are as defined in the first aspect of the present invention.

Substituted fluorobenzene **XI**, substituted or unsubstituted five-membered nitrogen-containing heteroaromatic ring compound **XII** are dissolved in dimethyl sulfoxide solution, sodium hydroxide is added, and the mixture is reacted overnight at 40-80 °C under nitrogen protection. After the reaction is completed, water is added to the system, the reaction solution is extracted three times with ethyl acetate, washed with water, washed with saturated saline, dried with anhydrous sodium sulfate, filtered, concentrated, and the residue was separated by column chromatography to obtain intermediate **XIII**.

Intermediate **XIII** is dissloved in a mixed solution of ethanol and water, iron powder and ammonium chloride are added, and the mixture is reacted at 60-80 °C for 1 hour. After the reaction is completed, the reaction solution is suction filtered, the filtrate is concentrated, extracted three times with dichloromethane, washed with water, washed with saturated saline, dried with anhydrous sodium sulfate, filtered, concentrated, and the residue is separated by column chromatography to obtain intermediate **XIV**.

Intermediate **XIV**, substituted or unsubstituted sulfonyl chloride are dissloved in a mixed solution of pyridine and tetrahydrofuran, the reaction is carried out overnight at 50-100 °C in a sealed tube. After the reaction is completed, the solvent is evaporated to dryness, and the residue is separated by column chromatography to obtain intermediate **XV**.

Intermediate **XV** is dissloved in tetrahydrofuran solution, the atmosphere is protected with nitrogen, and lithium aluminum tetrahydrogen is added under ice bath condition. The mixture is reacted at room temperature for 1-4 hours. Post treatment: water, sodium hydroxide aqueous solution, and water are sequentially added to the system, the obtained

mixture is suction filtered, the filtrate is evaporated to dryness, and the residue is separated by column chromatography to obtain intermediate **XVI**.

Intermediate **XVI** is dissloved in dichloromethane solution, pyridinium chlorochromate is added, and the mixture is reacted at room temperature for 1 hour. Post treatment: the obtained mixture is suction filtered, the filtrate is evaporated to dryness, and the residue is separated by column chromatography to obtain intermediate **XVII**.

Intermediate **XVII** is dissloved in ethanol solution, substituted amine is added, and the mixture is reacted overnight at room temperature, and sodium borohydride is added in batches to the system the next day and the reaction is carried out for 1 hour. After the reaction is completed, the solvent is evaporated to dryness and the residue is separated by column chromatography to obtain compound $\mathbf{I_Q}$.

Synthesis of $\mathbf{I_R}$ and $\mathbf{I_S}$:

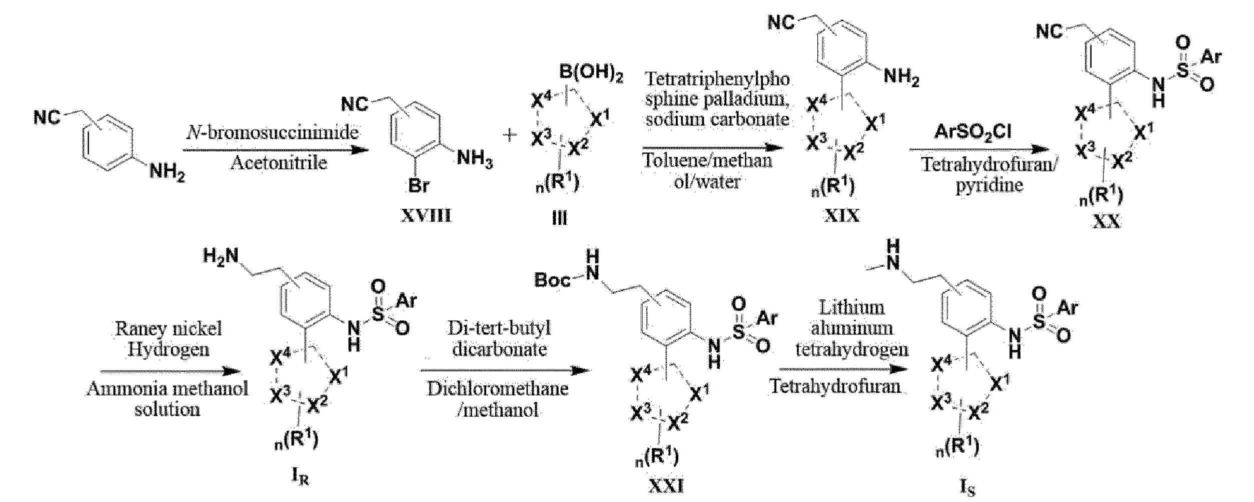


wherein, $X^1$, $X^2$, $X^3$, $X^4$, $R^1$, n, and Ar are as defined in the first aspect of the present invention.

Amino substituted phenylacetonitrile is dissolved in acetonitrile solution, N-bromosuccinimide is added, and the mixture is reacted at room temperature for 0.5-2 hours. After the reaction is completed, the solvent is evaporated and the residue is separated by column chromatography to obtain the intermediate **XVIII**.

Intermediate **XVIII**, substituted or unsubstituted five-membered heteroaryl boronic acid **III**, tetratriphenylphosphine palladium, and sodium carbonate are dissolved in a mixed solution of toluene, methanol, and water. The mixture is heated to reflux overnight under nitrogen protection. After the reaction is completed, the solvent is evaporated to dryness, water is added to the system, the reaction solution is extracted three times with ethyl acetate, washed with water, washed with saturated saline, dried with anhydrous sodium sulfate, filtered, concentrated, and the residue was separated by column chromatography to obtain intermediate **XIX**.

Intermediate **XIX**, substituted or unsubstituted sulfonyl chloride are dissloved in a mixed solution of pyridine and tetrahydrofuran, the reaction is carried out overnight at 50-100 °C in a sealed tube. After the reaction is completed, the solvent is evaporated to dryness, and the residue is separated by column chromatography to obtain intermediate **XX**.

Intermediate **XX** is dissloved in ammonia methanol solution, Raney nickel is added, hydrogen is introduced to the system, and the reaction is carried out overnight at room temperature. After the reaction is completed, Raney nickel is removed by suction filtration, the filtrate is evaporated to dryness, and the residue is separated by column chromatography to obtain compound $\mathbf{I_R}$.

Compound $\mathbf{I_R}$ is dissloved in a mixed solution of dichloromethane and methanol, di-tert-butyl dicarbonate is added, and the mixture is reacted overnight at room temperature. After the reaction is completed, the solvent is evaporated to to dryness, and the residue is separated by column chromatography to obtain intermediate **XXI**.

Intermediate **XXI** is dissoloved in tetrahydrofuran solution, lithium aluminum tetrahydrogen is added under ice bath and nitrogen protection, and the mixture is reacted overnight at 60-80 °C. Post treatment: water, sodium hydroxide aqueous solution, and water are sequentially added to the system, the obtained solution is filtered, and the filtrate is evaporated to dryness, and the residue is separated by column chromatography to obtain compound $\mathbf{I_S}$.

Synthesis of $I_U$:

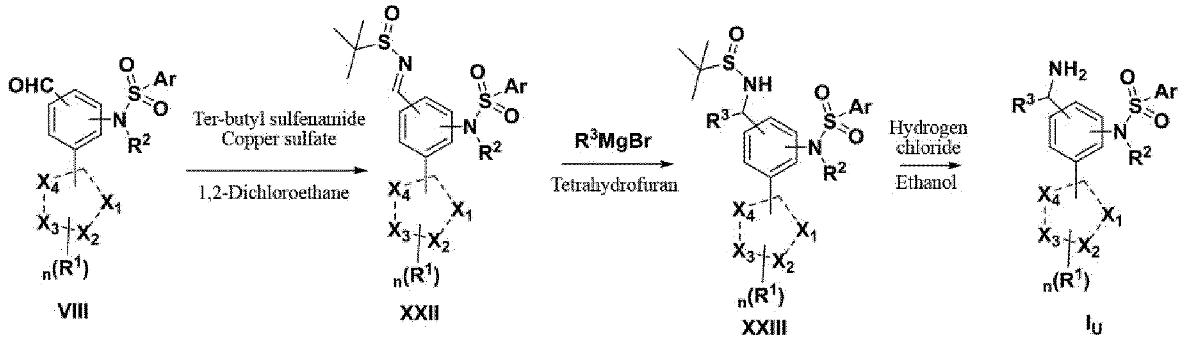


VIII XXII XXIII $I_U$

wherein, $X^1$, $X^2$, $X^3$, $X^4$, $R^1$, n, and Ar are as defined in the first aspect of the present invention.

Intermediate **VIII**, ter-butyl sulfenamide, and copper sulfate are dissloved in 30 milliliters of anhydrous 1,2-dichloroethane solution, the mixture is heated and reacted overnight. After the reaction is completed and cool to room temperature, the obtained solution is filtered, the filtrate is concentrated, and the residue is separate by column chromatography to obtain the intermediate **XXII**.

Intermediate **XXII** is dissloved in anhydrous tetrahydrofuran solution, and a solution of methylmagnesium bromide in tetrahydrofuran is slowly added dropwise at -78 °C. After addition, the temperature is slowly elevated to -20 °C to react. After the reaction is completed, ammonium chloride aqueous solution is added to the system under ice bath condition for quenching, water is added, the obtained solution is extracted with ethyl acetate, washed with water, washed with saturated saline, dried with anhydrous sodium sulfate, filtered, concentrated, and the residue is separated by column chromatography to obtain intermediate **XXIII**.

Intermediate **XXIII** is dissloved in ethanol solution, a solution of hydrochloric acid in ethanol is added, and the reaction is carried out at 60 °C. After the reaction is completed, the solvent is evaporated to dryness, and water is added to the residue, an appropriate amount of sodium bicarbonate aqueous solution is added, the pH is adjusted to 8-9, the obtained solution is extracted with ethyl acetate, washed with water, washed with saturated saline, dried with anhydrous sodium sulfate, filtered, concentrated, and the residue was separated by column chromatography to obtain compound $I_U$.

Synthesis of $I_V$:

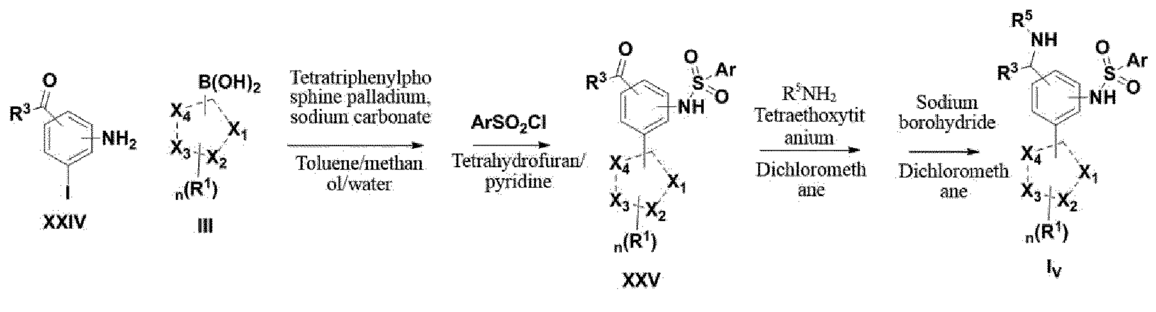


XXIV III XXV $I_V$

wherein, $X^1$, $X^2$, $X^3$, $X^4$, $R^1$, n, and Ar are as defined in the first aspect of the present invention.

Intermediate **XXV** is obtained using a similar method to synthesize intermediate **V**.

Intermediate **XXV** is dissolved in dichloromethane solution, a solution of methylamine in ethanol, tetraethoxytitanium are added, and the mixture is reacted overnight at room temperature. Sodium borohydride is slowly added under ice water bath condition and the mixture is reacted at room temperature. After the reaction is completed, water is added to the system, the reaction solution is extracted with dichloromethane, washed with water, washed with saturated saline, dried with anhydrous sodium sulfate, filtered, concentrated, and the residue was separated by column chromatography to obtain compound $I_V$.

Synthesis of $I_W$:

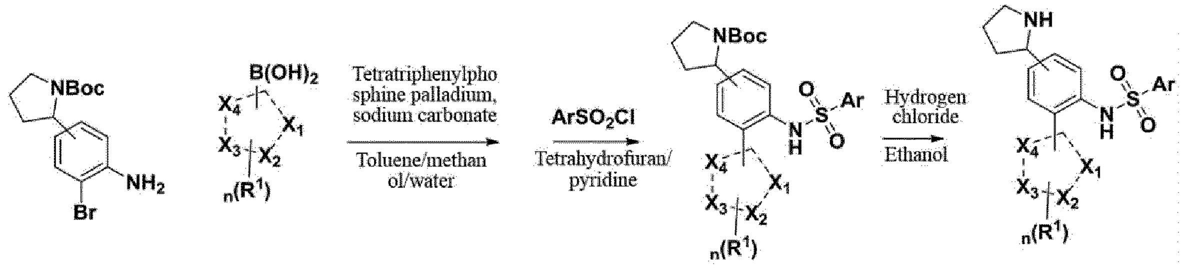


XXVI III XXVII $I_W$

wherein, $X^1$, $X^2$, $X^3$, $X^4$, $R^1$, n, and Ar are as defined in the first aspect of the present invention.

Intermediate **XXVII** is obtained using a similar method to synthesize intermediate **V**.

Intermediate XXVII is dissolved in ethyl acetate solution, a solution of hydrochloric acid in ethanol is added, and the reaction is carried out at room temperature. After the reaction is completed, the solvent is evaporated to dryness, and water is added to the residue, an appropriate amount of sodium bicarbonate aqueous solution is added, the pH is adjusted to 8-9, the obtained solution is extracted with ethyl acetate, washed with water, washed with saturated saline, dried with anhydrous sodium sulfate, filtered, concentrated, and the residue was separated by column chromatography to obtain compound $\mathbf{I_W}$.

## Transient receptor potential channel protein (TRP)

Transient receptor potential channel proteins are a protein superfamily composed of important cation channels that exists on the cell membrane. Transient receptor potential channel protein include multiple subfamilies, such as TRPA1, TRPC, TRPM, TRPV, TRPML, and TRPP subfamilies.

Research has found that TRPA1 channel protein is related to inflammatory bowel disease, pain, irritable bowel syndrome, inflammation and other diseases. TRPA1 is a target for treating inflammatory bowel disease, pain, irritable bowel syndrome, inflammation and other diseases.

## Use

The present invention also provides a method for inhibiting transient receptor potential channel protein TRPA1 and a method for treating diseases related to TRPA1.

The compounds of the present invention can be used to inhibit the transient receptor potential channel protein TRPA1, thereby preventing or treating diseases related to the transient receptor potential channel protein TRPA1.

In the present invention, examples of diseases related to transient receptor potential channel protein TRPA1 include (but are not limited to): inflammatory bowel disease, pain, irritable bowel syndrome, inflammation. Representatively, the inflammatory bowel disease includes ulcerative colitis and Crohn's disease; the pain includes (but is not limited to): visceral pain, acute inflammatory pain, chronic inflammatory pain, neurogenic pain, fibromyalgia, headache, neuralgia, or pain caused by cancer.

In a preferred embodiment, the present invention provides a method for *in vitro* non-therapeutically and non-diagnostically inhibiting the activity of transient receptor potential channel protein TRPA1, comprising, for example, in an *in vitro* culture system, contacting transient receptor potential channel protein TRPA1 or cells expressing the transient receptor potential channel protein with the compound of the present invention, thereby inhibiting the activity of transient receptor potential channel protein TRPA1.

The present invention also provides a method for inhibiting the transient receptor potential channel protein TRPA1, which can be therapeutic or non-therapeutic. Typically, the method comprises the steps of administering the compound of the present invention to a subject in need thereof.

Preferably, the subject include humans and non-human mammals (such as rodents, rabbits, monkeys, domestic animals, dogs, cats, etc.).

## Crohn's disease

Crohn's disease is an inflammatory bowel disease that can cause gastrointestinal inflammation. People of any

age are at risk of developing Crohn's disease, but it usually occurs between the ages of 13 and 30. The most common lesion location are the lower part of the small intestine (known as the ileum) and the upper part of the colon. Any part of the gastrointestinal tract from the mouth to the anus may show lesion of Crohn's disease.

**Ulcerative colitis**

Ulcerative colitis is a chronic non-specific inflammatory disease of the rectum and colon with unclear etiology. The lesion are mainly limited to the mucosa and submucosa of the large intestine. The main clinical manifestations include diarrhea, mucopurulent and bloody stools, and abdominal pain, which often recur and is protracted course of disease.

**Composition and administration method**

The present invention provides a composition for inhibiting the activity of transient receptor potential channel protein TRPA1. The compositions include (but are not limited to): pharmaceutical composition, food composition, dietary supplement, beverage composition, etc.

Typically, the composition is a pharmaceutical composition, comprising the compound as described in the present invention; and pharmaceutically acceptable carriers.

In the present invention, the dosage forms of the pharmaceutical composition include (but are not limited to) oral fomulation, injectable formulation, and topical formulation.

Representatives include (but are not limited to): tablet, injection, infusion solution, paste, gel, solution, microsphere and film.

The term "pharmaceutically acceptable carrier" refers to one or more compatible solid, semi-solid, liquid or gel fillers, which are suitable for human or animal use and should be of sufficient purity and sufficient low toxicity. "Compatibility" means that the each component in the pharmaceutical composition can be admixed with the active ingredient of medication and with each other without significantly reducing its efficacy.

It should be understood that in the present invention, there are no special restrictions on the carrier, and commonly used materials in the art can be selected, or prepared by conventional methods, or purchased from the market. Some examples of pharmaceutically acceptable carriers include cellulose and the derivatives thereof (such as methyl cellulose, ethyl cellulose, hydroxypropyl methyl cellulose, sodium carboxymethyl cellulose, etc.), gelatin, talc powder, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween), wetting agents (such as sodium dodecyl sulfate), buffering agents, chelating agents, thickeners, pH regulators, transdermal enhancers, coloring agents, seasoning agents, stabilizers, antioxidants, preservatives, antibacterial agents, pyrogen-free water, etc.

Representatively, liquid formulations, in addition to active pharmaceutical ingredients, may include inert diluent commonly used in the art, such as water or other solvents, solubilizers, and emulsifiers, such as ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil and sesame oil, or mixtures of these substances. In addition to these inert diluents, the composition may also include additives such as wetting agents, emulsifiers, and suspension agents.

The pharmaceutical formulation should match the administration method. The pharmaceutical formulation of the present invention can also be used in combination (including before, during, or after use) with other synergistic therapeutic agents. When using pharmaceutical combination or formulation, a safe and effective amount of the drug is administered to the subject in need thereof (such as human or non-human mammal), the safe and effective amount is typically at least about 10 μg/kg body weight, and in most cases not exceeding about 8 μg/kg body weight, preferably about 10 μg/kg body weight to about 1 mg/kg body weight. Of course, the specific dosage should also consider factors such as the route of administration and the patient's health status, all of which are within the scope of skilled physicians.

**The main advantages of the present invention include:**

(a) The present invention provides a class of structurally novel compound of formula **I** with excellent TRPA1 inhibitory activity.

(b) The compounds of the present invention have excellent therapeutic effect on inflammatory bowel disease.

(c) The compounds of the present invention have excellent analgesic effect.

(d) The compounds of the present invention have low toxicity and strong efficacy, thus possessing a large safety window.

(e) The compounds of the present invention have good druggability.
(f) The compounds of the present invention have excellent pharmacokinetic property.
(g) The compounds of the present invention are suitable for oral administration.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, percentage and parts are calculated by weight.

**Example 1**

**Synthesis of compound $I_A$:**

**Step 1:** 3-amino-4-(furan-2-yl)benzonitrile **(IV-1)**

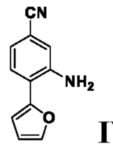

**IV-1**

3-Amino-4-iodobenzonitrile (244.0mg, 1mmol), furan-2-boronic acid (123.1mg, 1.1mmol), tetratriphenylphosphine palladium (57.8mg, 0.05mmol), and sodium carbonate (318.0mg, 3mmol) were dissolved in 26 milliliter of mixed solution of toluene/methanol/water (volume/volume/volume=9:3:1). The mixture was refluxed overnight. After the reaction was finished, the solvent was evaporated to dryness, and water was added to the residue. The resulting mixture was extracted three times with ethyl acetate, washed with water, washed with saturated brine, dried with anhydrous sodium sulfate, filtered, concentrated and the residue was separated by column chromatography to obtain **intermediate IV-1**, as an off-white solid (172.0mg, 93.4%).

$^1H$ NMR (400 MHz, $CDCl_3$) $\delta$ 7.69 (d, $J$ = 1.9 Hz, 1H), 7.50 (dd, $J$ = 1.8, 0.7 Hz, 1H), 7.30 (dd, $J$ = 8.4, 2.0 Hz, 1H), 6.70 (d, $J$ = 8.4 Hz, 1H), 6.59 (dd, $J$ = 3.4, 0.7 Hz, 1H), 6.51 (dd, $J$ = 3.4, 1.9 Hz, 1H).

**Step 2:** N-(5-cyano-2-(furan-2-yl)phenyl)-4-fluorobenzenesulfonamide **(V-1)**

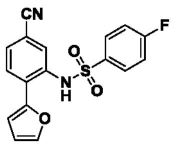

**V-1**

Intermediate **IV-1** (92.1mg, 0.5mmol) and 4-fluorobenzenesulfonyl chloride (97.3mg, 0.5mmol) were dissolved in 10 milliliter of mixed solution of tetrahydrofuran/pyridine (volume/volume=1:1). The mixture was placed in a sealed tube and reacted overnight. After the reaction was completed, the solvent was evaporated and the residue was separated by column chromatography to obtain intermediate **V-1**, as a yellow solid (94.8mg, 55.4%)

$^1H$ NMR (500 MHz, DMSO) $\delta$ 10.13 (s, 1H), 8.04 (d, $J$ = 1.9 Hz, 1H), 7.81 - 7.77 (m, 2H), 7.76 (d, $J$ = 1.3 Hz, 1H), 7.67 (dd, $J$ = 8.4, 2.0 Hz, 1H), 7.38 (dd, $J$ = 12.2, 5.5 Hz, 2H), 7.20 (d, $J$ = 8.4 Hz, 1H), 7.01 (d, $J$ = 3.4 Hz, 1H), 6.60 (dd, $J$ = 3.4, 1.8 Hz, 1H).

**Step 3:** 4-Fluoro-N-(5-formyl-2-(furan-2-yl)phenyl)benzenesulfonamide (intermediate **VI-**1)

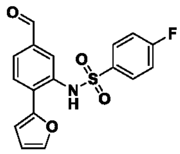

VI-1

Intermediate **V-1** (68.5mg, 0.2mmol) was dissolved in 12 milliliter of mixed solution of tetrahydrofuran/acetic acid (volume/volume=5:1). An appropriate amount of Raney nickel aqueous solution was added into the reaction system, then the hydrogen gas was introduced. The reaction was carried out at 60 °C for 1 hour. After the reaction was finished, Raney nickel was removed by suction filtration, the filtrate was evaporated to dryness, and the residue was separated by column chromatography to obtain intermediate **VI-1**, as a yellow solid (32.9mg, 47.6%).

$^1$H NMR (400 MHz, DMSO) $\delta$ 10.11 (s, 1H), 9.94 (s, 1H), 8.13 (d, *J* = 1.9 Hz, 1H), 7.82 - 7.75 (m, 3H), 7.71 (dd, *J* = 8.3, 2.0 Hz, 1H), 7.40 - 7.33 (m, 2H), 7.20 (d, *J* = 8.3 Hz, 1H), 6.98 (dd, *J* = 3.4, 0.5 Hz, 1H), 6.60 (dd, *J* = 3.4, 1.8 Hz, 1H).

**Steps 4-5:** 4-Fluoro-*N*-(2-(furan-2-yl)-5-((methylamino)methyl)phenyl) benzenesulfonamide (compound **I$_A$-1**)

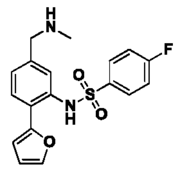

I$_A$-1

Intermediate **VI-1** (27.6mg, 0.08mmol) was dissolved in ethanol solution, then 1 milliliter of the solution of methylamine (30-33 wt%) in ethanol was added. After reaction was carried out overnight, sodium borohydride (3.0mg, 0.08mmol) was added. After the reaction was finished, the solvent was evaporated and the residue was separated by column chromatography to obtain compound **I$_A$-1**, as a white solid (14.0mg, 48.7%).

HPLC: 99.1%; LC-MS (m/z): 361.10 (M+H)$^+$; $^1$H NMR (400 MHz, DMSO) $\delta$7.82 - 7.66 (m, 2H), 7.66 - 7.54 (m, 2H), 7.38 (d, *J* = 3.1 Hz, 1H), 7.25 (d, *J* = 1.5 Hz, 1H), 7.22 - 7.10 (m, 2H), 6.81 (d, *J* = 7.7 Hz, 1H), 6.51 (dd, *J* = 3.3, 1.8 Hz, 1H), 3.83 (s, 2H), 2.40 (s, 3H).

Similar methods for synthesizing **I$_A$-1** were applied to obtain the following compounds: 2-Fluoro-*N*-(2-(furan-2-yl)-5-((methylamino)methyl)phenyl)benzenesulfonamide (**I$_A$-2**)

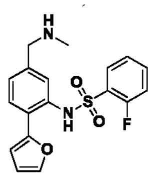

I$_A$-2

HPLC: 99.0%; LC-MS (m/z): 361.09 (M+H)$^+$; $^1$H NMR (400 MHz, DMSO) $\delta$7.74 (td, *J* = 7.6, 1.5 Hz, 1H), 7.64 - 7.55 (m, 2H), 7.51 (s, 1H), 7.43 - 7.30 (m, 2H), 7.13 (dd, *J* = 15.1, 7.9 Hz, 2H), 6.72 (d, *J* = 7.8 Hz, 1H), 6.49 (dd, *J* = 3.2, 1.8 Hz, 1H), 3.85 (s, 2H), 2.43 (s, 3H).

3-Fluoro-*N*-(2-(furan-2-yl)-5-((methylamino)methyl)phenyl)benzenesulfonamide (**I$_A$-3**)

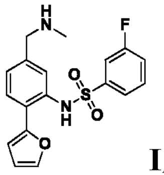

I$_A$-3

HPLC: 98.9%; LC-MS (m/z): 361.11 $(M+H)^+$; $^1H$ NMR (400 MHz, DMSO) $\delta$ 7.64 - 7.55 (m, 2H), 7.53 (d, *J* = 7.8 Hz, 1H), 7.48 - 7.35 (m, 3H), 7.32 (d, *J* = 1.3 Hz, 1H), 7.19 (td, *J* = 8.5, 2.3 Hz, 1H), 6.76 (d, *J* = 8.1 Hz, 1H), 6.51 (dd, *J* = 3.2, 1.8 Hz, 1H), 3.87 (s, 2H), 2.43 (s, 3H).

*N*-(2-(furan-2-yl)-5-((methylamino)methyl)phenyl)benzenesulfonamide (**$I_A$-4**)

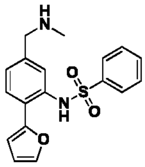

$I_A$-4

HPLC: 99.4%; LC-MS (m/z): 343.10 $(M+H)^+$; $^1H$ NMR (400 MHz, DMSO) $\delta$ 7.75 - 7.66 (m, 2H), 7.60 (d, *J* = 8.0 Hz, 2H), 7.43 - 7.31 (m, 4H), 7.21 (s, 1H), 6.82 (d, *J* = 8.0 Hz, 1H), 6.51 (dd, *J* = 3.3, 1.8 Hz, 1H), 3.76 (s, 2H), 2.35 (s, 3H).

2-Methoxy-*N*-(2-(furan-2-yl)-5-((methylamino)methyl)phenyl)benzenesulfonamide (**$I_A$-5**)

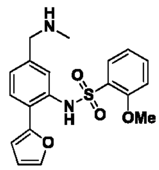

$I_A$-5

HPLC: 98.8%; LC-MS (m/z): 373.11 $(M+H)^+$; $^1H$ NMR (400 MHz, DMSO) $\delta$ 7.75 (d, *J* = 1.2 Hz, 1H), 7.67 (dd, *J* = 7.8, 1.6 Hz, 1H), 7.58 (d, *J* = 8.0 Hz, 1H), 7.54 - 7.45 (m, 1H), 7.15 - 7.08 (m, 3H), 7.03 (t, *J* = 11.0 Hz, 1H), 6.96 (dd, *J* = 17.7, 10.3 Hz, 1H), 6.63 - 6.53 (m, 1H), 3.70 (s, 3H), 3.59 (s, 2H), 2.16 (s, 3H).

4-Methoxy-*N*-(2-(furan-2-yl)-5-((methylamino)methyl)phenyl)benzenesulfonamide (**$I_A$-6**)

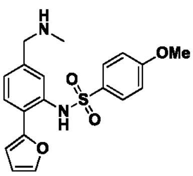

$I_A$-6

HPLC: 97.9%; LC-MS (m/z): 373.16 $(M+H)^+$; $^1H$ NMR (400 MHz, DMSO) $\delta$ 7.67 - 7.56 (m, 4H), 7.24 (d, *J* = 3.1 Hz, 1H), 7.11 (s, 1H), 6.94 (d, *J* = 8.8 Hz, 3H), 6.53 (dd, *J* = 3.3, 1.8 Hz, 1H), 3.77 (s, 3H), 3.70 (s, 2H), 2.30 (s, 3H).

4-Chloro-*N*-2-(furan-2-yl)-5-methlamino)methyl)phenyl)benzenesulfonamide (**$I_A$-7**)

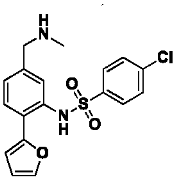

$I_A$-7

HPLC: 99.4%; LC-MS (m/z): 377.11 $(M+H)^+$; $^1H$ NMR (400 MHz, DMSO) $\delta$ 7.72 - 7.66 (m, 2H), 7.61 (d, *J* = 8.1 Hz, 2H), 7.42 (d, *J* = 8.5 Hz, 3H), 7.28 (s, 1H), 6.80 (d, *J* = 7.5 Hz, 1H), 6.51 (dd, *J* = 3.3, 1.8 Hz, 1H), 3.87 (s, 2H), 2.44 (s, 3H).

2-Trifluoromethoxy-N (2-(furan-2-yl)-5-((methylamino)methyl)phenyl) benzenesulfonamide (**$I_A$-8**)

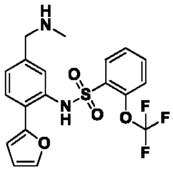

$I_A$-8

HPLC: 96.9%; LC-MS (m/z): 427.10 $(M+H)^+$; $^1H$ NMR (400 MHz, DMSO) $\delta$ 7.93 - 7.87 (m, 1H), 7.64 - 7.56 (m, 2H), 7.53 (s, 1H), 7.46 (t, *J* = 7.1 Hz, 1H), 7.35 - 7.24 (m, 3H), 6.73 (d, *J* = 7.3 Hz, 1H), 6.47 (dd, *J* = 3.2, 1.8 Hz, 1H), 3.85 (s, 2H), 2.43 (s, 3H).

4-Trifluoromethoxy-N (2-(furan-2-yl)-5-((methylamino)methyl)phenyl) benzenesulfonamide (**$I_A$-9**)

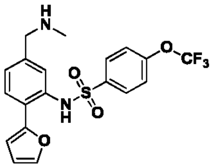

$I_A$-9

HPLC: 98.8%; LC-MS (m/z): 427.19 $(M+H)^+$; $^1H$ NMR (400 MHz, DMSO) $\delta$ 7.82 - 7.75 (m, 2H), 7.61 (dd, *J* = 11.1, 4.5 Hz, 2H), 7.34 (t, *J*= 10.5 Hz, 3H), 7.30 (d, *J* = 1.5 Hz, 1H), 6.89 (d, *J*= 7.5 Hz, 1H), 6.50 (dd, *J* = 3.3, 1.8 Hz, 1H), 3.90 (s, 2H), 2.42 (s, 3H).

4-Methyl-*N*-(2-(furan-2-yl)-5-((methylamino)methyl)phenyl)benzenesulfonamide (**$I_A$-10**)

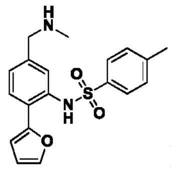

$I_A$-10

HPLC: 98.7%; LC-MS (m/z): 357.13 $(M+H)^+$; $^1H$ NMR (400 MHz, DMSO) $\delta$ 7.62 (d, *J* = 1.0 Hz, 1H), 7.59 (dd, *J* = 8.1, 4.0 Hz, 3H), 7.29 (d, *J* = 3.2 Hz, 1H), 7.20 (d, *J* = 8.0 Hz, 2H), 7.16 (d, *J* = 1.2 Hz, 1H), 6.88 (d, *J* = 7.9 Hz, 1H), 6.52 (dd, *J* = 3.3, 1.8 Hz, 1H), 3.74 (s, 2H), 2.33 (s, 3H), 2.30 (s, 3H).

2,4-Difluoro-*N*-(2-(furan-2-yl)-5-((methylamino)methyl)phenyl)benzenesulfonamide (**$I_A$-11**)

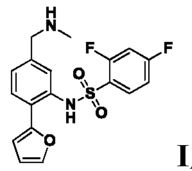

$I_A$-11

HPLC: 99.6%; LC-MS (m/z): 379.10 $(M+H)^+$; $^1H$ NMR (400 MHz, DMSO) $\delta$ 7.72 (dd, *J* = 15.3, 8.4 Hz, 1H), 7.63 (dd, *J* = 13.7, 4.5 Hz, 2H), 7.36 - 7.22 (m, 3H), 7.06 (t, *J* = 8.4 Hz, 2H), 6.50 (dd, *J* = 3.3, 1.8 Hz, 1H), 3.95 (s, 2H), 2.47 (s, 3H).

2,6-Difluoro-*N*-(2-(furan-2-yl)-5-((methylamino)methyl)phenyl)benzenesulfonamide (**$I_A$-12**)

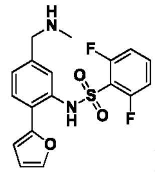

$I_A$-12

HPLC: 99.1%; LC-MS (m/z): 379.11 $(M+H)^+$; $^1H$ NMR (400 MHz, DMSO) $\delta$ 7.51 (dd, *J* = 10.2, 5.4 Hz, 3H), 7.32 - 7.24 (m, 2H), 6.91 (t, *J* = 8.3 Hz, 2H), 6.72 (d, *J* = 8.1 Hz, 1H), 6.48 - 6.39 (m, 1H), 3.73 (s, 2H), 2.33 (s, 3H).

4-Trifluoromethyl-N (2-(furan-2-yl)-5-((methylamino)methyl)phenyl)benzenesulfonamide (**$I_A$-13**)

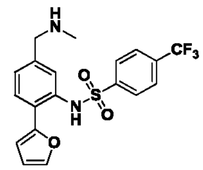

$I_A$-13

HPLC: 98.5%; LC-MS (m/z): 411.10 $(M+H)^+$; $^1H$ NMR (400 MHz, DMSO) $\delta$ 7.88 (d, *J* = 8.1 Hz, 2H), 7.74 (d, *J* = 8.3 Hz, 2H), 7.61 (dd, *J* = 14.2, 4.5 Hz, 2H), 7.33 (t, *J* = 4.8 Hz, 2H), 6.90 (s, 1H), 6.49 (dd, *J* = 3.3, 1.8 Hz, 1H), 3.92 (s, 2H), 2.44 (s, 3H).

N (2-(furan-2-yl)-5-((methylamino)methyl)phenyl)naphthalene-2-sulfonamide (**$I_A$-14**)

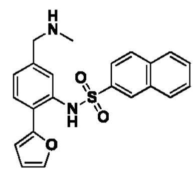

$I_A$-14

HPLC: 97.9%; LC-MS (m/z): 393.13 $(M+H)^+$; $^1H$ NMR (500 MHz, DMSO) $\delta$ 8.33 (s, 1H), 8.00 (d, *J* = 7.2 Hz, 1H), 7.92 (dd, *J* = 7.6, 4.7 Hz, 2H), 7.77 (dd, *J* = 8.6, 1.5 Hz, 1H), 7.64 - 7.53 (m, 4H), 7.38 (s, 1H), 7.27 (s, 1H), 6.84 (d, *J* = 7.6 Hz, 1H), 6.50 (dd, *J* = 3.1, 1.8 Hz, 1H), 3.78 (s, 2H), 2.28 (s, 3H).

*N*-(2-(furan-2-yl)-5-((methylamino)methyl)phenyl)-2,3-dihydrobenzofuran-5-sulfonamide (**$I_A$-15**)

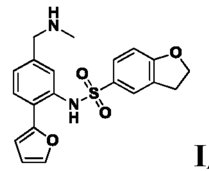

$I_A$-15

HPLC: 97.7%; LC-MS (m/z): 385.14 $(M+H)^+$; $^1H$ NMR (400 MHz, DMSO) $\delta$ 7.67 (d, $J$ = 1.2 Hz, 1H), 7.60 (d, $J$ = 8.0 Hz, 1H), 7.51 (s, 1H), 7.42 (dd, $J$ = 8.4, 1.9 Hz, 1H), 7.19 - 7.11 (m, 2H), 7.05 (t, $J$= 7.8 Hz, 1H), 6.75 (d, $J$ = 8.4 Hz, 1H), 6.54 (dd, $J$ = 3.3, 1.8 Hz, 1H), 4.58 (t, $J$ = 8.8 Hz, 2H), 3.74 (s, 2H), 3.15 (t, $J$ = 8.7 Hz, 2H), 2.32 (s, 3H).

N (2-(furan-2-yl)-5-((methylamino)methyl)phenyl)benzothiazole-6-sulfonamide (**$I_A$-16**)

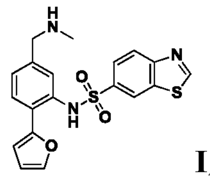

$I_A$-16

HPLC: 99.3%; LC-MS (m/z): 400.07 $(M+H)^+$; $^1H$ NMR (400 MHz, DMSO) $\delta$ 9.46 (d, $J$ = 8.4 Hz, 1H), 8.54 (d, $J$ = 1.4 Hz, 1H), 8.04 (d, $J$ = 8.5 Hz, 1H), 7.84 (dd, $J$ = 8.5, 1.6 Hz, 1H), 7.60 (d, $J$ = 8.1 Hz, 2H), 7.40 (d, $J$ = 18.2 Hz, 1H), 7.29 (s, 1H), 6.81 (s, 1H), 6.50 (dd, $J$ = 3.2, 1.8 Hz, 1H), 3.84 (s, 2H), 2.35 (s, 3H).

*N*-(2-(furan-2-yl)-5-((methylamino)methyl)phenyl)pyridine-3-sulfonamide (**$I_A$-17**)

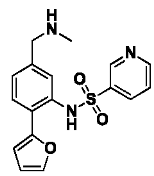

$I_A$-17

HPLC: 99.6%; LC-MS (m/z): 344.11 $(M+H)^+$; $^1H$ NMR (400 MHz, DMSO) $\delta$ 8.83 (s, 1H), 8.53 (d, $J$ = 3.9 Hz, 1H), 8.10 - 7.93 (m, 1H), 7.66 - 7.54 (m, 2H), 7.39 (dd, $J$ = 15.7, 10.9 Hz, 3H), 6.79 (s, 1H), 6.51 (dd, $J$ = 3.2, 1.8 Hz, 1H), 3.91 (s, 2H), 2.46 (s, 3H).

6-Chloro-*N*-(2-(furan-2-yl)-5-((methylamino)methyl)phenyl)pyridine-3-sulfonamide (**$I_A$-18**)

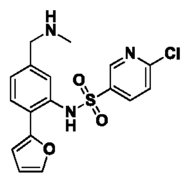

$I_A$-18

HPLC: 98.1%; LC-MS (m/z): 378.06 $(M+H)^+$; $^1H$ NMR (500 MHz, DMSO) $\delta$ 8.55 (d, $J$ = 2.5 Hz, 1H), 8.01 (dd, $J$ = 8.4, 2.6 Hz, 1H), 7.71 (d, $J$ = 8.1 Hz, 1H), 7.67 (d, $J$ = 1.3 Hz, 1H), 7.64 (d, $J$ = 8.4 Hz, 1H), 7.50 (d, $J$ = 7.6 Hz, 1H), 7.32 (d, $J$ = 1.6 Hz, 1H), 6.98 (d, $J$ = 3.1 Hz, 1H), 6.53 (dd, $J$ = 3.4, 1.8 Hz, 1H), 4.09 (s, 2H), 2.50 (s, 3H).

*N*-(2-(furan-2-yl)-5-((methylamino)methyl)phenyl)-1-methyl-1*H*-imidazol-4-sulfonamide (**I$_A$-19**)

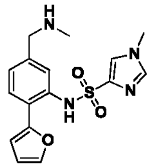

**I$_A$-19**

HPLC: 98.5%; LC-MS (m/z): 347.12 $(M+H)^+$; $^1H$ NMR (400 MHz, DMSO) $\delta$ 7.79 - 7.71 (m, 4H), 7.48 (dd, *J* = 8.2, 1.7 Hz, 1H), 7.34 (d, *J* = 1.5 Hz, 1H), 7.13 (t, *J* = 5.4 Hz, 1H), 6.62 (dd, *J* = 3.4, 1.8 Hz, 1H), 4.04 (s, 2H), 3.70 - 3.64 (m, 3H), 2.50 (s, 3H).

*N*-(2-(furan-2-yl)-5-((methylamino)methyl)phenyl)thiophene-2-sulfonamide (**I$_A$-20**)

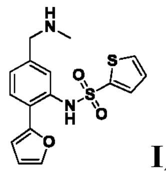

**I$_A$-20**

HPLC: 98.9%; LC-MS (m/z): 349.06 $(M+H)^+$; $^1H$ NMR (400 MHz, DMSO) $\delta$ 7.66 - 7.58 (m, 2H), 7.52 (d, *J* = 4.7 Hz, 1H), 7.45 (s, 1H), 7.40 (d, *J* = 1.4 Hz, 1H), 7.31 - 7.24 (m, 1H), 6.95 - 6.87 (m, 1H), 6.82 (d, *J* = 6.9 Hz, 1H), 6.52 (dd, *J* = 3.3, 1.8 Hz, 1H), 3.91 (s, 2H), 2.46 (s, 3H).

*N*-(2-(furan-2-yl)-5-((methylamino)methyl)phenyl)thiophene-3-sulfonamide (**I$_A$-21**)

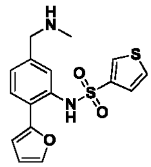

**I$_A$-21**

HPLC: 99.0%; LC-MS (m/z): 349.08 $(M+H)^+$; $^1H$ NMR (400 mhz, DMSO) $\delta$ 7.96 (d, *J* = 1.9 Hz, 1H), 7.68 (d, *J* = 7.9 Hz, 2H), 7.58 (dd, *J* = 5.1, 3.0 Hz, 1H), 7.26 (s, 1H), 7.18 (dd, *J* = 5.1, 1.2 Hz, 3H), 6.55 (dd, *J* = 3.4, 1.8 Hz, 1H), 3.94 (s, 2H), 2.45 (s, 3H).

5-Chloro-*N*-(2-(furan-2-yl)-5-((methylamino)methyl)phenyl)thiophene-2-sulfonamide (**I$_A$-22**)

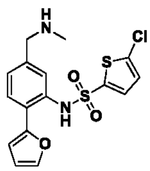

**I$_A$-22**

HPLC: 96.9%; LC-MS (m/z): 383.03 $(M+H)^+$; $^1H$ NMR (400 mhz, DMSO) $\delta$ 7.74 - 7.65 (m, 2H), 7.40 (s, 1H), 7.32 (d, *J* = 1.6 Hz, 1H), 7.23 (d, *J* = 4.0 Hz, 1H), 7.08 (d, *J* = 4.0 Hz, 1H), 6.98 (s, 1H), 6.53 (dd, *J* = 3.4, 1.8 Hz, 1H),

4.06 (s, 2H),2.50 (s, 3H).

5-Bromo-N-(2-(furan-2-yl)-5-((methylamino)methyl)phenyl)thiophene-2-sulfonamide (**$I_A$**-23)

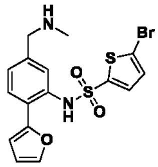

$I_A$-23

HPLC: 97.4%; LC-MS (m/z): 426.99 $(M+H)^+$; $^1H$ NMR (400 mhz, DMSO) $\delta$ 7.73 - 7.58 (m, 2H), 7.30 (d, *J* = 22.8 Hz, 2H), 7.14 (d, *J* = 5.6 Hz, 3H), 6.52 (d, *J* = 1.7 Hz, 1H), 4.02 (s, 2H), 2.50 (s, 3H).

*N*-(2-(furan-2-yl)-5-((methylamino)methyl)phenyl)-1-phenylmethanesulfonamide (**$I_A$-24**)

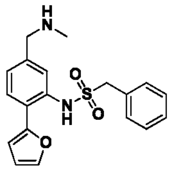

$I_A$-24

HPLC: 97.8%; LC-MS (m/z): 357.16 $(M+H)^+$; $^1H$ NMR (400 mhz, DMSO) $\delta$ 7.68 - 7.61 (m, 2H), 7.33 - 7.24 (m, 6H), 7.13 (d, *J* = 3.4 Hz, 1H), 7.03 (d, *J* = 7.9 Hz, 1H), 6.51 (dd, *J* = 3.2, 1.8 Hz, 1H), 4.31 (s, 2H), 3.81 (s, 2H), 2.42 (s, 3H).

N (2-(furan-2-yl)-5-(2,2,2-trifluoroethyl)amino)methyl)phenyl)benzenesulfonamide (**$I_A$-25**)

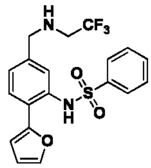

$I_A$-25

HPLC: 99.3%; LC-MS (m/z): 411.10 $(M+H)^+$; $^1H$ NMR (400 mhz, DMSO) $\delta$ 7.72 (dd, *J* = 4.4, 2.6 Hz, 3H), 7.69 - 7.61 (m, 2H), 7.55 (t, *J* = 7.6 Hz, 2H), 7.29 (d, *J* = 8.0 Hz, 1H), 6.97 (d, *J* = 3.3 Hz, 1H), 6.90 (s, 1H), 6.58 (dd, *J* = 3.3, 1.8 Hz, 1H), 3.68 (d, *J* = 3.5 Hz, 2H), 3.10 (dt, *J* = 15.5, 7.8 Hz, 2H).

*N*-(2-(furan-2-yl)-4-((methylamino)methyl)phenyl)benzenesulfonamide (**$I_A$-26**)

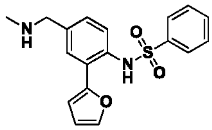

$I_A$-26

HPLC: 98.8%; LC-MS (m/z): 343.16 $(M+H)^+$; $^1H$ NMR (400 mhz, DMSO) $\delta$ 7.77 - 7.62 (m, 4H), 7.44 (d, *J* = 7.3 Hz, 3H), 7.33 (s, 1H), 7.06 (d, *J* = 7.8 Hz, 2H), 6.56 (dd, *J* = 3.3, 1.8 Hz, 1H), 3.96 (s, 2H), 2.50(s, 3H).

4-Fluoro-*N*-(2-(furan-2-yl)-4-((methylamino)methyl)phenyl)benzenesulfonamide (**$I_A$-27**)

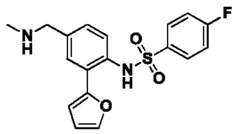

**$I_A$-27**

HPLC: 99.2%; LC-MS (m/z): 361.14 $(M+H)^+$; $^1H$ NMR (500 mhz, meod) $\delta$ 7.67 (ddd, *J* = 8.1, 4.9, 2.5 Hz, 3H), 7.56 (d, *J* = 1.7 Hz, 1H), 7.39 (d, *J* = 8.3 Hz, 1H), 7.27 (dd, *J* = 8.3, 2.1 Hz, 1H), 7.14 - 7.09 (m, 2H), 6.83 (d, *J* = 3.4 Hz, 1H), 6.49 (dd, *J* = 3.4, 1.8 Hz, 1H), 3.98 (s, 2H), 2.59 (s, 3H).

2-Fluoro-*N*-(2-(furan-2-yl)-4-((methylamino)methyl)phenyl)benzenesulfonamide (**$I_A$-28**)

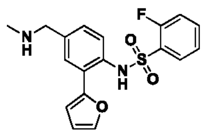

**$I_A$-28**

HPLC: 97.0%; LC-MS (m/z): 361.09 $(M+H)^+$; $^1H$ NMR (400 mhz, DMSO) $\delta$ 7.70 - 7.64 (m, 2H), 7.56 (s, 1H), 7.53 (s, 1H), 7.39 - 7.31 (m, 1H), 7.16 (d, *J* = 8.6 Hz, 1H), 7.10 (t, *J* = 8.0 Hz, 2H), 6.87 (d, *J* = 8.4 Hz, 1H), 6.49 - 6.46 (m, 1H), 3.89 (s, 2H), 2.45 (s, 3H).

*N*-(2-(furan-2-yl)-4-((methylamino)methyl)phenyl)thiophene-2-sulfonamide (**$I_A$-29**)

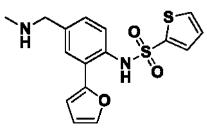

**$I_A$-29**

HPLC: 98.1%; LC-MS (m/z): 349.07 $(M+H)^+$; $^1H$ NMR (400 mhz, DMSO) $\delta$ 7.74 (d, *J* = 1.9 Hz, 1H), 7.65 - 7.57 (m, 2H), 7.34 - 7.25 (m, 2H), 7.16 (d, *J* = 8.3 Hz, 1H), 7.08 (d, *J* = 7.8 Hz, 1H), 6.99 - 6.93 (m, 1H), 6.52 (dd, *J* = 3.2, 1.8 Hz, 1H), 3.97 (s, 2H), 2.5(s, 3H).

*N*-(2-(furan-2-yl)-4-((methylamino)methyl)phenyl)thiophene-3-sulfonamide (**$I_A$-30**)

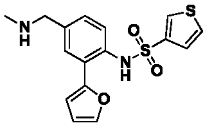

**$I_A$-30**

HPLC: 98.4%; LC-MS (m/z): 349.09 $(M+H)^+$; H NMR (500 mhz, $cdcl_3$) $\delta$ 7.70 - 7.65 (m, 1H), 7.62 (d, *J*= 8.3 Hz, 1H), 7.48 (dt, *J* = 5.1, 2.6 Hz, 1H), 7.37 (d, *J* = 1.9 Hz, 1H), 7.25 (dd, *J* = 8.3, 2.0 Hz, 1H), 7.18 - 7.12 (m, 1H), 6.98 - 6.92 (m, 1H), 6.43 (ddd, *J* = 4.1, 3.4, 1.3 Hz, 2H), 3.72 (s, 2H), 2.45 (s, 3H).

*N*-(2-(furan-2-yl)-4-((methylamino)methyl)phenyl)pyridine-3-sulfonamide (**$I_A$-31**)

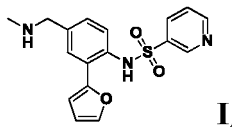

$I_A$-31

HPLC: 98.6%; LC-MS (m/z): 344.11 $(M+H)^+$; $^1H$ NMR (400 mhz, DMSO) $\delta$ 8.80 (s, 1H), 8.51 (d, *J* = 4.3 Hz, 1H), 7.99 (d, *J* = 7.9 Hz, 1H), 7.70 (s, 1H), 7.61 (s, 1H), 7.53 (d, *J* = 2.8 Hz, 1H), 7.38 (dd, *J* = 7.8, 4.9 Hz, 1H), 7.22 (d, *J* = 8.4 Hz, 1H), 6.93 (d, *J* = 8.5 Hz, 1H), 6.53 (s, 1H), 3.93 (s, 2H), 2.49 - 2.48 (m, 3H).

N (4-((ethylamino)methyl)-2-(furan-2-yl)phenyl)thiophene-3-sulfonamide (**$I_A$-32**)

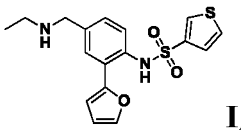

$I_A$-32

HPLC: 99.3%; LC-MS (m/z): 363.09 $(M+H)^+$; $^1H$ NMR (500 mhz, DMSO) $\delta$ 7.77 (s, 1H), 7.70 (d, *J* = 2.1 Hz, 1H), 7.63 (s, 1H), 7.45 (d, *J* = 16.5 Hz, 2H), 7.19 (d, *J* = 8.2 Hz, 1H), 7.14 (d, *J* = 5.0 Hz, 1H), 6.98 (d, *J* = 7.4 Hz, 1H), 6.54 (dd, *J* = 3.1, 1.8 Hz, 1H), 3.92 (s, 2H), 2.87 (q, *J* = 7.1 Hz, 2H), 1.15 (t, *J* = 7.2 Hz, 3H).

N (4-((cyclopropylamino)methyl)-2-(furan-2-yl)phenyl)thiophene-3-sulfonamide (**$I_A$-33**)

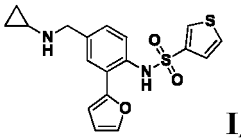

$I_A$-33

HPLC: 99.4%; LC-MS (m/z): 375.09 $(M+H)^+$; $^1H$ NMR (500 mhz, DMSO) $\delta$ 7.98 (d, *J* = 1.8 Hz, 1H), 7.72 (d, *J* = 1.4 Hz, 1H), 7.67 (dd, *J* = 4.9, 2.8 Hz, 2H), 7.21 (dd, *J* = 5.1, 1.2 Hz, 1H), 7.11 (dd, *J* = 8.2, 1.9 Hz, 1H), 7.06 (d, *J* = 2.8 Hz, 1H), 6.88 (d, *J* = 8.1 Hz, 1H), 6.57 (dd, *J* = 3.4, 1.8 Hz, 1H), 3.75 (s, 2H), 2.10 (s, 1H), 0.35 (dd, *J* = 32.6, 11.5 Hz, 4H).

*N*-(4-((cyclobutylamino)methyl)-2-(furan-2-yl)phenyl)thiophene-3-sulfonamide (**$I_A$-34**)

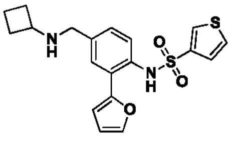

$I_A$-34

HPLC: 96.9%; LC-MS (m/z): 389.09 $(M+H)^+$; $^1H$ NMR (500 mhz, $cdcl_3$) $\delta$ 7.69 - 7.64 (m, 1H), 7.61 (d, *J* = 8.3 Hz, 1H), 7.47 (d, *J* = 5.5 Hz, 1H), 7.38 (s, 1H), 7.24 (d, *J* = 1.9 Hz, 1H), 7.16 (dd, *J* = 5.1, 3.1 Hz, 1H), 6.97 - 6.94 (m, 1H), 6.46 - 6.39 (m, 2H), 3.68 (s, 2H), 3.32 - 3.24 (m, 1H), 2.25 - 2.14 (m, 2H), 1.69 (dd, *J* = 16.8, 12.4 Hz, 4H).

N (5-((methylamino)methyl)-2-(5-methylfuran-2-yl)phenyl)benzenesulfonamide (**I$_A$-35**)

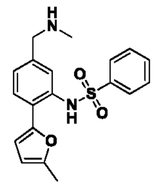

I$_A$-35

HPLC: 96.8%; LC-MS (m/z): 357.12 $(M+H)^+$; $^1H$ NMR (400 mhz, DMSO) $\delta$ 7.70 - 7.64 (m, 2H), 7.57 (d, *J* = 8.0 Hz, 1H), 7.48 - 7.38 (m, 3H), 7.18 (s, 1H), 7.11 (s, 1H), 6.96 (d, *J* = 8.0 Hz, 1H), 6.11 (d, *J* = 2.2 Hz, 1H), 3.80 (s, 2H), 2.37 (s, 3H), 2.27 (d, *J* = 15.0 Hz, 3H).

N (3-(furan-2-yl)-4-((methylamino)methyl)phenyl)benzenesulfonamide (**I$_A$-36**)

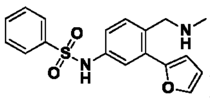

I$_A$-36

HPLC: 97.5%; LC-MS (m/z): 343.13 $(M+H)^+$; $^1H$ NMR (400 mhz, $CD_3OD$) $\delta$ 7.83 - 7.78 (m, 2H), 7.69 (dd, *J* = 1.8, 0.7 Hz, 1H), 7.57 - 7.52 (m, 1H), 7.49 - 7.44 (m, 2H), 7.41 (d, *J* = 2.3 Hz, 1H), 7.33 (d, *J* = 8.3 Hz, 1H), 7.13 (dd, *J* = 8.3, 2.3 Hz, 1H), 6.71 (dd, *J* = 3.4, 0.7 Hz, 1H), 6.59 (dd, *J* = 3.5, 1.9 Hz, 1H), 4.19 (s, 2H), 2.66 (s, 3H).

*N*-(4-(furan-2-yl)-3-((methylamino)methyl)phenyl)benzenesulfonamide (**I$_A$-37**)

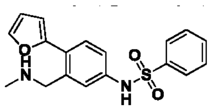

I$_A$-37

HPLC: 99.2%; LC-MS (m/z): 343.03 $(M+H)^+$; $^1H$ NMR (400 mhz, DMSO) $\delta$ 7.78 - 7.71 (m, 2H), 7.69 (dd, *J* = 1.8, 0.7 Hz, 1H), 7.59 - 7.47 (m, 3H), 7.44 (d, *J* = 8.5 Hz, 1H), 7.26 (d, *J* = 2.3 Hz, 1H), 7.02 (dd, *J* = 8.5, 2.4 Hz, 1H), 6.62 (dd, *J* = 3.4, 0.6 Hz, 1H), 6.53 (dd, *J* = 3.4, 1.8 Hz, 1H), 3.68 (s, 2H), 2.21 (s, 3H).

*N*-(4-(furan-2-yl)-2-((methylamino)methyl)phenyl)benzenesulfonamide (**I$_A$-38**)

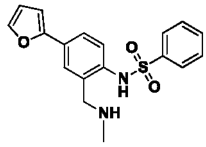

I$_A$-38

HPLC: 97.6%; LC-MS (m/z): 343.06 $(M+H)^+$; $^1H$ NMR (400 mhz, DMSO) $\delta$ 7.82 - 7.73 (m, 2H), 7.61 (d, *J* = 1.1 Hz, 1H), 7.42 (dd, *J* = 10.6, 5.2 Hz, 4H), 7.34 (dd, *J* = 8.5, 2.1 Hz, 1H), 7.11 (d, *J* = 8.6 Hz, 1H), 6.60 (d, *J* = 3.2 Hz, 1H), 6.50 (dd, *J* = 3.3, 1.8 Hz, 1H), 3.96 (s, 2H), 2.47 (s, 3H).

*N*-(3-(furan-2-yl)-5-((methylamino)methyl)phenyl)benzenesulfonamide (**I$_A$-39**)

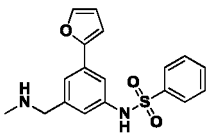

**I$_A$-39**

HPLC: 98.4%; LC-MS (m/z): 343.17 $(M+H)^+$; $^1$H NMR (500 mhz, DMSO) $\delta$ 7.83 - 7.78 (m, 2H), 7.76 (d, *J* = 1.7 Hz, 1H), 7.60 (dt, *J* = 5.0, 4.4 Hz, 1H), 7.55 (t, *J* = 7.4 Hz, 2H), 7.46 (s, 1H), 7.36 (t, *J* = 1.6 Hz, 1H), 7.12 (s, 1H), 6.80 (d, *J* = 3.4 Hz, 1H), 6.59 (dd, *J* = 3.4, 1.8 Hz, 1H), 3.91 (s, 2H), 2.40 (s, 3H).

*N*-(5-(furan-2-yl)-2-((methylamino)methyl)phenyl)benzenesulfonamide (**I$_A$-40**)

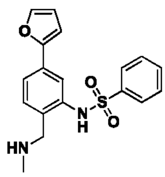

**I$_A$-40**

HPLC: 99.0%; LC-MS (m/z): 343.14 $(M+H)^+$; $^1$H NMR (400 mhz, DMSO) $\delta$ 7.80 (dd, *J* = 6.6, 3.0 Hz, 2H), 7.68 (d, *J* = 1.2 Hz, 1H), 7.48 - 7.39 (m, 4H), 7.09 (d, *J* = 7.8 Hz, 1H), 6.98 (d, *J* = 7.5 Hz, 1H), 6.65 (d, *J* = 3.1 Hz, 1H), 6.53 (dd, *J* = 3.3, 1.8 Hz, 1H), 3.90 (s, 2H), 2.44 (s, 3H).

4-Fluoro-N (2-(furan-3-yl)-5-((methylamino)methyl)phenyl)benzenesulfonamide (**I$_A$-41**)

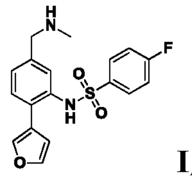

**I$_A$-41**

HPLC: 96.7%; LC-MS (m/z): 361.20 $(M+H)^+$; $^1$H NMR (400 mhz, DMSO) $\delta$ 7.95 (s, 1H), 7.66 (dd, *J* = 7.0, 3.5 Hz, 3H), 7.57 (d, *J* = 7.3 Hz, 1H), 7.51 (d, *J* = 8.0 Hz, 1H), 7.31 (t, *J* = 8.8 Hz, 2H), 7.17 (s, 1H), 6.73 (s, 1H), 4.02 (s, 2H), 2.44 (s, 3H).

*N*-(2-(furan-3-yl)-5-((methylamino)methyl)phenyl)benzenesulfonamide (**I$_A$-42**)

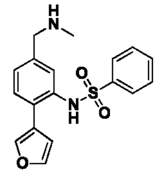

**I$_A$-42**

HPLC: 97.7%; LC-MS (m/z): 343.10 $(M+H)^+$; $^1$H NMR (400 mhz, DMSO) $\delta$ 8.32 (s, 1H), 7.72 - 7.64 (m, 2H), 7.62 (t, *J* = 1.7 Hz, 1H), 7.50 - 7.35 (m, 4H), 7.11 (s, 1H), 6.91 (d, *J* = 7.4 Hz, 1H), 6.84 (s, 1H), 3.73 (s, 2H), 2.32 (s, 3H).

*N*-(2-(furan-3-yl)-5-((methylamino)methyl)phenyl)thiophene-3-sulfonamide (**$I_A$-43**)

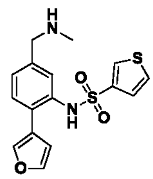

$I_A$-43

HPLC: 97.9%; LC-MS (m/z): 349.08 $(M+H)^+$; $^1H$ NMR (400 mhz, DMSO) $\delta$ 7.97 (d, *J* = 1.7 Hz, 1H), 7.93 (s, 1H), 7.65 (d, *J* = 1.6 Hz, 1H), 7.62 (dd, *J* = 5.2, 2.9 Hz, 1H), 7.50 (d, *J* = 8.1 Hz, 1H), 7.34 (s, 1H), 7.18 (s, 1H), 7.12 (dd, *J* = 5.1, 1.4 Hz, 1H), 6.72 (s, 1H), 4.00 (s, 1H), 2.50 (s, 3H).

*N*-(2-(furan-3-yl)-5-((methylamino)methyl)phenyl)thiophene-2-sulfonamide (**$I_A$-44**)

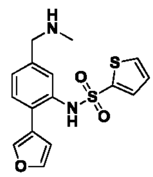

$I_A$-44

HPLC: 96.6%; LC-MS (m/z): 349.07 $(M+H)^+$; $^1H$ NMR (400 mhz, $CD_3OD$) $\delta$ 7.70 (s, 1H), 7.63 (dd, *J* = 5.0, 1.3 Hz, 1H), 7.46 (t, *J* = 1.7 Hz, 1H), 7.41 (dt, *J* = 7.8, 4.1 Hz, 2H), 7.35 (dd, *J* = 3.7, 1.3 Hz, 1H), 7.22 (dd, *J* = 8.0, 1.8 Hz, 1H), 7.00 (dd, *J* = 5.0, 3.7 Hz, 1H), 6.50 (dd, *J* = 1.9, 0.8 Hz, 1H), 3.99 (s, 2H), 2.58 (s, 3H).

4-Fluoro-N (5-((methylamino)methyl)-2-(thiophene-2-yl)phenyl)benzenesulfonamide (**$I_A$**-45)

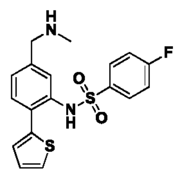

$I_A$-45

HPLC: 98.5%; LC-MS (m/z): 377.11 $(M+H)^+$; $^1H$ NMR (400 mhz, DMSO) $\delta$ 7.66 (dd, *J* = 8.6, 5.3 Hz, 2H), 7.60 (t, *J* = 6.9 Hz, 2H), 7.49 (d, *J* = 8.2 Hz, 1H), 7.38 (d, *J* = 3.3 Hz, 1H), 7.32 (t, *J* = 8.8 Hz, 2H), 7.15 (s, 1H), 7.10 - 7.03 (m, 1H), 4.04 (s, 2H), 2.48 (s, 3H).

*N*-(2-(thiophene-2-yl)-5-((methylamino)methyl)phenyl)benzenesulfonamide (**$I_A$-46**)

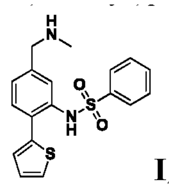

$I_A$-46

HPLC: 99.2%; LC-MS (m/z): 359.08 $(M+H)^+$; $^1H$ NMR (400 mhz, DMSO) $\delta$ 7.72 (dd, *J* = 7.8, 1.5 Hz, 2H), 7.61 (d, *J* = 8.0 Hz, 1H), 7.50 (d, *J* = 3.2 Hz, 1H), 7.45 - 7.34 (m, 4H), 7.26 (s, 1H), 7.02 (dd, *J* = 5.1, 3.8 Hz, 1H), 6.86 (s,

1H), 3.85 (s, 2H), 2.40 (s, 3H).

*N*-(2-(thiophene-2-yl)-5-((methylamino)methyl)phenyl)thiophene-2-sulfonamide (**$I_A$-47**)

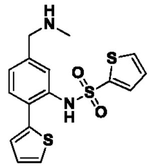

$I_A$-47

HPLC: 98.9%; LC-MS (m/z): 365.05 $(M+H)^+$; $^1H$ NMR (400 mhz, CD3OD) $\delta$ 7.67 (dd, *J* = 5.0, 1.3 Hz, 1H), 7.60 (d, *J* = 1.7 Hz, 1H), 7.50 (d, *J* = 8.0 Hz, 1H), 7.42 (dd, *J* = 5.1, 1.2 Hz, 1H), 7.36 (dd, *J* = 3.8, 1.4 Hz, 1H), 7.33 - 7.29 (m, 1H), 7.02 (ddd, *J* = 5.3, 3.7, 1.8 Hz, 2H), 6.99 - 6.93 (m, 1H), 4.14 (s, 2H), 2.68 (s, 3H).

*N*-(2-(thiophene-2-yl)-5-((methylamino)methyl)phenyl)thiophene-3-sulfonamide (**$I_A$-48**)

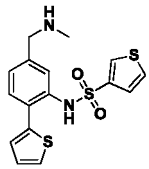

$I_A$-48

HPLC: 97.4%; LC-MS (m/z): 365.07 $(M+H)^+$; $^1H$ NMR (400 mhz, CD3OD) $\delta$ 7.87 (dd, *J* = 3.1, 1.3 Hz, 1H), 7.56 (d, *J* = 1.7 Hz, 1H), 7.51 - 7.40 (m, 3H), 7.31 (dd, *J* = 8.0, 1.9 Hz, 1H), 7.08 (dd, *J* = 5.2, 1.3 Hz, 1H), 7.02 (ddd, *J* = 4.9, 4.3, 2.4 Hz, 2H), 4.12 (s, 2H), 2.66 (s, 3H).

N (2-(thiophene-3-yl)-5-((methylamino)methyl)phenyl)benzenesulfonamide (**$I_A$-49**)

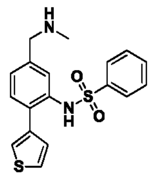

$I_A$-49

HPLC: 96.7%; LC-MS (m/z): 359.08 $(M+H)^+$; $^1H$ NMR (400 mhz, DMSO) $\delta$ 7.68 (d, *J* = 1.9 Hz, 1H), 7.64 - 7.59 (m, 2H), 7.54 - 7.47 (m, 2H), 7.43 (t, *J* = 7.5 Hz, 2H), 7.34 (d, *J* = 7.8 Hz, 1H), 7.29 (d, *J* = 5.0 Hz, 1H), 7.10 (s, 1H), 7.05 (d, *J* = 7.9 Hz, 1H), 3.70 (s, 2H), 2.29 (s, 3H).

*N*-(5-((methylamino)methyl)-2-(1*H*-pyrrole-2-yl)phenyl)benzenesulfonamide (**$I_A$-50**)

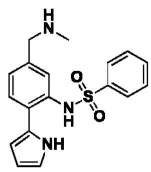

$I_A$-50

HPLC: 98.8%; LC-MS (m/z): 342.12 $(M+H)^+$; $^1H$ NMR (400 mhz, DMSO) $\delta$ 7.79 - 7.69 (m, 2H), 7.50 (d, *J* = 8.0 Hz, 1H), 7.44 - 7.31 (m, 3H), 7.24 (s, 1H), 6.81 (s, 2H), 6.46 (d, *J* = 2.6 Hz, 1H), 6.06 (s, 1H), 3.84 (s, 2H), 2.39 (s, 3H).

N (5-((methylamino)methyl)-2-(thiazol-2-yl)phenyl)benzenesulfonamide (**$I_A$-51**)

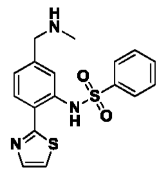

**$I_A$-51**

HPLC: 98.1%; LC-MS (m/z): 360.10 $(M+H)^+$; $^1H$ NMR (400 mhz, DMSO) $\delta$ 8.20 (d, *J* = 8.1 Hz, 1H), 7.85 (t, *J* = 4.4 Hz, 1H), 7.79 (dt, *J* = 7.5, 3.9 Hz, 2H), 7.56 (dd, *J* = 7.8, 3.7 Hz, 2H), 7.41 - 7.32 (m, 3H), 6.72 (t, *J* = 23.0 Hz, 1H), 3.94 (s, 2H), 2.47 (s, 3H).

*N*-(5-(methylamino)methyl)-2-(1*H*-pyrazol-4-yl)phenyl)benzenesulfonamide (**$I_A$-52**)

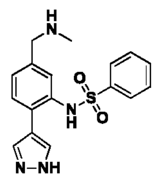

**$I_A$-52**

HPLC: 99.4%; LC-MS (m/z): 343.12 $(M+H)^+$; $^1H$ NMR (400 mhz, DMSO) $\delta$ 7.97 (s, 2H), 7.68 (d, *J* = 7.4 Hz, 2H), 7.56 (t, *J* = 7.3 Hz, 1H), 7.48 (t, *J* = 7.1 Hz, 3H), 7.12 (d, *J* = 7.7 Hz, 1H), 7.01 (s, 1H), 3.73 (s, 2H), 2.31 (s, 3H).

N (2-(3,5-dimethylisoxazol-4-yl)-5-((methylamino)methyl)phenyl)benzenesulfonamide (**$I_A$-53**)

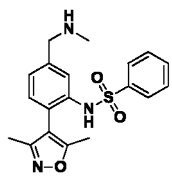

**$I_A$-53**

HPLC: 97.3%; LC-MS (m/z): 372.34 $(M+H)^+$; $^1H$ NMR (400 mhz, DMSO) $\delta$ 7.73 (d, *J* = 6.8 Hz, 2H), 7.58 - 7.45 (m, 3H), 7.09 (d, *J* = 7.7 Hz, 2H), 6.98 (d, *J* = 7.7 Hz, 1H), 3.70 (s, 2H), 2.30 (s, 3H), 2.20 (s, 3H), 2.06 (s, 3H).

*N*-(5-((methylamino)methyl)-2-(1*H*-pyrazol-5-yl)phenyl)benzenesulfonamide (**$I_A$-54**)

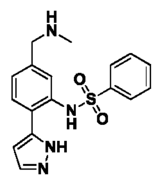

**$I_A$-54**

HPLC: 96.0%; LC-MS (m/z): 343.16 $(M+H)^+$; $^1H$ NMR(400 mhz, DMSO) $\delta$ 7.81 (d, *J* = 2.2 Hz, 1H), 7.73 (d, *J*

= 7.4 Hz, 2H), 7.67 (d, *J* = 8.0 Hz, 1H), 7.56 - 7.49 (m, 2H), 7.44 (t, *J* = 7.5 Hz, 2H), 6.97 (d, *J* = 7.9 Hz, 1H), 6.78 (d, *J* = 2.2 Hz, 1H), 3.72 (s, 2H), 2.29 (s, 3H).

*N*-(5-((methylamino)methyl)-2-(1*H*-pyrazol-3-yl)phenyl)benzenesulfonamide (**$I_A$-55**)

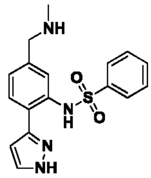

$I_A$-55

HPLC: 97.4%; LC-MS (m/z): 343.19 $(M+H)^+$; $^1H$ NMR (400 mhz, DMSO) $\delta$ 7.81 (d, *J* = 2.0 Hz, 1H), 7.72 (d, *J* = 7.4 Hz, 2H), 7.67 (d, *J* = 8.0 Hz, 1H), 7.55 - 7.48 (m, 2H), 7.44 (t, *J* = 7.5 Hz, 2H), 6.98 (d, *J* = 7.6 Hz, 1H), 6.77 (d, *J* = 2.1 Hz, 1H), 3.73 (s, 2H), 2.29 (s, 3H).

*N*-(2-(1-methyl-1*H*-pyrazole-4-yl)-5-((methylamino)methyl)phenyl)benzenesulfonamide (**$I_A$-56**)

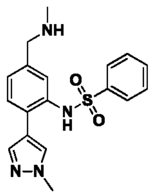

$I_A$-56

HPLC: 97.8%; LC-MS (m/z): 357.13 $(M+H)^+$; $^1H$ NMR (400 mhz, DMSO) $\delta$ 8.05 (s, 1H), 7.77 (s, 1H), 7.69 (d, *J* = 7.2 Hz, 2H), 7.56 (t, *J* = 7.2 Hz, 1H), 7.49 (t, *J* = 7.4 Hz, 2H), 7.43 (d, *J* = 8.3 Hz, 1H), 7.06 (d, *J* = 5.2 Hz, 2H), 3.86 (s, 3H), 3.72 (s, 2H), 2.32 (s, 3H).

*N*-(5-((methylamino)methyl)-2-(1,3,5-trimethyl-1*H*-pyrazole-4-yl)phenyl) benzenesulfonamide (**$I_A$-57**)

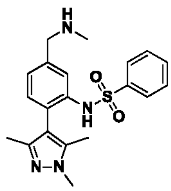

$I_A$-57

HPLC: 98.1%; LC-MS (m/z): 385.16 $(M+H)^+$; $^1H$ NMR (400 mhz, DMSO) $\delta$ 7.77 (d, *J* = 7.3 Hz, 2H), 7.64 (t, *J* = 7.4 Hz, 1H), 7.55 (t, *J* = 7.5 Hz, 2H), 7.20 (d, *J* = 5.7 Hz, 2H), 7.07 (d, *J* = 8.2 Hz, 1H), 3.84 (s, 2H), 3.67 (s, 3H), 2.38 (s, 3H), 1.94 (s, 3H), 1.90 (s, 3H).

*N*-(2-(3,5-dimethyl-1*H*-pyrazole-4-yl)-5-((methylamino)methyl)phenyl) benzenesulfonamide (**$I_A$-58**)

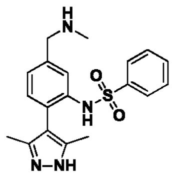

IA-58

HPLC: 98.5%; LC-MS (m/z): 371.15 $(M+H)^+$; $^1H$ NMR (400 mhz, DMSO) $\delta$ 7.82 (t, *J* = 8.6 Hz, 2H), 7.66 (t, *J* = 7.4 Hz, 1H), 7.56 (dd, *J* = 18.5, 11.1 Hz, 2H), 7.28 (t, *J* = 9.2 Hz, 1H), 7.21 (s, 1H), 7.15 (t, *J* = 9.5 Hz, 1H), 3.99 (s, 2H), 2.48 (s, 3H), 1.95 (s, 6H).

*N*-(5-((methylamino)methyl)-2-(5-methylthiazol-2-yl)phenyl)benzenesulfonamide (**$I_A$-59**)

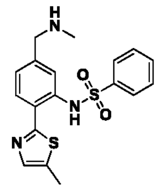

IA-59

HPLC: 99.3%; LC-MS (m/z): 374.19 $(M+H)^+$; $^1H$ NMR (400 mhz, DMSO) $\delta$ 8.09 (t, *J* = 7.6 Hz, 1H), 7.77 (dd, *J* = 6.5, 3.0 Hz, 2H), 7.58 - 7.45 (m, 2H), 7.43 - 7.32 (m, 3H), 6.75 (d, *J* = 7.7 Hz, 1H), 3.90 (s, 2H), 2.47 (s, 3H), 2.44 (s, 3H).

*N*-(2-(1-methyl-1*H*-imidazol-5-yl)-5-((methylamino)methyl)phenyl)benzenesulfonamide (**$I_A$-60**)

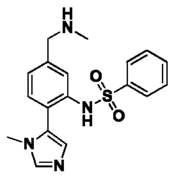

IA-60

HPLC: 99.4%; LC-MS (m/z): 357.13 $(M+H)^+$; $^1H$ NMR (400 mhz, DMSO) $\delta$ 7.64 (dd, *J* = 9.5, 8.2 Hz, 3H), 7.50 (t, *J* = 7.2 Hz, 1H), 7.44 (t, *J* = 7.3 Hz, 2H), 7.28 (d, *J* = 1.1 Hz, 1H), 7.11 (d, *J* = 7.7 Hz, 1H), 6.94 (d, *J* = 7.4 Hz, 1H), 6.67 (d, *J* = 0.7 Hz, 1H), 3.78 (s, 2H), 3.40 - 3.34 (s, 3H), 2.36 (s, 3H).

*N*-(2-(1*H*-imidazol-5-yl)-5-((methylamino)methyl)phenyl)benzenesulfonamide (**$I_A$-61**)

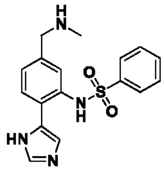

IA-61

HPLC: 98.3%; LC-MS (m/z): 343.13 $(M+H)^+$; $^1H$ NMR (400 mhz, DMSO) $\delta$ 8.00 (s, 1H), 7.69 (d, *J* = 9.0 Hz, 3H), 7.62 (d, *J* = 8.0 Hz, 1H), 7.56 - 7.51 (m, 2H), 7.43 (t, *J* = 7.7 Hz, 2H), 6.99 (t, *J* = 10.5 Hz, 1H), 3.71 (s, 2H), 2.28 (s, 3H).

*N*-(2-(furan-2-yl)-5-(2,2,2-trifluoroethyl)amino)methyl)phenyl)benzenesulfonamide (**$I_A$-62**)

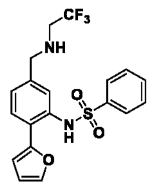

$I_A$-62

HPLC: 99.3%; LC-MS (m/z): 411.35 $(M+H)^+$; $^1H$ NMR (400 mhz, DMSO) $\delta$ 9.66 (s, 1H), 7.72 (dd, *J* = 4.4, 2.6 Hz, 3H), 7.69 - 7.61 (m, 2H), 7.55 (t, *J* = 7.6 Hz, 2H), 7.29 (d, *J* = 8.0 Hz, 1H), 6.97 (d, *J* = 3.3 Hz, 1H), 6.90 (s, 1H), 6.58 (dd, *J* = 3.3, 1.8 Hz, 1H), 3.68 (d, *J* = 3.5 Hz, 2H), 3.10 (dt, *J* = 15.5, 7.8 Hz, 2H), 2.85 (s, 1H).

**Example 2**

**Synthesis of compound $I_C$:**

*N*-(2-(furan-2-yl)-5-((oximido)methyl)phenyl)benzenesulfonamide (IC-1)

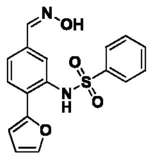

$I_C$-1

Intermediate **VI-4** (65.5mg, 0.2mmol) was dissolved in 10 milliliter of ethanol solution, then 1 milliliter of hydroxylamine aqueous solution (50wt%) was added. The mixture reacted overnight at room temperature. After the reaction was finished, the solvent was evaporated and the residue was separated by column chromatography to obtain compound **$I_C$-1**, as a yellow solid (32.9mg, 47.6%).

HPLC: 99.2%; LC-MS (m/z): 343.13 $(M+H)^+$; $^1H$ NMR (500 MHz, DMSO) $\delta$ 11.33 (s, 1H), 9.74 (s, 1H), 8.01 (s, 1H), 7.74 - 7.66 (m, 4H), 7.62 (t, *J* = 7.4 Hz, 1H), 7.53 (t, *J* = 7.6 Hz, 2H), 7.48 (dd, *J* = 8.2, 1.5 Hz, 1H), 7.20 (d, *J* = 1.5 Hz, 1H), 7.01 (d, *J* = 3.4 Hz, 1H), 6.58 (dd, *J* = 3.4, 1.8 Hz, 1H).

A similar method for synthesizing **Ic-1** was applied to obtain the following compound:

*N*-2-(furan-2-yl)-5-((methoxyimino)methyl)phenyl)benzenesulfonamide (**$I_C$-2**)

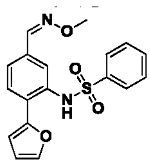

$I_C$-2

HPLC: 97.5%; LC-MS (m/z): 357.01 $(M+H)^+$; $^1H$ NMR (400 MHz, DMSO) $\delta$ 9.76 (s, 1H), 8.02 (d, *J* = 5.7 Hz, 2H), 7.73 (dd, *J* = 19.4, 9.4 Hz, 5H), 7.61 (t, *J* = 7.2 Hz, 1H), 7.52 (t, *J* = 7.6 Hz, 2H), 7.06 (d, *J* = 3.3 Hz, 1H), 6.59 (s, 1H), 3.74 (s, 3H).

## Example 3

### Synthesis of compound $I_D$:

*N*-(5-(aminomethyl)-2-(furan-2-yl)phenyl)benzenesulfonamide (**$I_D$-1**)

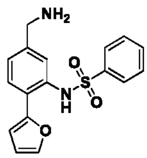

$I_D$-1

Intermediate **V-4** (65.5mg, 0.2mmol) was dissolved in 5 milliliter of ammonia-methanol solution. An appropriate amount of Raney nickel aqueous solution was added into the reaction system, then the hydrogen gas was introduced. The mixture reacted overnight at room temperature. Post-treatment: Raney nickel was removed by suction filtration, the filtrate was evaporated to dryness, and the residue was separated by column chromatography to obtain intermediate **$I_D$-1**, as a white solid (44.3mg, 63.8%).
HPLC: 99%; LC-MS (m/z): 329.10 $(M+H)^+$; $^1H$ NMR (400 MHz, DMSO) $\delta$ 7.71 (dd, *J* = 7.7, 1.4 Hz, 2H), 7.61 (d, *J* = 8.1 Hz, 2H), 7.39 (q, *J* = 6.2 Hz, 3H), 7.29 (s, 2H), 6.90 (d, *J* = 7.2 Hz, 1H), 6.50 (dd, *J* = 3.1, 1.8 Hz, 1H), 3.81 (s, 2H).
Similar methods for synthesizing **$I_D$-1** were applied to obtain the following compounds:

*N*-(5-(aminomethyl)-2-(furan-2-yl)phenyl)-4-fluorobenzenesulfonamide (**$I_D$-2**)

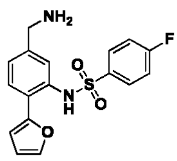

$I_D$-2

HPLC: 98.7%; LC-MS (m/z): 347.09 $(M+H)^+$; $^1H$ NMR (400 MHz, DMSO) $\delta$ 7.74 (dd, *J* = 8.7, 5.6 Hz, 2H), 7.60 (d, *J* = 8.7 Hz, 2H), 7.36 (s, 1H), 7.30 (s, 1H), 7.18 (t, *J* = 8.9 Hz, 2H), 6.83 (d, *J* = 7.6 Hz, 1H), 6.50 (dd, *J* = 3.2, 1.8 Hz, 1H), 3.81 (s, 2H).

*N*-5-(aminomethyl)-2-(furan-2-yl)phenyl)thiophene-3-sulfonamide (**$I_D$-3**)

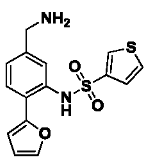

$I_D$-3

HPLC: 99.3%; LC-MS (m/z) 335.04 $(M+H)^+$; $^1H$ NMR (400 MHz, DMSO) $\delta$ 7.81 - 7.75 (m, 1H), 7.59 (d, *J* = 8.0 Hz, 2H), 7.47 - 7.41 (m, 2H), 7.36 (s, 1H), 7.15 (dd, *J* = 5.0, 1.1 Hz, 1H), 6.74 (d, *J* = 8.1 Hz, 1H), 6.50 (dd, *J* = 3.2, 1.8 Hz, 1H), 3.79 (s, 2H).

*N*-(4-(aminomethyl)-2-(furan-2-yl)phenyl)benzenesulfonamide (**$I_D$-4**)

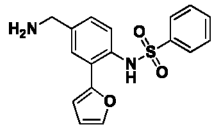

$I_D$-4

HPLC: 98.2%; LC-MS (m/z): 329.15 $(M+H)^+$; $^1H$ NMR (400 MHz, DMSO) $\delta$ 7.70 - 7.64 (m, 3H), 7.60 (d, *J* = 0.9 Hz, 1H), 7.54 (d, *J* = 3.1 Hz, 1H), 7.37 - 7.31 (m, 3H), 7.14 (d, *J* = 8.4 Hz, 1H), 6.87 (dd, *J* = 8.5, 2.2 Hz, 1H), 6.52 (dd, *J* = 3.2, 1.8 Hz, 1H), 3.82 (s, 2H).

*N*-(4-(aminomethyl)-2-(furan-2-yl)phenyl)thiophene-3-sulfonamide (**$I_D$-5**)

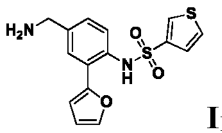

$I_D$-5

HPLC: 97.5%; LC-MS (m/z): 335.06 $(M+H)^+$; $^1H$ NMR (400 MHz, DMSO) $\delta$ 8.09 (d, *J* = 1.8 Hz, 1H), 7.87 (d, *J* = 1.5 Hz, 1H), 7.80 (s, 1H), 7.75 (dd, *J* = 5.0, 3.0 Hz, 1H), 7.33 (dd, *J* = 8.2, 1.5 Hz, 1H), 7.29 (d, *J* = 4.3 Hz, 1H), 7.05 (d, *J* = 3.3 Hz, 1H), 6.95 (d, *J* = 8.2 Hz, 1H), 6.64 (dd, *J* = 3.2, 1.7 Hz, 1H), 4.02 (d, *J* = 3.7 Hz, 2H).

*N*-(4-(aminomethyl)-2-(furan-2-yl)phenyl)-4-fluorobenzenesulfonamide (**$I_D$-6**)

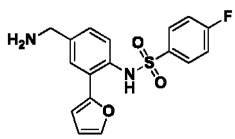

$I_D$-6

HPLC: 98.9%; LC-MS (m/z): 347.11 $(M+H)^+$; $^1H$ NMR (400 MHz, DMSO) $\delta$ 7.67 (dt, *J* = 11.6, 5.8 Hz, 2H), 7.63 (d, *J* = 2.0 Hz, 1H), 7.57 (s, 1H), 7.51 (d, *J* = 3.0 Hz, 1H), 7.16 - 7.08 (m, 3H), 6.85 (dd, *J* = 8.5, 2.0 Hz, 1H), 6.49 (dd, *J* = 3.1, 1.8 Hz, 1H), 3.80 (s, 2H).

N (4-(aminomethyl)-2-(1H-pyrazole-3-yl)phenyl)thiophene-3-sulfonamide (**$I_D$-7**)

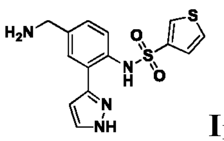

$I_D$-7

HPLC: 97.9%; LC-MS (m/z): 335.11 $(M+H)^+$; $^1H$ NMR (400 MHz, DMSO) $\delta$ 7.96 (s, 1H), 7.71 (s, 1H), 7.65 (s, 1H), 7.55 - 7.46 (m, 1H), 7.40 (d, *J* = 8.4 Hz, 1H), 7.14 (d, *J* = 4.9 Hz, 1H), 7.07 (d, *J* = 8.0 Hz, 1H), 6.70 (s, 1H), 3.84 (s, 2H).

*N*-(5-(aminomethyl)-2-(1*H*-pyrazole-3-yl)phenyl)thiophene-3-sulfonamide (**$I_D$-8**)

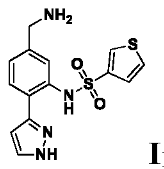

ID-8

HPLC: 97.6%; LC-MS (m/z) 335.16 $(M+H)^+$; $^1H$ NMR (400 MHz, DMSO) $\delta$ 8.10 (d, *J* = 1.9 Hz, 1H), 7.70 (dd, *J* = 9.0, 4.8 Hz, 2H), 7.59 - 7.49 (m, 2H), 7.17 (d, *J* = 5.1 Hz, 1H), 6.96 (d, *J* = 7.8 Hz, 1H), 6.77 (d, *J* = 1.6 Hz, 1H), 3.84 (s, 2H).

**Example 4**

**Synthesis of compound $I_E$:**

*N*-(3-(furan-2-yl)-4-(thiophene-3-sulfonamide)benzyl)acetamide (**$I_E$**)

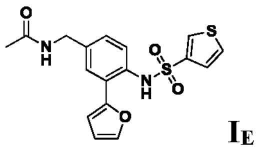

IE

Compound **$I_D$-5** (66.8mg, 0.2mmol) was dissolved in 10 milliliter of dichloromethane solution, then acetic anhydride (26.5mg, 0.26mmol) and triethylamine (60.7mg, 0.6mmol) were added. The mixture was reacted overnight at room temperature. After the reaction was finished, the solvent was evaporated and the residue was separated by column chromatography to obtain compound **$I_E$**, as a white solid (35.1mg, 46.6%).

HPLC: 98.8%; LC-MS (m/z): 377.08 $(M+H)^+$; $^1H$ NMR (400 MHz, $CDCl_3$) $\delta$ 7.70 (dd, *J* = 3.0, 1.2 Hz, 1H), 7.62 (d, *J* = 8.3 Hz, 1H), 7.49 (d, *J* = 1.2 Hz, 1H), 7.31 (d, *J* = 1.7 Hz, 1H), 7.22 - 7.16 (m, 2H), 6.99 (dd, *J* = 5.2, 1.2 Hz, 1H), 6.49 - 6.38 (m, 2H), 4.40 (d, *J* = 5.8 Hz, 2H), 2.04 (d, *J* = 3.1 Hz, 3H).

**Example 5**

**Synthesis of compound $I_B$:**

**Step 1:** *N*-(4-cyano-2-(furan-2-yl)phenyl)-*N*-methylthiophene-3-sulfonamide (**VII-1**)

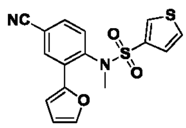

VII-1

Intermediate **V-30** (1.3g, 3.93mmol) and potassium carbonate (2.2g, 15.9mmol) were dissolved in 20 milliliter of acetonitrile solution, then iodomethane (1.7g, 12.0mmol) was added. The mixture reacted at 75 °C for three hours. After the reaction was finished, the solvent was evaporated and the residue was separated by column chromatography to obtain intermediate **VII-1**, as an off-white solid (0.7g, 2.0mmol).

$^1H$ NMR (400 MHz, DMSO) $\delta$ 8.33 - 8.29 (m, 1H), 8.27 (d, *J* = 1.8 Hz, 1H), 7.90 (dd, *J* = 5.1, 2.9 Hz, 2H), 7.77 (dd, *J* = 8.3, 1.8 Hz, 1H), 7.34 (dd, *J* = 5.1, 0.9 Hz, 1H), 7.23 (d, *J* = 3.4 Hz, 1H), 6.99 (d, *J* = 8.3 Hz, 1H), 6.74 (dd, *J* = 3.3, 1.7 Hz, 1H), 3.18 (s, 3H).

**Steps 2-4:** *N*-(2-(furan-2-yl)-4-((methylamino)methyl)phenyl)-*N*-methylthiophene-3-sulfonamide (**$I_B$**)

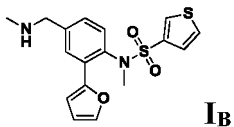

$I_B$

A similar method for synthesizing **$I_A$-1** was applied to obtain compound **$I_B$**, as an off-white solid (90mg, 36.5%).

HPLC: 99.1%; LC-MS (m/z) 363.15 $(M+H)^+$; $^1H$ NMR (400 MHz, DMSO) $\delta$ 8.27 (dt, *J* = 8.2, 4.1 Hz, 1H), 7.95 (d, *J* = 1.7 Hz, 1H), 7.90 (dd, *J* = 5.1, 3.0 Hz, 1H), 7.85 (d, *J* = 1.4 Hz, 1H), 7.37 - 7.28 (m, 2H), 7.14 (d, *J* = 3.4 Hz, 1H), 6.75 (d, *J* = 8.1 Hz, 1H), 6.71 (dd, *J* = 3.4, 1.8 Hz, 1H), 3.92 (s, 2H), 3.17 (s, 3H), 2.44 (s, 3H).

**Example 6**

**Synthesis of compound $I_F$:**

*N*-(4-(aminomethyl)-2-(furan-2-yl)phenyl)-*N*-methylthiophene-3-sulfonamide (**$I_F$**)

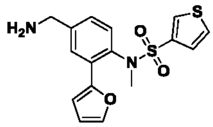

$I_F$

A similar method for synthesizing **$I_D$** was applied to obtain compound **$I_F$**, as an off-white solid (35.6mg, 31.3%).

HPLC: 99.4%; LC-MS (m/z) 349.11 $(M+H)^+$; $^1H$ NMR (400 MHz, DMSO) $\delta$ 8.26 (dd, *J* = 2.9, 1.2 Hz, 1H), 7.91 - 7.86 (m, 2H), 7.82 (s, 1H), 7.35 (dd, *J* = 5.1, 1.2 Hz, 1H), 7.23 (dd, *J* = 8.1, 1.7 Hz, 1H), 7.11 (d, *J* = 3.3 Hz, 1H), 6.68 (t, *J* = 5.6 Hz, 2H), 3.79 (s, 2H), 3.16 (s, 3H), 2.33 (s, 2H).

**Example 7**

**Synthesis of compound $I_G$:**

Methyl 4-(furan-2-yl)-3-(benzenesulfonamide)benzoate (**$I_G$**)

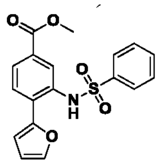

$I_G$

A similar method for synthesizing **V-1** was applied to obtain compound **$I_G$**, as a yellow solid (810mg, 32.1%).

HPLC: 98.9%; LC-MS (m/z) 358.08 $(M+H)^+$; $^1H$ NMR (400 MHz, DMSO) $\delta$ 7.84 (dd, *J* = 21.9, 11.2 Hz, 3H), 7.70 (d, *J* = 7.7 Hz, 2H), 7.64 (t, *J* = 7.2 Hz, 1H), 7.55 (t, *J* = 7.5 Hz, 2H), 7.42 (s, 1H), 7.17 (d, *J*= 3.3 Hz, 1H), 6.63 (s, 1H), 3.84 (s, 3H).

**Example 8**

**Synthesis of compound $I_H$:**

*N*-(2-(furan-2-yl)-5-(hydroxymethyl)phenyl)benzenesulfonamide (**$I_H$**)

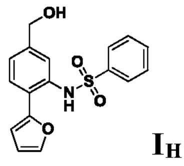

$\mathbf{I_H}$

Compound $\mathbf{I_G}$ (500mg, 1.4mmol) was dissolved in 10 milliliter of anhydrous tetrahydrofuran solution, under nitrogen protection, then lithium aluminum hydride (213mg, 5.6mmol) was added at 0 °C. The mixture reacted at room temperature for one hour. After the reaction was finished, 213 microliters of water, 213 microliters of sodium hydroxide aqueous solution (15% wt), and 639 microliters of water were added into the system sequentially. The mixture was filtered. The filtrate was evaporated to dryness, and the residue was separated by column chromatography to obtain compound $\mathbf{I_H}$, as a yellow oily substance (270mg, 58.6%).

HPLC: 97.0%; LC-MS (m/z): 330.11 $(M+H)^+$; $^1H$ NMR (400 MHz, DMSO) $\delta$ 9.66 (s, 1H), 7.71 (dd, *J* = 5.2, 3.3 Hz, 3H), 7.64 (ddd, *J* = 6.4, 4.2, 2.5 Hz, 2H), 7.55 (dd, *J* = 10.4, 4.7 Hz, 2H), 7.26 (dd, *J* = 8.1, 1.6 Hz, 1H), 6.94 (d, *J* = 2.9 Hz, 2H), 6.57 (dd, *J* = 3.4, 1.8 Hz, 1H), 5.22 (t, *J* = 5.7 Hz, 1H), 4.40 (d, *J* = 5.5 Hz, 2H).

### Example 9

### Synthesis of compound $\mathbf{I_J}$:

*N*-(4-((dimethylamino)methyl)-2-(furan-2-yl)phenyl)thiophene-3-sulfonamide ($\mathbf{I_J}$**-1**)

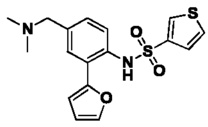

$\mathbf{I_J}$**-1**

N-(2-(furan-2-yl)-4-(hydroxymethyl)phenyl)thiophene-3-sulfonamide (1.68g, 5mmol) was dissolved in 50 milliliters of dichloromethane solution, and triphenylphosphine (1.97g, 7.5mmol) and carbon tetrabromide (2.49g, 7.5mmol) were added under ice bath. The mixture reacted at room temperature for two hours. After the solvent was evaporated, the residue was dissolved in 20milliliters of *N,N*-dimethylformamide solution. Then dimethylamine hydrochloride (0.82g, 10mmol) and potassium carbonate (0.83g, 6mmol) were added and the mixture reacted overnight at 80 °C. Post treatment, water was added to the system, then extracted three times with ethyl acetate, washed with water, washed with saturated salt water, dried with anhydrous sodium sulfate, filtered, concentrated, and the residue was separated by column chromatography to obtain compound $\mathbf{I_J}$**-1**, as a yellow solid (105mg, 5.8%).

HPLC: 96.6%; LC-MS (m/z): 363.08 $(M+H)^+$; $^1H$ NMR (500 MHz, DMSO) $\delta$ 8.00 (dd, *J* = 2.9, 1.2 Hz, 1H), 7.71 (t, *J* = 3.5 Hz, 1H), 7.70 - 7.63 (m, 2H), 7.24 - 7.20 (m, 1H), 7.11 - 7.07 (m, 2H), 6.90 (t, *J* = 8.1 Hz, 1H), 6.60 - 6.56 (m, 1H), 3.50 (s, 2H), 2.23 (s, 6H).

*N*-(4-((3-fluoroazetidin-1-yl)methyl)-2-(furan-2-yl)phenyl)thiophene-3-sulfonamide ($\mathbf{I_J}$**-2**)

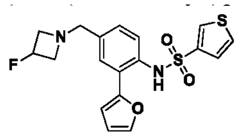

$\mathbf{I_J}$**-2**

A similar method for synthesizing $\mathbf{I_J}$**-1** was applied to obtain compound $\mathbf{I_J}$**-2**, as a yellow solid (84mg, 4.1%).

HPLC: 98.3%; LC-MS (m/z): 393.09 $(M+H)^+$; $^1H$ NMR (500 MHz, DMSO) $\delta$ 8.04 (dd, *J* = 2.9, 1.1 Hz, 1H), 7.77 - 7.67 (m, 2H), 7.61 (d, *J* = 1.6 Hz, 1H), 7.23 (dd, *J* = 5.1, 1.2 Hz, 1H), 7.09 (dd, *J* = 8.1, 1.7 Hz, 1H), 7.01 (d, *J* = 3.3 Hz, 1H), 6.84 (d, *J* = 8.1 Hz, 1H), 6.59 (dd, *J* = 3.3, 1.8 Hz, 1H), 3.63 (s, 2H), 3.60 - 3.48 (m, 2H), 3.14 (d, *J* = 22.3 Hz, 2H), 2.05 - 1.92 (m, 1H).

**Example 10**

**Synthesis of compound $I_P$:**

4-(Furan-2-yl)-*N*-methyl-3-(benzenesulfonamido)benzamide (**$I_P$-1**)

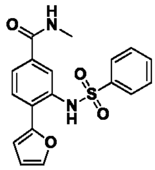

**$I_P$-1**

Compound **$I_G$** (0.71g, 2mmol) was dissolved in 10 milliliters of ethanol solution, then a solution of methylamine (2mL, 30-33wt%) in ethanol was added. The mixture reacted at 60 °C for four hours. Post-treatment, the residue was subjected to column chromatography (petroleum ether: ethyl acetate=1:3) to obtain compound **$I_P$-1**, as a yellow solid (64mg, 9.0%).

HPLC: 97.7%; LC-MS (m/z): 357.09 $(M+H)^+$; $^1H$ NMR (400 MHz, DMSO) $\delta$ 9.83 (s, 1H), 8.46 (d, *J* = 4.5 Hz, 1H), 7.81 - 7.71 (m, 3H), 7.66 (d, *J* = 7.7 Hz, 2H), 7.61 (t, *J* = 7.2 Hz, 1H), 7.51 (dd, *J* = 13.8, 5.9 Hz, 3H), 7.05 (d, *J* = 3.3 Hz, 1H), 6.58 (s, 1H), 2.74 (d, *J* = 4.4 Hz, 3H).

Similar methods for synthesizing **$I_P$-1** were applied to obtain the following compounds:

4-(Furan-2-yl)-3-(benzenesulfonamido)benzamide (**$I_P$-2**)

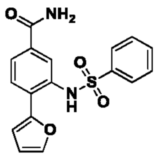

**$I_P$-2**

HPLC: 98.2%; LC-MS (m/z): 343.11 $(M+H)^+$; $^1H$ NMR (400 MHz, DMSO) $\delta$ 9.79 (s, 1H), 7.94 (s, 1H), 7.76 (d, *J* = 8.2 Hz, 1H), 7.70 (d, *J* = 8.5 Hz, 2H), 7.62 (d, *J* = 8.2 Hz, 2H), 7.57 (t, *J* = 6.9 Hz, 1H), 7.52 - 7.42 (m, 3H), 7.33 (s, 1H), 7.03 (d, *J* = 3.5 Hz, 1H), 6.55 (dt, *J* = 2.9, 1.4 Hz, 1H).

*N*-(2-(furan-2-yl)-5-(hydrazinocarbonyl)phenyl)benzenesulfonamide (**$I_P$-3**)

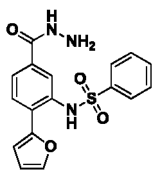

**$I_P$-3**

HPLC: 97.5%; LC-MS (m/z): 358.04 $(M+H)^+$; $^1H$ NMR (400 MHz, DMSO) $\delta$ 9.77 (s, 2H), 7.73 - 7.71 (m, 1H), 7.69 (s, 2H), 7.66 - 7.62 (m, 2H), 7.58 (t, *J* = 7.4 Hz, 1H), 7.48 (t, *J* = 7.8 Hz, 2H), 7.43 (s, 1H), 7.04 (d, *J* = 3.4 Hz, 1H), 6.58 - 6.52 (m, 1H), 4.47 (s, 2H).

**Example 11**

**Synthesis of compound $I_K$:**

4-(Furan-2-yl)-3-(benzenesulfonamide)benzoic acid (**$I_K$**)

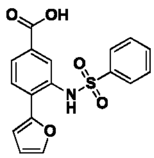

$I_K$

Compound **$I_G$** (0.36g, 1mmol) was dissolved in 10 milliliters of tetrahydrofuran solution, then 10 milliliters of lithium hydroxide aqueous solution (50% wt) was added. The mixture reacted overnight at 50 °C. After the reaction was finished, the solvent was evaporated and an appropriate amount of dilute hydrochloric acid was added to the system to adjust the pH to 3 -4. The obtained mixture was extracted three times with ethyl acetate, washed with water, washed with saturated salt water, dried with anhydrous sodium sulfate, filtered, concentrated, and the residue was separated by column chromatography to obtain compound **$I_K$**, as a white solid (0.12g, 34.9%).

HPLC: 99.1%; LC-MS (m/z): 344.06 $(M+H)^+$; $^1H$ NMR (400 MHz, DMSO) $\delta$ 12.99 (s, 1H), 9.89 (s, 1H), 7.84 (dd, *J* = 21.9, 11.2 Hz, 3H), 7.70 (d, *J*= 7.7 Hz, 2H), 7.64 (t, *J* = 7.2 Hz, 1H), 7.55 (t, *J* = 7.5 Hz, 2H), 7.42 (s, 1H), 7.17 (d, *J* = 3.3 Hz, 1H), 6.63 (s, 1H).

**Example 12**

**Synthesis of compound $I_Q$:**

**Step 1:** Methyl 3-nitro-4-(1*H*-pyrrole-1-yl)benzoate **(XIII-1)**

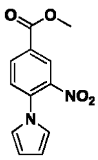

**XIII-1**

Methyl 4-fluoro-3-nitrobenzoate (2.0g, 10.0mmol), pyrrole (2g, 29.8mmol), and sodium hydroxide (0.56g, 14.0mmol) were dissolved in 5 milliliters of anhydrous dimethyl sulfoxide solution. The mixture reacted overnight at 50 °C under nitrogen protection. Post-treatment, water was added to the system, then extracted with ethyl acetate and dried with anhydrous sodium sulfate. The solvent was evaporated to dryness and the residue was separated by column chromatography to obtain intermediate **XIII-1,** as a yellow solid (700mg, 28.3%).

$^1H$ NMR (400 MHz, DMSO) $\delta$ 8.51 (d, *J* = 3.2 Hz, 1H), 8.38 - 8.25 (m, 1H), 7.84 (dd, *J* = 8.1, 5.3 Hz, 1H), 7.06 (d, *J* = 2.3 Hz, 2H), 6.36 (d, *J* = 2.2 Hz, 2H), 3.96 (s, 3H).

**Step 2:** Methyl 3-amino-4-(1*H*-pyrrole-1-yl)benzoate **(XIV-1)**

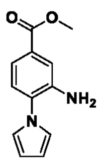

**XIV-1**

Intermediate **XIII-1** (0.7g, 2.84mmol) was dissolved in 20 milliliters of mixed solution of ethanol and water (v/v=1:1), then iron powder (0.8g, 14.2mmol) and ammonium chloride (0.76g, 14.2mmol) were added into the system. The mixture reacted at 75 °C for 1 hour. Post-treatment: the mixture was filtered, the filtrate was concentrated, extracted with dichloromethane, dried with sodium sulfate, and subjected to solvent column chromatography (petroleum ether: ethyl acetate=5:1) to obtain **XIV-1,** as a yellow solid (540mg, 87.8%)
$^1$H NMR (400 MHz, DMSO) $\delta$ 7.56 (d, *J* = 1.3 Hz, 1H), 7.27 (dd, *J* = 8.1, 1.5 Hz, 1H), 7.20 (d, *J* = 8.1 Hz, 1H), 7.03 (s, 2H), 6.33 (s, 2H), 5.23 (s, 2H), 3.88 (s, 3H).

**Steps 3-4:** *N*-(5-(hydroxymethyl)-2-(1*H*-pyrrole-1-yl)phenyl)benzenesulfonamide **(XVI-1)**

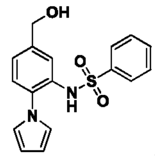

XVI-1

A similar method for synthesizing **$I_H$** was applied to obtain intermediate **XVI-1,** as a yellow solid (270mg, 58.6%).
$^1$H NMR (400 MHz, DMSO) $\delta$ 7.90 (dd, *J* = 8.3, 1.4 Hz, 1H), 7.71 - 7.61 (m, 4H), 7.57 (t, *J* = 7.7 Hz, 2H), 7.49 (d, *J* = 8.3 Hz, 1H), 7.03 (s, 2H), 6.27 (s, 2H), 3.84 (s, 3H).

**Step 5:** *N*-(5-formyl-2-(1H-pyrrole-1-yl)phenyl)benzenesulfonamide **(XVII-1)**

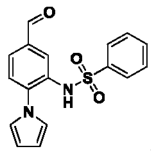

XVII-1

Intermediate **XVI-1** (270mg, 0.82mmol) was dissolved in 10 milliliters of anhydrous dichloromethane solution, then 204 milligrams of pyridinium chlorochromate (204 mg, 0.95 mmol) was added. The mixture reacted at room temperature for one hour. After the reaction was finished, the filtrate was filtered and evaporated to dryness. The residue was separated by column chromatography to obtain intermediate **XVII-1,** as a yellow oily substance (160mg, 59.8%).
$^1$H NMR (500 MHz, DMSO) $\delta$ 10.06 (s, 1H), 9.90 (s, 1H), 7.87 (dd, *J* = 8.2, 1.8 Hz, 1H), 7.70 - 7.60 (m, 3H), 7.53 (ddd, *J* = 8.3, 6.5, 2.8 Hz, 4H), 7.01 (t, *J* = 2.2 Hz, 2H), 6.32 - 6.19 (m, 2H).

**Step 6:** *N*-(5-((methylaminono)methyl)-2-(1*H*-pyrrole-1-yl)phenyl)benzenesulfonamide (**$I_Q$-1**)

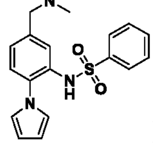

$I_Q$-1

A similar method for synthesizing **$I_A$-1** was applied to obtain compound **$I_Q$-1**, as a white solid (130mg, 77.7%).
HPLC: 99.2%; LC-MS (m/z): 342.15 (M+H)$^+$; $^1$H NMR (500 MHz, DMSO) $\delta$ 7.63 (dd, *J* = 8.6, 7.0 Hz, 2H), 7.44 - 7.35 (m, 3H), 7.27 (d, *J* = 1.8 Hz, 1H), 7.07 (d, *J* = 8.0 Hz, 1H), 6.95 (t, *J* = 2.1 Hz, 2H), 6.83 (dd, *J* = 8.0, 1.7 Hz, 1H), 6.09 (t, *J* = 2.1 Hz, 2H), 3.74 (s, 2H), 2.33 (s, 3H).
Similar methods for synthesizing **$I_Q$-1** were applied to obtain the following compounds:

*N*-(5-((methylamino)methyl)-2-(1*H*-pyrazol-1-yl)phenyl)benzenesulfonamide (**$I_Q$-2**)

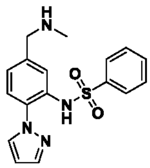

**$I_Q$-2**

HPLC: 99.5%; LC-MS (m/z): 343.10 $(M+H)^+$; $^1$H NMR (400 MHz, DMSO) $\delta$ 8.53 (s, 1H), 7.70 (s, 1H), 7.63 (d, *J* = 7.2 Hz, 2H), 7.54 (d, *J* = 8.2 Hz, 1H), 7.49 (s, 1H), 7.40 (dt, *J* = 23.4, 7.0 Hz, 3H), 6.91 (d, *J* = 8.0 Hz, 1H), 6.43 (s, 1H), 3.87 (s, 2H), 2.41 (s, 3H).

*N*-(5-((methylamino)methyl)-2-(2*H*-1,2,3-triazol-2-yl)phenyl)benzenesulfonamide (**$I_Q$-3**)

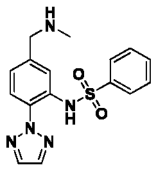

**$I_Q$-3**

HPLC: 98.9%; LC-MS (m/z): 344.09 $(M+H)^+$; $^1$H NMR (400 MHz, DMSO) $\delta$ 8.03 (s, 2H), 7.62 (d, *J* = 7.5 Hz, 2H), 7.50 (s, 1H), 7.44 (t, *J* = 7.1 Hz, 1H), 7.37 (t, *J* = 7.5 Hz, 2H), 7.32 (d, *J* = 8.1 Hz, 1H), 6.94 (d, *J* = 8.1 Hz, 1H), 3.84 (s, 2H), 2.38 (s, 3H).

*N*-(5-((methylamino)methyl)-2-(1*H*-1,2,3-triazol-1-yl)phenyl)benzenesulfonamide (**$I_Q$-4**)

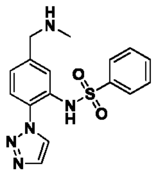

**$I_Q$-4**

HPLC: 97.5%; LC-MS (m/z): 344.05 $(M+H)^+$; $^1$H NMR (400 MHz, DMSO) $\delta$ 8.65 (s, 1H), 7.85 (s, 1H), 7.76 - 7.68 (m, 2H), 7.56 - 7.46 (m, 2H), 7.39 (d, *J* = 4.7 Hz, 3H), 6.81 (d, *J* = 7.8 Hz, 1H), 3.97 (s, 2H), 2.48 (s, 3H).

**Example 13**

**Synthesis of compound $I_R$:**

**Step 1:** 2-(4-amino-3-bromophenyl)acetonitrile (**XVIII-1**)

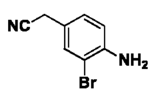

**XVIII-1**

P-aminophenylacetonitrile (5.0g, 37.83mmol) was dissolved in 50 milliliters of acetonitrile solution, then N-bromosuccinimide (6.7g, 37.83mmol) was added into the system. The mixture reacted at room temperature for 30 minutes and raw material was reacted to completion by TLC monitoring. Post-treatment: the solvent was evaporated to dryness, and the residue was separated by column chromatography (petroleum ether: ethyl acetate=4:1) to obtain the title compound intermediate **XVIII-1** (7.3g, 91.4%), as a light yellow solid.

$^1$H NMR (400 MHz, $CDCl_3$) $\delta$ 7.40 (d, *J* = 1.7 Hz, 1H), 7.09 (dd, *J* = 8.2, 1.9 Hz, 1H), 6.79 (d, *J* = 8.2 Hz, 1H), 3.65 (s, 2H).

**Steps 2-4:** *N*-(4-(2-aminoethyl)-2-(furan-2-yl)phenyl)thiophene-3-sulfonamide **(IR)**

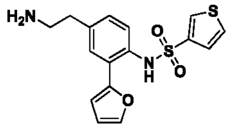

$I_R$

A similar method for synthesizing **$I_A$-1** was applied to obtain compound **$I_R$**, as a light brown solid (2.1g, 90.22%).

HPLC: 98.0%; LC-MS (m/z): 349.07 $(M+H)^+$; $^1$H NMR (400 MHz, DMSO) (58.08 (dd, *J* = 3.0, 1.3 Hz, 1H), 7.77 (d, *J* = 1.2 Hz, 1H), 7.75 (dd, *J* = 5.1, 3.0 Hz, 1H), 7.62 (d, *J* = 2.0 Hz, 1H), 7.29 (dd, *J* = 5.1, 1.3 Hz, 1H), 7.14 (dd, *J* = 8.2, 2.0 Hz, 1H), 7.05 (d, *J* = 3.4 Hz, 1H), 6.88 (d, *J* = 8.2 Hz, 1H), 6.62 (dd, *J* = 3.4, 1.8 Hz, 1H), 3.06 (s, 2H), 2.97 - 2.89 (m, 2H).

**Example 14**

**Synthesis of compound $I_S$:**

**Step 1:** tert-butyl 3-(furan-2-yl)-4-(thiophene-3-sulfonamide) phenethylcarbamate (**XX-1**)

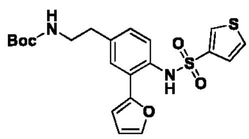

XX-1

Compound **$I_R$** (600mg, 1.72mmol) was dissolved in 35 milliliter of mixed solution of dichloromethane/methanol (volume/volume=6:1), then di-tert-butyl dicarbonate (530mg, 2.43mmol) was added. The mixture reacted overnight at room temperature. After the reaction was finished, the solvent was evaporated to dryness, and the residue was separated by column chromatography to obtain intermediate **XX-1,** as a colorless oily substance (520mg, 67.40%).

$^1$H NMR (400 MHz, DMSO) $\delta$ 8.06 (dd, *J* = 3.0, 1.3 Hz, 1H), 7.77 - 7.72 (m, 2H), 7.56 (d, *J* = 1.9 Hz, 1H), 7.27 (dd, *J* = 5.1, 1.2 Hz, 1H), 7.09 - 7.01 (m, 2H), 6.92 (t, *J* = 5.4 Hz, 1H), 6.83 (d, *J* = 8.1 Hz, 1H), 6.62 (dd, *J* = 3.4, 1.8 Hz, 1H), 3.17 (dd, *J* = 13.5, 6.6 Hz, 2H), 2.72 (t, *J* = 7.2 Hz, 2H), 1.38 (s, 9H).

**Step 2:** *N*-(2-(furan-2-yl)-4-(2-(methylamino)ethyl)phenyl)thiophene-3-sulfonamide (**$I_S$**)

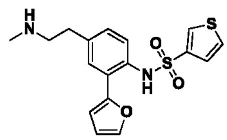

$I_S$

Intermediate **XX-1** (520mg, 1.16mmol) was dissolved in 30 milliliters of tetrahydrofuran solution, then lithium aluminum hydride (221mg, 5.82mmol) was added at 0 °C. The mixture reacted overnight at 80 °C. After the reaction was finished, 221 microliters of water, 221 microliters of sodium hydroxide aqueous solution (10% wt) and 221 microliters

of water were added into the system sequentially. The mixture was filtered. Then the filtrate was evaporated to dryness, and the residue was separated by column chromatography to obtain compound **$I_S$,** as a light brown solid (70mg, 16.65%).

HPLC: 97.3%; LC-MS (m/z): 363.10 $(M+H)^+$; $^1H$ NMR (400 MHz, DMSO) $\delta$ 7.79 (d, *J* = 1.9 Hz, 1H), 7.64 (s, 1H), 7.53 - 7.47 (m, 2H), 7.41 (d, *J* = 3.2 Hz, 1H), 7.19 - 7.15 (m, 1H), 7.12 (d, *J* = 8.3 Hz, 1H), 6.84 (dd, *J* = 8.3, 2.1 Hz, 1H), 6.55 (dd, *J* = 3.2, 1.8 Hz, 1H), 3.01 - 2.93 (m, 2H), 2.78 - 2.71 (m, 2H), 2.50 (s, 3H).

**Example 15**

**Synthesis of compound $I_U$:**

**Step 1:** *N*-(4-((tert-butylsulfoxide)imino)methyl)-2-(furan-2-yl)phenyl)-*N*-methylbenzenesulfonamide (**XXII-1**)

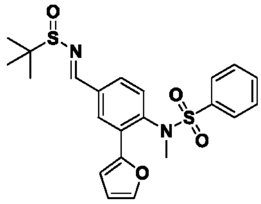

**XXII-1**

Intermediate *N*-(4-formyl-2-(furan-2-yl)phenyl)-*N*-toluenesulfonamide **(VIII-2)** (682.8mg, 2.0mmol), tert-butylsulfonamide (266.6mg, 2.2mmol), and copper sulfate (478.8mg, 3.0mmol) were dissolved in 30 milliliter of anhydrous 1,2-dichloroethane solution. The mixture was heated to 85 °C and reacted overnight. After the reaction was finished, when the system cooled down to temperature, the mixture was suction filtered and the filtrate was concentrated, and the residue was separated by column chromatography to obtain intermediate **XXII-1,** as an off-white solid (683.4mg, 76.9%).

$^1H$ NMR (400 MHz, DMSO) $\delta$ 8.64 (s, 1H), 8.43 (d, *J* = 1.8 Hz, 1H), 7.88 (d, *J* = 1.3 Hz, 1H), 7.81 (ddd, *J* = 14.7, 7.9, 4.7 Hz, 4H), 7.72 (t, *J* = 7.6 Hz, 2H), 7.22 (d, *J* = 3.4 Hz, 1H), 6.89 (d, *J* = 8.2 Hz, 1H), 6.74 (dd, *J* = 3.4, 1.8 Hz, 1H), 3.19 (s, 3H), 1.24 (s, 9H).

**Step 2:** *N*-(4-(1-(tert-butylsulfoxidede)amino)ethyl)-2-(furan-2-yl)phenyl)-N-toluenesulfonamide **(XXIII-1)**

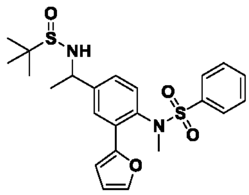

**XXIII-1**

Intermediate **XXII-1** (666.8mg, 1.5mmol) was dissolved in 30 milliliters of anhydrous tetrahydrofuran solution, then 1.5 milliliters of a solution of methylmagnesium bromide in tetrahydrofuran (1M/L, 1.5mmol) was slowly added dropwise at -78 °C. After addition, the system temperature was slowly elevated to -20 °C, then the mixture reacted for 6 hours. After the reaction was finished, ammonium chloride aqueous solution was added into the system under ice bath condition for quenching and water was added, then extracted three times with ethyl acetate, washed with water, washed with saturated salt, dried with anhydrous sodium sulfate, filtered, concentrated, and the residue was separated by column chromatography to obtain intermediate **XXIII-1,** as a yellow solid (425.4mg, 61.6%).

$^1H$ NMR (400 MHz, DMSO) $\delta$ 7.95 (d, *J* = 1.7 Hz, 1H), 7.85 - 7.76 (m, 4H), 7.72 (t, *J* = 7.6 Hz, 2H), 7.28 (dt, *J* = 12.4, 6.2 Hz, 1H), 7.14 (d, *J* = 3.4 Hz, 1H), 6.73 - 6.65 (m, 2H), 5.84 (d, *J* = 7.3 Hz, 1H), 4.52 - 4.42 (m, 1H), 3.16 (s, 3H), 1.46 (d, *J* = 6.8 Hz, 3H), 1.18 (s, 9H).

**Step 3:** *N*-(4-(1-aminoethyl)-2-(furan-2-yl)phenyl)-*N*-toluenesulfonamide (**$I_U$-1**)

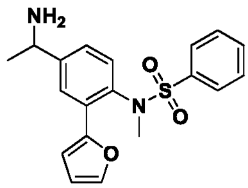

$I_U$-1

Intermediate **XXIII-1** (368.5mg, 0.8mmol) was dissolved in 20 milliliters of ethanol solution, then 2 mL of a solution of hydrochloric acid in ethanol (2M/L, 4.0 mmol) was added. The mixture reacted at 60 °C for two hours. After the reaction was finished, the solvent was evaporated to dryness, and water was added to the residue. An appropriate amount of sodium bicarbonate aqueous solution was added to adjust the pH to 8-9, then the mixture was extracted three times with ethyl acetate, washed with water, washed with saturated salt water, dried with anhydrous sodium sulfate, filtered, concentrated and the residue was separated by column chromatography to obtain intermediate **$I_U$-1**, as an off-white solid (180.1mg, 63.2%).

HPLC: 99.1%; LC-MS (m/z): 357.62 $(M+H)^+$; $^1H$ NMR (400 MHz, DMSO) $\delta$ 7.90 (d, *J* = 1.6 Hz, 1H), 7.83 - 7.74 (m, 4H), 7.70 (t, *J* = 7.6 Hz, 2H), 7.24 (dd, *J* = 8.2, 1.7 Hz, 1H), 7.12 (d, *J* = 3.3 Hz, 1H), 6.69 (dd, *J* = 3.3, 1.7 Hz, 1H), 6.61 (d, *J* = 8.2 Hz, 1H), 4.13 - 3.95 (m, 1H), 3.14 (s, 3H), 1.28 (d, *J* = 6.6 Hz, 3H).

Similar methods for synthesizing **$I_U$-1** were applied to obtain the following compounds:

*N*-(4-(1-aminoethyl)-2-(furan-2-yl)phenyl)benzenesulfonamide (**$I_U$-2**)

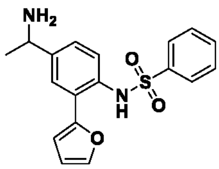

$I_U$-2

HPLC: 99.3%; LC-MS (m/z): 343.52 $(M+H)^+$; $^1H$ NMR (400 MHz, $CDCl_3$) $\delta$ 7.57 (t, *J* = 7.2 Hz, 3H), 7.43 (dd, *J* = 14.5, 7.0 Hz, 2H), 7.35 (d, *J* = 1.7 Hz, 1H), 7.32 - 7.24 (m, 3H), 6.40 (dd, *J* = 3.2, 1.8 Hz, 1H), 6.34 (d, *J* = 3.3 Hz, 1H), 4.09 (q, *J* = 6.5 Hz, 1H), 1.36 (d, *J* = 6.6 Hz, 3H).

*N*-(4-(1-aminopropyl)-2-(furan-2-yl)phenyl)benzenesulfonamide (**$I_U$-3**)

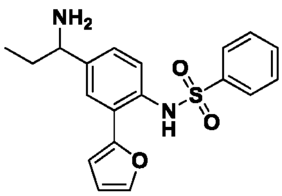

$I_U$-3

HPLC: 99.1%; LC-MS (m/z): 357.55 $(M+H)^+$; $^1H$ NMR (400 MHz, $CDCl_3$) $\delta$ 7.78 - 7.69 (m, 4H), 7.56 - 7.45 (m, 3H), 7.29 (d, *J* = 3.0 Hz, 1H), 7.17 - 7.11 (m, 1H), 7.06 (d, *J* = 8.4 Hz, 1H), 6.59 (dd, *J* = 3.3, 1.8 Hz, 1H), 4.10 - 4.00 (m, 1H), 1.98 - 1.73 (m, 2H), 0.78 (t, *J* = 7.4 Hz, 3H).

*N*-(4-(1-aminobutyl)-2-(furan-2-yl)phenyl)benzenesulfonamide (**$I_U$-4**)

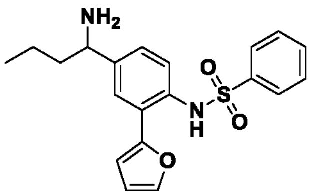

$I_U$-4

HPLC: 99.3%; LC-MS (m/z): 371.45 $(M+H)^+$; $^1H$ NMR (400 MHz, DMSO) $\delta$ 7.79 - 7.69 (m, 4H), 7.51 (dq, *J* = 14.4, 7.1 Hz, 3H), 7.28 (d, *J* = 2.9 Hz, 1H), 7.15 (d, *J* = 6.9 Hz, 1H), 7.05 (d, *J* = 8.4 Hz, 1H), 6.60 (dd, *J* = 3.2, 1.8 Hz, 1H), 4.14 (dd, *J* = 9.1, 5.9 Hz, 1H), 1.92 - 1.69 (m, 2H), 1.18 (ddd, *J* = 32.6, 16.7, 8.4 Hz, 2H), 0.86 (t, *J* = 7.3 Hz, 3H).

*N*-(4-(1-amino-2-methylpropyl)-2-(furan-2-yl)phenyl)benzenesulfonamide (**$I_U$-5**)

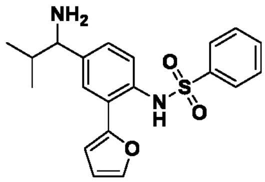

$I_U$-5

HPLC: 99.1%; LC-MS (m/z): 371.62 $(M+H)^+$; $^1H$ NMR (400 MHz, DMSO) $\delta$ 7.78 - 7.67 (m, 4H), 7.57 - 7.44 (m, 3H), 7.29 (d, *J* = 2.7 Hz, 1H), 7.11 - 7.01 (m, 2H), 6.59 (dd, *J* = 3.3, 1.8 Hz, 1H), 3.86 (d, *J* = 8.4 Hz, 1H), 2.06 (td, *J* = 14.5, 7.5 Hz, 1H), 1.02 (d, *J* = 6.6 Hz, 3H), 0.73 (d, *J* = 6.7 Hz, 3H).

*N*-(4-(amino(cyclopropyl)methyl)-2-(furan-2-yl)phenyl)benzenesulfonamide (**$I_U$-6**)

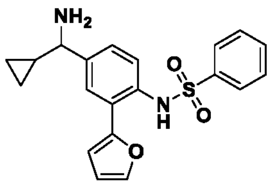

$I_U$-6

HPLC: 99.0%; LC-MS (m/z): 369.62 $(M+H)^+$; $^1H$ NMR (400 MHz, DMSO) $\delta$ 7.75 (dd, *J* = 6.5, 2.6 Hz, 3H), 7.67 (s, 1H), 7.51 (d, *J* = 2.9 Hz, 1H), 7.46 - 7.38 (m, 3H), 7.16 (d, *J* = 8.4 Hz, 1H), 7.06 - 6.97 (m, 1H), 6.58 (d, *J* = 1.8 Hz, 1H), 3.43 (d, *J* = 9.6 Hz, 1H), 1.28 (s, 1H), 0.65 (t, *J* = 6.9 Hz, 1H), 0.58 - 0.45 (m, 2H), 0.31 (dd, *J* = 14.4, 8.5 Hz, 1H).

*N*-(4-(amino(phenyl)methyl)-2-(furan-2-yl)phenyl)benzenesulfonamide (**$I_U$-7**)

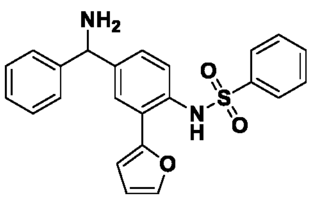

$I_U$-7

HPLC: 99.2%; LC-MS (m/z): 405.52 $(M+H)^+$; $^1H$ NMR (400 MHz, DMSO) $\delta$ 8.23 (s, 3H), 7.78 (s, 1H), 7.77 - 7.71 (m, 3H), 7.58 - 7.46 (m, 5H), 7.42 (t, *J* = 7.6 Hz, 2H), 7.34 (t, *J* = 7.2 Hz, 1H), 7.23 (d, *J* = 2.8 Hz, 1H), 7.15 (d, *J* =

8.5 Hz, 1H), 7.00 (d, *J* = 8.4 Hz, 1H), 6.60 (d, *J* = 1.8 Hz, 1H), 5.46 (s, 1H).

*N*-(4-(1-amino-2-phenylethyl)-2-(furan-2-yl)phenyl)benzenesulfonamide (**$I_U$-8**)

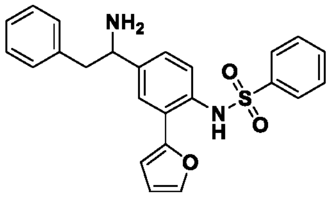

**$I_U$-8**

HPLC: 99.5%; LC-MS (m/z): 419.71 $(M+H)^+$; $^1H$ NMR (400 MHz, DMSO) $\delta$ 8.48 (s, 3H), 7.74 - 7.66 (m, 4H), 7.54 (t, *J* = 7.1 Hz, 1H), 7.48 (t, *J* = 7.4 Hz, 2H), 7.24 (t, *J* = 7.2 Hz, 2H), 7.19 (d, *J* = 7.0 Hz, 2H), 7.10 (d, *J* = 7.2 Hz, 3H), 6.95 (d, *J* = 8.4 Hz, 1H), 6.59 - 6.54 (m, 1H), 4.42 (dd, *J* = 9.2, 5.9 Hz, 1H), 3.28 - 3.20 (m, 1H), 3.08 (dd, *J* = 13.5, 9.6 Hz, 1H).

*N*-(5-(1-aminoethyl)-2-(furan-2-yl)phenyl)benzenesulfonamide (**$I_U$-9**)

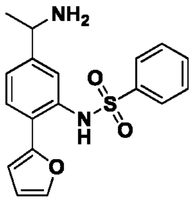

**$I_U$-9**

HPLC: 99.2%; LC-MS (m/z): 343.51 $(M+H)^+$; $^1H$ NMR (400 MHz, DMSO) $\delta$ 7.77 (d, *J* = 3.4 Hz, 2H), 7.61 (d, *J* = 6.5 Hz, 2H), 7.46 (d, *J* = 2.3 Hz, 1H), 7.40 (s, 3H), 7.28 (s, 1H), 6.79 (d, *J* = 8.1 Hz, 1H), 6.53 (s, 1H), 4.07 (d, *J* = 6.4 Hz, 1H), 1.33 (d, *J*= 6.4 Hz, 3H).

**Example 16**

**Synthesis of compound $I_V$:**

**Steps 1-2:** *N*-(4-acetyl-2-(furan-2-yl)phenyl)benzenesulfonamide **(XXV-1)**

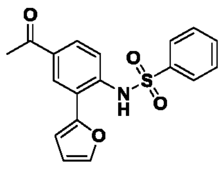

**XXV-1**

A similar method for synthesizing **V-1** was applied to obtain intermediate **XXV-1.**

$^1H$ NMR (400 MHz, $CDCl_3$) $\delta$ 8.33 (s, 1H), 8.04 (s, 1H), 7.86 (d, *J* = 8.2 Hz, 1H), 7.75 (d, *J* = 8.1 Hz, 3H), 7.60 (s, 1H), 7.54 (t, *J* = 7.1 Hz, 1H), 7.42 (t, *J* = 7.3 Hz, 2H), 6.57 (d, *J* = 11.6 Hz, 2H), 2.60 (s, 3H).

**Step 3:** *N*-(2-(furan-2-yl)-4-(1-(methylamino)ethyl)phenyl)benzenesulfonamide **(IV-1)**

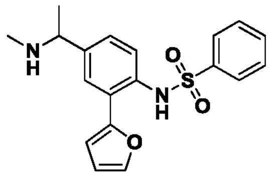

$I_V$-1

Intermediate **XXV-1** (682.8mg, 2mmol) was dissolved in 20 milliliters of dichloromethane solution, then 1 milliliter of a solution of methylamine in ethanol (30-33% wt), and tetraethoxytitanium (456.2mg, 2mmol) were added. The mixture reacted overnight at room temperature. The next day, sodium borohydride (151.3mg, 4mmol) was slowly added under ice water bath condition and the system reacted at room temperature for four hours. After the reaction was finished, water was added into the system., then extracted three times with dichloromethane, washed with water, washed with saturated salt water, dried with anhydrous sodium sulfate, filtered, concentrated, and the residue was separated by column chromatography to obtain compound **$I_V$-1**, as a white solid (165.1mg, 23.2%).

HPLC: 99.0%; LC-MS (m/z): 357.40 $(M+H)^+$; $^1H$ NMR (400 MHz, $CDCl_3$) $\delta$7.62 (dd, *J* = 12.1, 7.9 Hz, 3H), 7.52 - 7.43 (m, 2H), 7.35 (dd, *J* = 16.8, 4.8 Hz, 3H), 7.27 (dd, *J* = 8.4, 1.9 Hz, 1H), 6.45 (dd, *J* = 3.3, 1.8 Hz, 1H), 6.40 (d, *J* = 3.3 Hz, 1H), 3.68 (q, *J* = 6.5 Hz, 1H), 2.32 (s, 3H), 1.39 (d, *J* = 6.6 Hz, 3H).

A similar method for synthesizing **Iv-1** was applied to obtain the following compound:

*N*-(2-(furan-2-yl)-5-(1-(methylamino)ethyl)phenyl)benzenesulfonamide (**$I_V$-2**)

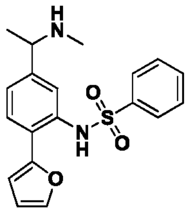

$I_V$-2

HPLC: 99.0%; LC-MS (m/z): 357.45 $(M+H)^+$; $^1H$ NMR (400 MHz, DMSO) $\delta$7.74 (d, *J* = 6.6 Hz, 2H), 7.65 (dd, *J* = 9.3, 4.5 Hz, 2H), 7.52 - 7.41 (m, 3H), 7.30 (d, *J* = 3.1 Hz, 1H), 7.07 (s, 1H), 6.95 (d, *J* = 8.1 Hz, 1H), 6.56 (dd, *J* = 3.2, 1.8 Hz, 1H), 3.72 (q, *J* = 6.4 Hz, 1H), 2.18 (s, 3H), 1.26 (d, *J* = 6.7 Hz, 3H).

**Example 17**

**Synthesis of compound $I_W$:**

**Step 1:** tert-butyl 2-(4-(furan-2-yl)-3-(benzenesulfonamide)phenyl)pyrrolidin-1-carboxylate **(XXVII-1)**

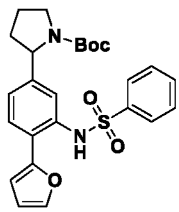

XXVII-1

A similar method for synthesizing **V-1** was applied to obtain intermediate **XXVII-1.**

$^1H$ NMR (400 MHz, DMSO) $\delta$9.71 (s, 1H), 7.69 (dd, *J* = 22.9, 16.6 Hz, 5H), 7.56 (d, *J* = 29.8 Hz, 2H), 7.13 (t, *J* = 13.5 Hz, 1H), 7.01 (t, *J* = 10.5 Hz, 1H), 6.59 (d, *J* = 17.1 Hz, 2H), 4.80 - 4.47 (m, 1H), 3.37 (d, *J* = 11.6 Hz, 1H), 3.21 (s, 1H), 2.16 (d, *J* = 48.0 Hz, 1H), 1.81 - 1.69 (m, 1H), 1.51 (d, *J* = 11.7 Hz, 1H), 1.40 (s, 3H), 1.27 (s, 1H), 1.14 (s, 6H).

**Step 2:** *N*-(2-(furan-2-yl)-5-(pyrrolidin-2-yl)phenyl)benzenesulfonamide **(IW)**

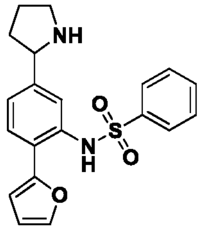

$I_W$

Intermediate **XXVI-1** (234.3mg, 0.5mmol) was dissolved in 30 milliliters of ethyl acetate solution, then 1 milliliter of a solution of hydrochloric acid in ethanol (2M/L, 2.0mmol) was added. The mixture reacted at room temperature for three hours. After the reaction was finished, the solvent was evaporated to dryness, and water was added to the residue. An appropriate amount of sodium bicarbonate aqueous solution was added into the system to adjust the pH to 8-9then extracted three times with ethyl acetate, washed with water, washed with saturated salt water, dried with anhydrous sodium sulfate, filtered, concentrated and the residue was separated by column chromatography to obtain compound $\mathbf{I_W}$, as an off-white solid (70.0mg, 38.0%).

HPLC: 99.1%; LC-MS (m/z): 368.34 $(M+H)^+$; $^1H$ NMR (400 MHz, DMSO) $\delta$7.75 (dd, *J* = 7.3, 1.9 Hz, 2H), 7.61 (d, *J* = 9.9 Hz, 2H), 7.41 (dd, *J* = 12.4, 5.0 Hz, 4H), 7.19 (s, 1H), 6.85 (d, *J* = 8.0 Hz, 1H), 6.54 (dd, *J* = 3.0, 1.7 Hz, 1H), 4.20 - 4.14 (m, 1H), 3.10 (t, *J* = 7.2 Hz, 2H), 2.13 (td, *J* = 12.0, 7.2 Hz, 1H), 1.92 - 1.84 (m, 2H), 1.66 (dq, *J* = 17.5, 8.8 Hz, 1H).

## Example 18

### The inhibitory activity of TRPA1

In this example, the inhibitory activity of the compounds prepared in some examples of the present invention on transient receptor potential channel protein ankyrin 1 (TRPA1) was tested. Among them, compound of formula A (WO2010075353) was used as the positive control compound:

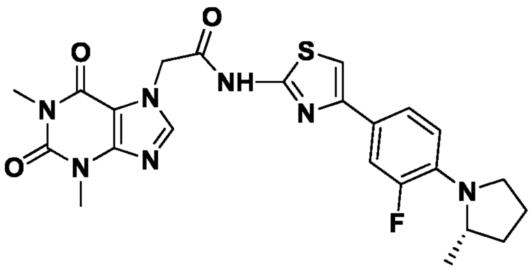

**Compound of formula A**

The method was as below:

Test method using IonWorks Barracuda (IWB) automated patch clamp detection: HEK293 cells stably expressing mTRPA1 were placed in the T175 culture flask with DMEM medium containing 15 g/mL Blasticidin S HCl, 200 g/mL Hygromycin B and 10% FBS, and cultured in 37°C, 5% $CO_2$ incubator. When the cell density reached about 80%, the culture medium was removed, washed once with phosphate buffered saline (PBS) without calcium and magnesium. 3 mL of Trypsin was added to digest for 2 min, 7 mL of culture solution was added to terminate the digestion. The cells were collected to 15 mL centrifuge tube and centrifuged at 800 rpm for 3 min. After the supernatant was removed, the cells were added to appropriate volume of extracellular fluid for re-suspending, and the cell density was controlled at 2-3$\times 10^6$/mL for use in IWB experiment. Extracellular fluid formulation (in mM): 140 NaCl, 5 KCl, 1 $MgCl_2$, 10 HEPES, 0.5 EGTA, 10 Glucose (pH 7.4); intracellular fluid formulation (in mM): 140 CsCl, 10 HEPES, 5 EGTA, 0.1 CaClz, 1 MgCh (pH 7.2). 28 mg/mL of amphotericin B was freshly prepared with DMSO on the day of experiment, and then final concentration of 0.1 mg/mL was prepared with intracellular fluid.

Population patch clamp (PPC) plate was used in IWB experiment. The entire detection process was automatically carried out by the instrument, that is, extracellular fluid was added into 384 wells of PPC plate, and after intracellular

fluid was added under PPC plate (in plenum), 6 L of cell fluid was added for sealing test, and finally the intracellular fluid in plenum was replaced with intracellular fluid containing amphotericin B to establish a whole-cell recording mode after perforating sealed cells. The sampling frequency for recording TPRA1 current was 10 kHz, the cells were clamped at 0 mV, the voltage stimulation command (channel protocol) was a ramp voltage from -100 mV to +100 mV for 300 ms. This voltage stimulation was applied every 10s, mTRPA1 current was induced by 300 M AITC.

Data recording and current amplitude measurement export were carried out by IWB software (version 2.5.3, Molecular Devices Corporation, Union City, CA). No statistics was recorded for holes with sealing impedance lower than 20 MΩ. The original current data was corrected by software for leak subtraction. TRPA1 current amplitude was measured at +100 mV. Each PPC plate in the experiment had a dose-effect data of HC030031 as a positive control. If $IC_{50}$ value of HC030031 exceeded 3 times of the average $IC_{50}$ value previously obtained on each plate, it would be re-tested. The dose-effect curve of compounds and $IC_{50}$ were fitted and calculated by GraphPad Prism 5.02 (GraphPad Software, San Diego, CA).

**Experimental results**

Some compounds of the present invention were subjected to $IC_{50}$ inhibition activity test using the IonWorks Barracuda (IWB) automated patch clamp detection method. The activity data are shown in Table 2.

Table 2. Inhibition activity data of some compounds of the present invention on TRPA1 ($IC_{50}$, μM)

| Number | $IC_{50}$(μM) | Number | $IC_{50}$(μM) | Number | $IC_{50}$(μM) | Number | $IC_{50}$(μM) |
|---|---|---|---|---|---|---|---|
| **$I_A$-1** | +++++ | **$I_A$-2** | +++++ | **$I_A$-3** | +++++ | **$I_A$-4** | +++++ |
| **$I_A$-5** | +++++ | **$I_A$-6** | ++++ | **$I_A$-7** | ++++ | **$I_A$-8** | +++ |
| **$I_A$-9** | ++ | **$I_A$-10** | +++++ | **$I_A$-11** | +++++ | **$I_A$-12** | ++++ |
| **$I_A$-13** | +++ | **$I_A$-14** | + | **$I_A$-15** | +++++ | **$I_A$-16** | ++++ |
| **$I_A$-17** | ++++ | **$I_A$-18** | +++ | **$I_A$-20** | +++++ | **$I_A$-21** | +++++ |
| **$I_A$-22** | ++ | **$I_A$-23** | +++ | **$I_A$-24** | ++ | **$I_A$-25** | ++ |
| **$I_A$-26** | +++++ | **$I_A$-27** | +++++ | **$I_A$-28** | +++++ | **$I_A$-29** | +++++ |
| **$I_A$-30** | ++++++ | **$I_A$-31** | ++++ | **$I_A$-32** | ++++++ | **$I_A$-33** | +++++ |
| **$I_A$-34** | +++++ | **$I_A$-35** | +++++ | **$I_A$-36** | ++ | **$I_A$-39** | ++ |
| **$I_A$-40** | + | **$I_A$-41** | ++++ | **$I_A$-42** | +++++ | **$I_A$-43** | +++++ |
| **$I_A$-44** | +++++ | **$I_A$-45** | +++++ | **$I_A$-46** | +++++ | **$I_A$-47** | +++++ |
| **$I_A$-48** | +++++ | **$I_A$-49** | | **$I_A$-50** | ++ | **$I_A$-51** | |
| **$I_A$-52** | ++ | **$I_A$-53** | ++++ | **$I_A$-54** | +++++ | **$I_A$-55** | ++++++ |
| **$I_A$-56** | +++ | **$I_A$-57** | ++ | **$I_A$-58** | + | **$I_A$-59** | ++++ |
| **$I_A$-61** | +++ | **$I_B$** | ++++++ | **$I_C$-1** | ++ | **$I_D$-1** | ++++++ |
| **$I_D$-2** | +++++ | **$I_D$-3** | ++++++ | **$I_D$-4** | +++++ | **$I_D$-5** | ++++++ |
| **$I_D$-6** | +++++ | **$I_D$-7** | +++++ | **$I_D$-8** | +++++ | **$I_E$** | ++ |
| **$I_F$** | ++++++ | **$I_H$** | ++ | **$I_J$-1** | +++++ | **$I_J$-2** | +++++ |
| **$I_Q$-1** | +++++ | **$I_Q$-2** | +++++ | **$I_Q$-3** | +++++ | **$I_Q$-4** | ++++ |
| **$I_R$** | +++++ | **$I_S$** | +++++ | **Compound of formula A** | + | **$I_A$-62** | + |
| **$I_U$-1** | +++++: | **$I_U$-2** | +++++: | **$I_U$-3** | ++++++ | **$I_U$-4** | ++++++ |
| **$I_U$-5** | ++++++ | **$I_U$-6** | ++++++ | **$I_U$-7** | +++++ | **$I_U$-8** | +++ |

(continued)

| Number | $IC_{50}$(μM) | Number | $IC_{50}$(μM) | Number | $IC_{50}$(μM) | Number | $IC_{50}$(μM) |
|---|---|---|---|---|---|---|---|
| **$I_U$-9** | ++++++ | **$I_V$-1** | +++++ | **$I_V$-2** | +++++ | **$I_W$** | +++++: |
| Among them, activity: (μM):<br>$50 \le IC_{50} < 100$: +<br>$20 \le IC_{50} < 50$: ++<br>$10 \le IC_{50} < 20$: +++<br>$5 \le IC_{50} < 10$: ++++<br>$1 \le IC_{50} < 5$: +++++<br>$IC_{50} < 1$: ++++++ | | | | | | | |

The results indicate that the compounds of the present invention exhibit strong inhibitory activity against TRPA1.

**Example 19**

The therapeutic effect of compounds prepared in some examples of the present invention on 2,4-dinitrobenzenesulfonic acid (DNBS) induced ulcerative colitis in Wistar rats (corresponding to Crohn's disease) was investigated.

**Experimental process**

1. Experimental animals

Animal species and strains: Wistar rats
Administration record: No administration history
Gender and weight: Male, about 150g
Breeding party/supplier: Shanghai SLAC Laboratory Animal Co., Ltd
Adaptation period: 5 days
Room: Regular Area Rooms
Indoor temperature: 20-26 °C
Indoor relative humidity: 40-70%
Lighting: Fluorescent lighting, 12-hour illumination (08:00-20:00) and 12-hour no illumination
Animal feeding: 2-4 rats per cage (same administration group)
Food: Unlimited access to feed (disinfected by irradiation, Jiangsu Xietong Pharmaceutical Bio-engineering Co., Ltd., China)
Water: Unlimited access to drinking water (treated with reverse osmosis and high-pressure sterilization)
A total of 80 Wistar rats were purchased from Shanghai SLAC Laboratory Animal Co., Ltd, of which 10 were used as backup animals to select animals with suitable weight range included in the groups, in order to control the fluctuation of disease severity caused by weight fluctuations.

2. Grouping

BioBook software was used to screen 70 animals for random grouping based on animal weight to ensure that the weight values of each group of animals were similar to reduce bias.

Table 3. Animal grouping and administration methods

| Group | Group name | Modeling agent | Number of animals | Administration method | | Administration dosage |
|---|---|---|---|---|---|---|
| | | | | administration route | administration frequency | mg/kg |
| G1 | blank control group | 30% ethanol | 10 | oral | once a day | -- |

(continued)

| Group | Group name | Modeling agent | Number of animals | Administration method | | Administration dosage |
|---|---|---|---|---|---|---|
| | | | | administration route | administration frequency | mg/kg |
| G2 | model-solvent group | DNBS | 10 | oral | once a day | -- |
| G3 | osalazine sodium | DNBS | 10 | oral | once a day, from Day 0 to Day 6 | 300 |
| G4 | **$I_A$-51** | DNBS | 10 | oral | once a day, from Day 0 to Day 6 | 10 |
| G5 | **$I_A$-30** | DNBS | 10 | oral | once a day, from Day 0 to Day 6 | 10 |
| G6 | **$I_D$-5** | DNBS | 10 | oral | once a day, from Day 0 to Day 6 | 10 |
| G7 | **$I_A$-55** | DNBS | 10 | oral | once a day, from Day 0 to Day 6 | 10 |

3. Experimental design

3.1 DNBS powder was dissolved in 30% ethanol with a final concentration of 60 mg/mL.

3.2 Induction of colorectal inflammation: Before the experiment, rats were fasted for 40 hours and subcutaneously injected with 5% glucose saline during the fasting period. On the day 0 of the experiment, fasting rats were anesthetized by intraperitoneal injection of Zoletil and 5 mg/kg Xylazine. G2 group-G7 group, a hose was extended from the anus to the left curvature of the colorectum, and colorectal inflammation in rats was induced with DNBS enema. The normal control group (G1) was treated with 30% ethanol enema using the same method.

3.3. Dosage regimen: After DNBS induction in G1 and G2 groups of animals, solvent was administered. After DNBS induction in G3 group, 300 mg/kg of oxalazine sodium was administered. After DNBS induction in G4 to G7 groups, 10 mg/kg of the compounds of the present invention were administered respectively. Please refer to Table 3 for the administration time, specific administration dosage, and administration route. Starting from day 0 of modeling, the drugs were administered continuously for 7 days, and the animals were euthanized on day 6.

3.4 Testing indicators: After the experiment, all animals were euthanized by inhaling excessive $CO_2$ and dislocation of cervical vertebra. After the abdominal cavity was cut open, the colorectum was taken out and the length of it was immediately measured. Then the colorectum was longitudinally cut open and rinsed clean. The weight of the colorectum and the area of the ulcer were recorded and the whole was photographed. At the same time, the damage to the rat colorectum was evaluated macroscopically according to Table 4.

Table 4. Macroscopic evaluation of rat colorectum injury score

| Items | score |
|---|---|
| Degree of adhesion: | |
| No adhesion<br>Mild adhesion<br>Severe adhesion | 0<br>1<br>2 |
| Degree of intestinal obstruction: | |
| None<br>Mild obstruction<br>Severe obstruction | 0<br>1<br>2 |
| Severity of ulcer: | |
| None | 0 |

(continued)

| Severity of ulcer: | |
|---|---|
| One ulcer with a length <1cm<br>Two ulcers with a length <1cm<br>Three or more ulcer or one of them with a length>1cm | 1<br>2<br>3 |
| Degree of intestinal wall thickening: | |
| < 1mm<br>1-3mm<br>Greater than 3mm | 0<br>1<br>2 |

3.5 Experimental observation

The health status of animals and their comprehensive response to surgery and medication were observed every day.

4. Statistical analysis

The experimental data was calculated as mean ± standard error. Statistical analysis was performed using Graphpad Prism, SPSS, or Sigmaplot software. The specific data is presented in the form of charts. P<0.05 is considered to have statistical differences.

**Experimental results**

In the experimental observation, none of the animals showed any abnormal appearance or behavior.

According to the experimental regimen, Wistar rats were induced to develop inflammatory colitis by colonic perfusion with DNBS. Colitis manifests as a significant reduction in colon length, a significant increase in colon weight, a significant increase in colon ulcer area, and a significant increase in macroscopic colon injury score. The colon length, weight, and ulcer area data of each group of animals are shown in Table 5 and Figure 1. The macroscopic colon injury score data of each group of animals are shown in Table 6 and Figure 1. Representative colorectal photos of each group of animals are shown in Figure 2.

According to the experimental results, in this experiment, the positive drug oxalazine sodium can significantly reduce the colon weight and ulcer area of model mice. The compounds **$I_A$-51**, **$I_A$-30**, **$I_D$-5**, and **$I_A$-55** of the present invention can significantly inhibit colon shortening in model mice and significantly reduce intestinal weight, ulcer area, and macroscopic colon injury score. The efficacy of each compound group of the present invention with a dosage of 10 mg/kg is stronger or not weaker than that of the positive control group with 300 mg/kg, indicating that the compounds of the present invention have a strong therapeutic effect and a low effective dose for DNBS induced colitis in rats.

Table 5. Colon length, weight, and ulcer area

| Group | | Colon length (cm) | Colon weight (g) | Ulcer length (cm) | Ulcer width (cm) | Ulcer area ($cm^2$) |
|---|---|---|---|---|---|---|
| G1-blank control group | Mean | 15.60 | 1.29 | 0.04 | 0.04 | 0.01 |
| | SEM | 0.46 | 0.06 | 0.04 | 0.04 | 0.01 |
| G2-model-solvent group | Mean | 12.95 | 1.76 | 1.67 | 0.93 | 1.71 |
| | SEM | 0.31 | 0.10 | 0.21 | 0.12 | 0.43 |
| G3-osalazine sodium | Mean | 14.33 | 1.41 | 0.71 | 0.34 | 0.30 |
| | SEM | 0.39 | 0.04 | 0.14 | 0.06 | 0.07 |
| G4-$I_A$-51 | Mean | 15.49 | 1.38 | 0.31 | 0.20 | 0.27 |
| | SEM | 0.37 | 0.05 | 0.15 | 0.10 | 0.16 |
| G5-$I_A$-30 | Mean | 15.54 | 1.32 | 0.33 | 0.19 | 0.18 |
| | SEM | 0.37 | 0.05 | 0.13 | 0.07 | 0.07 |

(continued)

| Group | | Colon length (cm) | Colon weight (g) | Ulcer length (cm) | Ulcer width (cm) | Ulcer area ($cm^2$) |
|---|---|---|---|---|---|---|
| G6-$I_D$-5 | Mean | 16.31 | 1.39 | 0.34 | 0.12 | 0.15 |
| | SEM | 0.45 | 0.04 | 0.19 | 0.07 | 0.10 |
| G7-$I_A$-55 | Mean | 14.96 | 1.49 | 0.60 | 0.40 | 0.48 |
| | SEM | 0.41 | 0.06 | 0.20 | 0.14 | 0.21 |

Table 6. Macroscopic evaluation of colon injury score in rats

| Group | | Degree of adhesion | Degree of intestinal infarction | Severity of ulcer | Degree of intestinal wall thickening | Macroscopic colon score |
|---|---|---|---|---|---|---|
| G1-blank control group | Mean | 0.20 | 0.20 | 0.20 | 0.20 | 0.80 |
| | SEM | 0.20 | 0.20 | 0.20 | 0.20 | 0.80 |
| G2-model-solvent group | Mean | 0.60 | 0.20 | 3.00 | 1.70 | 5.50 |
| | SEM | 0.27 | 0.13 | 0.00 | 0.15 | 0.45 |
| G3-osalazine sodium | Mean | 0.00 | 0.00 | 1.50 | 0.40 | 1.90 |
| | SEM | 0.00 | 0.00 | 0.34 | 0.16 | 0.38 |
| G4-$I_A$-51 | Mean | 0.07 | 0.00 | 0.60 | 0.27 | 0.93 |
| | SEM | 0.07 | 0.00 | 0.27 | 0.15 | 0.46 |
| G5-$I_A$-30 | Mean | 0.00 | 0.00 | 0.80 | 0.13 | 0.93 |
| | SEM | 0.00 | 0.00 | 0.31 | 0.09 | 0.36 |
| G6-$I_D$-5 | Mean | 0.00 | 0.00 | 0.70 | 0.10 | 0.80 |
| | SEM | 0.00 | 0.00 | 0.40 | 0.10 | 0.47 |
| G7-$I_A$-55 | Mean | 0.10 | 0.10 | 1.20 | 0.30 | 1.70 |
| | SEM | 0.10 | 0.10 | 0.42 | 0.15 | 0.68 |

In summary, the compounds **$I_A$-51**, **$I_A$-30**, **$I_D$-5**, and **$I_A$-55** of the present invention have good preventive and therapeutic effects on DNBS induced colitis in rats, and can be developed as drugs for treating Crohn's disease.

**Example 20**

The therapeutic effect of compounds prepared in some examples of the present invention on DSS (dextran sulfate sodium) induced inflammatory colitis in C57BL/6 mice (corresponding to ulcerative colitis) was investigated.

**Experimental process**

1. Experimental animals

Animal species and strains: C57BL/6 mice
Administration record: No administration history
Gender and weight: Female, about 18-20 g
Breeding party/supplier: Shanghai SLAC Laboratory Animal Co., Ltd

Adaptation period: 7 days
Room: SPF Rooms
Indoor temperature: 20-26 °C
Indoor relative humidity: 40-70%
Lighting: Fluorescent lighting, 12-hour illumination (08:00-20:00) and 12 hour no illumination
Animal feeding: 2-5 mice per cage (same administration group)
Food: Unlimited access to feed (disinfected by irradiation, Shanghai SLAC Laboratory Animal Co., Ltd, China)
Water: Unlimited access to drinking water (tap water purified through ultra-pure water filtration system)

A total of 80 C57BL/6 mice were purchased from Shanghai SLAC Laboratory Animal Co., Ltd, of which 10 were used as backup animals to select animals with suitable weight range included in the groups. All backup animals are not subjected to any administration or modeling procedures and will be euthanized uniformly at the end of the experiment.

2. Grouping

70 animals were selected on Day 3 and randomly grouped based on animal weight using BioBook software to ensure that the weight values of each group of animals were similar to reduce bias. The specific grouping information is shown in Table 7 below.

Table 7. Grouping and dosage regimen

| Group | Test object | Number of animals | Administration route | Administration dosage (mg/kg) | Dosage regimen |
|---|---|---|---|---|---|
| G1 | solvent group | 10 | oral | N/A | once a day, from day 0 to day 7 |
| G2 | model group | 10 | oral | N/A | once a day, from day 0 to day 7 |
| G3 | cyclosporine A group | 10 | oral | 60 | once a day, from day 0 to day 7 |
| G4 | **$I_A$-51** | 10 | oral | 10 | once a day, from day 0 to day 7 |
| G5 | **$I_A$-30** | 10 | oral | 10 | once a day, from day 0 to day 7 |
| G6 | **$I_D$-5** | 10 | oral | 10 | once a day, from day 0 to day 7 |
| G7 | **$I_A$-55** | 10 | oral | 10 | once a day, from day 0 to day 7 |

3. Experimental design

3.1 An appropriate amount of DSS powder was dissolved in high-pressure sterilized drinking water solvent and a 3% DSS solution was prepared.
3.2 Induction of Colitis: On day 3, 70 mice were randomly divided into 7 groups according to Table 7. From day 0 to day 4, mice in groups 2 to 7 drank 3% DSS aqueous solution for 5 days, followed by drank normal water ad libitum (from day 5 to before autopsy). The day of initial administration of DSS drinking water was counted as day 0. DSS aqueous solution was wrapped in opaque dark bags to ensure light avoidance. DSS aqueous solution was replaced every 2 days.
3.3. Dosage regimen: See Table 7.
3.4. Evaluation of enteritis:

(1) Weight, feces, and bleeding situation in mice

Changes in body weight, feces, and bleeding of all groups of mice were record daily. Three items were scored according to Table 8 below, and the scores were added together to form the daily disease activity index (DAI) score.

Table 8. Disease activity index (DAI) score

| Score | Weight | Fecal hardness | Fecal occult blood |
|---|---|---|---|
| 0 | -- | normal | negative |
| 1 | 1-5% | soft stool, but formed | negative |
| 2 | 6-10% | soft stool | positive |
| 3 | 11-18% | very soft, wet | blood stains visible in feces |
| 4 | >18% | watery diarrhea | rectal bleeding |

(2) The weight and length of the intestine

After blood collection, the animals were euthanized by inhaling excessive $CO_2$ and dislocation of cervical vertebra. Then the abdominal cavity was cut open and the colorectum was taken out. After removing the tissue around the colon, the longitudinal length from the ileocecal valve to the anus was measured. Afterwards, the colorectum was cut open and the contents of feces were scored. Finally, the intestinal contents were cleaned, and the entire colon was photographed and weighed.

(3) ELISA analysis

Commercially available ELISA analysis kits were used to analyze the colorectal inflammation factors TNF-$\alpha$ and IL-10 in each group.

3.5 Experimental observation

The health status of animals and their comprehensive response to DSS and drugs were observed every day. Any abnormal appearance or behavior other than those related to the development of the disease was recorded in detail in the Pharmalegacy Biological Experimental Observation Form.

4. Statistical analysis

The data was calculated as mean $\pm$ standard error. Statistical analysis was performed using Graphpad Prism and SPSS software. The specific data is presented in the form of charts. When $P<0.05$, it is considered statistically significant.

**Experimental results**

In the experimental observation, none of the animals showed any abnormal appearance or behavior.

This experiment induced acute colitis in C57BL/6 mice using oral DSS drinking water method according to the experimental protocol. Animals receiving DSS drinking water exhibited clinical symptoms of acute colitis, including weight loss, diarrhea, and bloody stools, as well as shortened intestinal length and increased intestinal weight observed at autopsy. The DAI score data of each group of animals are shown in Figure 3; the results of the weight and length of the colorectum in each group of animals are shown in Figure 4; the ELISA analysis results of the colorectal inflammatory factors TNF-$\alpha$ and IL-10 in each group of animals are shown in Figure 5.

According to the experimental results, the positive drug cyclosporine A showed significant efficacy in multiple indicators in this experiment, indicating the successful construction of the model. The compounds **$I_A$-51**, **$I_A$-30**, **$I_D$-5**, and **$I_A$-55** of the present invention can significantly reduce the DAI score, decrease the weight of the colorectum, and increase the length of the colorectum in model mice. At the same time, they can significantly reduce the levels of colorectal inflammatory factors TNF-$\alpha$ and IL-10 in each group of animals. The efficacy of each compound group of the present invention with a dosage of 10 mg/kg is stronger or not weaker than that of the positive control group with 60 mg/kg, indicating that the compounds of the present invention have good therapeutic efficacy against DSS induced inflammatory colitis in C57BL/6 mice.

In summary, the compounds **$I_A$-51**, **$I_A$-30**, **$I_D$-5**, and **$I_A$-55** of the present invention have good preventive and therapeutic effects on DSS induced inflammatory colitis in C57BL/6 mice, and can be developed as drugs for treating ulcerative colitis.

**Example 21**

The pharmacological effect of compounds prepared in some examples of the present invention on an acetic acid induced writhing pain model in ICR mice was investigated.
The acetic acid writhing pain model in mice is a classic pharmacological model for evaluating the treatment of drugs for visceral pain and inflammatory pain.

**Experimental process**

1. Experimental animals

Animal species and strains: ICR mice
Medical treatment history: None
Gender, age, and weight: Male, 8 weeks old, 20-25 g
Breeder/Supplier: Shanghai SLAC Laboratory Animal Co., Ltd
Adaptation period: no less than 7 days
Room: Regular level room
Room temperature: 19-26 °C
Relative humidity: 40-70%
Lighting cycle: fluorescent lighting, 12 hours of illumination (08:00-20:00) and 12 hours of no illumination
Animal feeding: 5 animals/cage divided by group
Food: Free intake (irradiated feed, Shanghai SLAC Laboratory Animal Co., Ltd, China)
Water: Free intake (Mol Ultrapure Water System filtered municipal tap water)

A total of 70 mice were introduced from Shanghai SLAC Laboratory Animal Co., Ltd. The animals were SPF grade and purchased at approximately 7 weeks old.

2. Grouping

Animals are randomly assigned to each treatment group according to the weight using the BioBook randomization function to achieve an approximate average weight for each group and reduce inter group bias.

Table 9. Animal grouping and dosage regimen

| Group | Group name | Advance administration time (h) | Number of animals | Administration method and route | Administration dosage (mg/kg) |
|---|---|---|---|---|---|
| G1 | model group | -- | 10 | single administration, oral | -- |
| G2 | aspirin | 1 | 10 | single administration, oral | 200 |
| G3 | **$I_A$-51** | 1 | 10 | single administration, oral | 30 |
| G4 | **$I_A$-30** | 1 | 10 | single administration, oral | 30 |
| G5 | **$I_D$-5** | 1 | 10 | single administration, oral | 30 |
| G6 | **$I_A$-55** | 1 | 10 | single administration, oral | 30 |

3. Experimental design

3.1 Within 12 hours before use, a glacial acetic acid solution with a mass volume fraction of 0.6% was prepared with 0.9% physiological saline.
3.2. Administration treatment: G1 group animals were orally administered 0.9% physiological saline; G2-G6 were specifically administered the compounds of the present invention referred to the specific dosage regimen in Table 9.
3.3. Modeling treatment: G1 group animals were administered 0.9% physiological saline by gavage, and after 1 hour, 0.6% glacial acetic acid was injected intraperitoneally to induce pain response (10 ml/kg). G2-G8 group animals

were injected intraperitoneally with 0.6% glacial acetic acid to induce pain response (10 ml/kg) after different groups were administered according to the regimen designed in Table 9.

3.4 Measurement: The weight of all mice was recorded before modeling treatment. A torsion test was recorded immediately after acetic acid treatment. The behavioral observation period lasted for 20 minutes. Writhes were counted by a single preson (one standard writhe includes abdominal muscle contraction, muscle extension, and hind limb extension). After testing, the animals were euthanized with $CO_2$.

3.5 Experimental observation: Animal health observation and general response to drug treatment were conducted daily. All abnormal health and behavioral behaviors were recorded.

4. Statistical analysis

The experimental data was calculated as mean ± standard error. Statistical analysis was performed using Graphpad Prism, SPSS, or Sigmaplot software. The specific data is presented in the form of graphs. $P<0.05$ is considered to have statistical differences.

**Experimental results**

The statistics of the writhing frequency for each experimental group are shown in Figure 6. It can be seen from Figure 6 that compared with the G1 model group, the compounds **$I_A$-51**, **$I_A$-30, $I_D$-5**, and **$I_A$-55** of the present invention can significantly reduce the writhing frequency in model mice, and their efficacy is basically equivalent to that of 200 mpk of aspirin, showing strong analgesic activity.

In summary, the compounds **$I_A$-51**, **$I_A$-30**, **$I_D$-5**, and **$I_A$-55** of the present invention have good analgesic effect and can be developed as drugs for pain treatment.

**Example 22**

The antagonistic activity of compounds prepared in some examples of the present invention against chemokine receptor CCR9 was investigated.

**Experimental process**

1. Test drugs and positive control drug

1.1 Test drugs

Name: Refer to Table 10 for detail
Storage method: Protected from light and stored under refrigeration
Dosage concentrations: 10 mM, 1 mM, 100 μM, 10 μM, 1 μM, 100 nM, and 10 nM, DMSO.
Working concentrations: 100 μM, 10 μM, 1 μM, 100 nM, 10 nM, 1 nM, and 100 pM, DMSO.

1.2 Positive control: Vercirnon (CCR9 antagonist)

Name: Vercirnon
Storage method: Avoid light and store in a sealed environment at -80 °C
Dosage concentrations: 10 mM, 1 mM, 100 μM, 10 μM, 1 μM, 100 nM, and 10 nM, DMSO.
Working concentrations: 100 μM, 10 μM, 1 μM, 100 nM, 10 nM, 1 nM, and 100 pM, DMSO.

1.3 CCL25 (CCR9 agonist)

Name: CCL25
Storage method: Protected from light and store in a sealed environment at -80 °C
Dosage concentration: 15 μM.

Working concentration: 150 nM.

2. Reagents and instruments:

2.1 Main reagents: DMEM culture medium (GIBCO); dimethyl sulfoxide (Sigma); FLUO-4, AM (Invitrogen)
2.2 Main instrument: Flexstation-3 (Molecular Devices)

3. Grouping and dosage setting:

3.1 Dosage setting basis:
The test concentration gradient of the compound and replicates were set according to the requirement of testing $IC_{50}$.
3.2 Dosage setting and group:
All test substances with 8 concentrations were set, with 3 replicates for each concentration.

4. Experimental principles and methods:

Experimental principle:

By establishing a cell line that cotransformed the receptor and G16, the receptor being activated can induce the activation of $G\alpha 16$ protein, thereby activating phospholipase C (PLC) to produce IP3 and DAG. IP3 can bind to the IP3 receptor on the intracellular endoplasmic reticulum and mitochondria, leading to the release of intracellular calcium. Therefore, measuring changes in intracellular calcium can serve as a method for detecting the activation status of target receptor. Fluo-4/AM is a calcium fluorescent probe indicator used to measure calcium ion, as a non-polar lipophilic compound, under the action of cell lipase after entering cells, the AM group dissociates and releases Fluo-4; since Fluo-4 is a polar molecule, it is not easy to pass through lipid bilayers, allowing it to remain in the cell for a long time. Ultimately, the level of G protein activation can be reflected by measuring the excited fluorescence intensity. If the screened compound can stimulate the target receptor, it can significantly increase the calcium flow reaction. On the contrary, if the screened compound can antagonize the target receptor, it can significantly reduce the calcium flow reaction.

Experimental steps:

1. Cells stably expressing the target receptor/$G\alpha 16$ were planted on a 96 well plate and cultured overnight.
2. The culture solution was removed from the well planted cells, then 40 μL/well of freshly prepared dye was added. The mixture was incubated at a constant temperature in a 37 °C incubator for 40 minutes.
3. The drug to be tested was diluted with calcium buffer and mixed well.
Antagonistic mode:
4. The dye was absorbed and discarded. After washed once with freshly prepared calcium buffer, 50 μL of calcium buffer that dissolved the drug to be tested was added for replacement.
5. The FlexStation II instrument was used for detection. Starting from the 15th second, 25 μL of calcium buffer dissolved with known agonist was automatically added by the instrument, and finally the fluorescence value at 525 nm was readed.
5. Data processing and statistical analysis:
Antagonistic mode

The following formula was used to calculate the cell response rate (%*Response*) for each sample under each concentration.

$$\%\mathrm{Re}sponse = \frac{L_{Sample} - L_{Blank}}{L_{Agonist} - L_{Blank}} \times 100\%$$

$L_{Sample}$ represents the detection signal value of the sample to be tested, $L_{Blank}$ represents the detection signal value completely inhibited by positive antagonist, $L_{Agonist}$ represents the detection signal value after stimulating the

DMSO blank group with positive agonist.

$IC_{50}$ value was calculated using *GraphPad Prism.*

6. Experimental results:

The test results are shown in Table 10. From the results, it can be seen that the compounds of the present invention have an antagonistic activity with $IC_{50}$ greater than 10 μM against the chemokine receptor CCR9, indicating that the compounds of the present invention do not have antagonistic activity against the chemokine receptor CCR9.

Similarly, the compounds of the present invention were also tested for their antagonistic activities against other chemokine receptors, such as CCR1 to CCR10. The test results showed that none of the compounds of the present invention exhibited antagonistic activity against chemokine receptors.

Therefore, the treatment of inflammatory bowel disease by the compound of the present invention is not achieved through antagonism of chemokine receptors, but through a novel mechanism of drug action, namely inhibition of the ion channel TRPA1.

Table 10. Antagonistic activity results of compounds of the present invention against chemokine receptor CCR9

| CCR9 | | |
|---|---|---|
| Compound | IC50(nM) | Inhibition rate (%)(100 μM) |
| **$I_A$-1** | >>100000 | 14 |
| **$I_A$-5** | >>100000 | 0 |
| **$I_A$-11** | >>100000 | 5 |
| **$I_A$-15** | >>100000 | 0 |
| **$I_A$-21** | >>100000 | 25 |
| **$I_A$-30** | >>100000 | 3 |
| **$I_A$-33** | >>100000 | 10 |
| **$I_A$-35** | >>100000 | 35 |
| **$I_A$-43** | >>100000 | 22 |
| **$I_A$-44** | >>100000 | 12 |
| **$I_A$-45** | >>100000 | 30 |
| **$I_A$-49** | >>100000 | 19 |
| **$I_A$-51** | >>100000 | 3 |
| **$I_A$-55** | >>100000 | 8 |
| **$I_B$** | >>100000 | 10 |
| **$I_D$-1** | >>100000 | 15 |
| **$I_D$-3** | >>100000 | 13 |
| **$I_D$-5** | >>100000 | 8 |
| **$I_D$-7** | >>100000 | 11 |
| **$I_F$** | >>100000 | 22 |
| **$I_J$-2** | >>100000 | 30 |
| **$I_Q$-1** | >>100000 | 22 |
| **$I_Q$-2** | >>100000 | 17 |
| **$I_R$** | >>100000 | 25 |

(continued)

| CCR9 | | |
|---|---|---|
| Compound | IC50(nM) | Inhibition rate (%)(100 μM) |
| Vercirnon | 3.380 | 100 |

All the documents mentioned herein are cited in the invention as reference, as if each of them is individually cited as reference. Further, it would be understood that, after reading the above teaching content of the present invention, the skilled in the art could make various changes or modifications to the invention, and these equivalents are still in the scope of the invention defined by the appended claims of the application.

## Claims

1. A compound of formula I, or an optical isomer thereof, or a racemate thereof, or a pharmaceutically acceptable salt thereof, or a prodrug thereof;

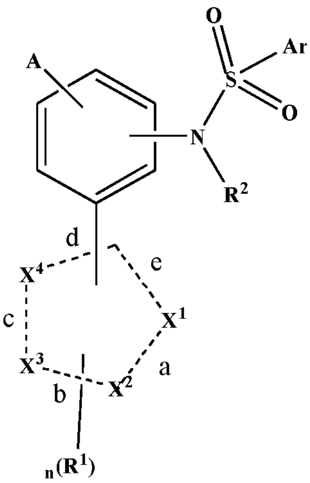

I

wherein,

Ar is substituted or unsubstituted C6-C12 aryl, substituted or unsubstituted 3-12 membered heteroaryl, 3-12 membered heterocycloalkyl ring fused C6-C12 aryl, substituted or unsubstituted C6-C12 aryl-substituted or unsubstituted C1-C8 alkyl-, or substituted or unsubstituted 3-12 membered heteroaryl-substituted or unsubstituted C1-C8 alkyl-;
$X^1$, $X^2$, $X^3$, and $X^4$ are each independently C, O, S, or N;
"------" labeled as a, b, c, d, and e are single bond or double bond;
$R^1$ is hydrogen, substituted or unsubstituted C1-C6 alkyl, substituted or unsubstituted C3-C7 cycloalkyl, or halogen;
$R^2$ is hydrogen, substituted or unsubstituted C1-C10 alkyl, or substituted or unsubstituted C3-C10 cycloalkyl;
A is

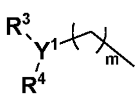, 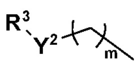 or, 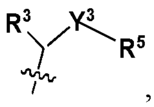, 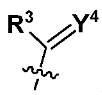, 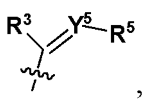,

substituted or unsubstituted C2-C6 ester group, substituted or unsubstituted C2-C6 carboxyl, substituted or unsubstituted C2-C6 amido, substituted or unsubstituted 3-8 membered heterocycloalkyl, or substituted or

unsubstituted $H_2N$-HN-C(O)-;
m is 0, 1, 2, or 3;
$Y^1$ is N;
$Y^2$ is O or S;
$Y^3$ is NH, O, or S;
$Y^4$ is O or S;
$Y^5$ is N;
$R^3$ and $R^4$ are each independently hydrogen, substituted or unsubstituted C1-C6 alkyl, substituted or unsubstituted C3-C8 cycloalkyl, substituted or unsubstituted C6-C12 aryl, substituted or unsubstituted C6-C12 aryl-substituted or unsubstituted C1-C3 alkyl-, substituted or unsubstituted C2-C6 acyl, or $R^3$ and $R^4$ together with the adjacent $Y^1$ form a substituted or unsubstituted 3-8 membered heterocycloalkyl;
$R^5$ is hydrogen, substituted or unsubstituted C1-C6 alkyl, hydroxyl, thiol, or substituted or unsubstituted C1-C6 alkoxy;
n is 0, 1, 2, 3, 4, or 5;
wherein, any one of the "substituted" refers to one or more (preferably 1, 2, 3, 4, 5, or 6) hydrogen atoms on the ring or group being substituted by substituents selected from the group consisting of C1-C8 alkyl, C3-C8 cycloalkyl, C1-C8 haloalkyl, C3-C8 halocycloalkyl, halogen, nitro, -CN, hydroxyl, thiol, amino, C1-C4 carboxyl, C2-C4 ester group, C2-C4 amido, C1-C8 alkoxy, C1-C8 alkylthio, C1-C8 haloalkoxy, C1-C8 haloalkylthio, C6-C12 aryl, 5-10 membered heteroaryl, and 5-10 membered heterocycloalkyl; and
the heterocycles in the heteroaryl, heterocycloalkyl ring, and heterocycloalkyl each independently contain 1-4 (preferably 1, 2, 3, or 4) heteroatoms selected from N, O, and S.

2. The compound according to claim 1, wherein Ar is substituted or unsubstituted C6-C10 aryl, substituted or unsubstituted 3-10 membered heteroaryl, 3-10 membered heterocycloalkyl ring fused C6-C10 aryl, substituted or unsubstituted C6-C10 aryl-substituted or unsubstituted C1-C6 alkyl-, or substituted or unsubstituted 3-10 membered heteroaryl-substituted or unsubstituted C1-C6 alkyl-.

3. The compound according to claim 1, wherein Ar is substituted or unsubstituted C6-C10 aryl, or substituted or unsubstituted 3-10 membered heteroaryl.

4. The compound according to claim 1, wherein $X^1$, $X^2$, $X^3$, $X^4$ and "-----" labeled as a, b, c, d, and e form a heteroaromatic ring, and the heterocycle in the heteroaromatic ring each independently contains 1-4 (preferably 1, 2, 3, or 4) heteroatoms selected from N, O, and S.

5. The compound according to claim 1, wherein $R^1$ is hydrogen, or substituted or unsubstituted C1-C4 alkyl; $R^2$ is hydrogen, or substituted or unsubstituted C1-C4 alkyl.

6. The compound according to claim 1, wherein A is

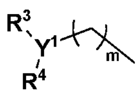 or 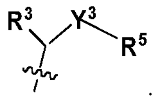 .

7. The compound according to claim 1, wherein the compound has the structure of formula Z:

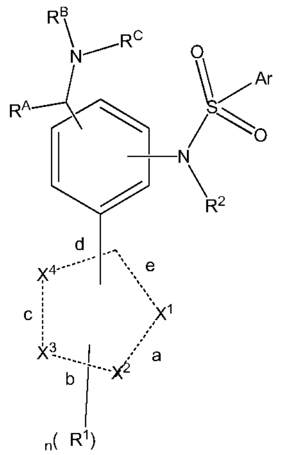

Z

wherein, $R^1$, $R^2$, $X^1$, $X^2$, $X^3$, $X^4$, Ar, a, b, c, d, e, and n are defined as claim 1;
$R^A$, $R^B$, and $R^C$ are each independently hydrogen, substituted or unsubstituted C1-C6 alkyl.

**8.** The compound according to claim 1, wherein the compound is selected from the group consisting of:

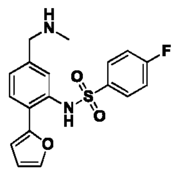 , 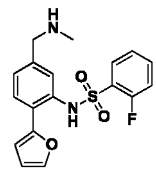 , 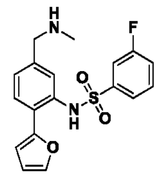 , 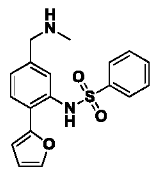 , 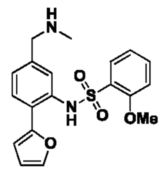 ,

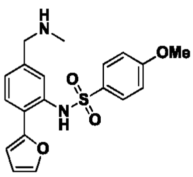 , 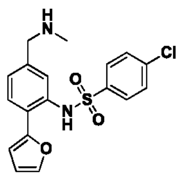 , 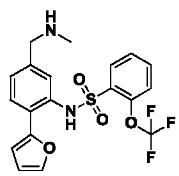 , 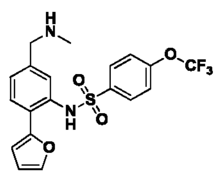 , 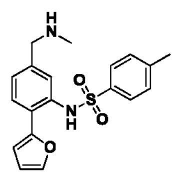 ,

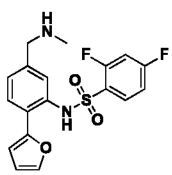 , 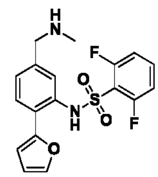 , 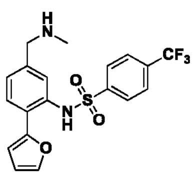 , 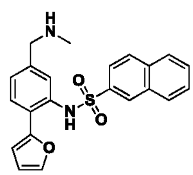 , 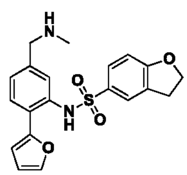 ,

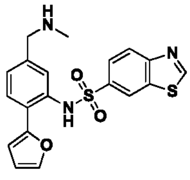 , 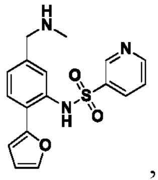 , 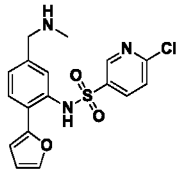 , 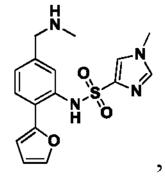 , 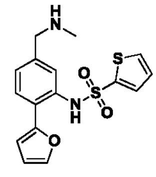 ,

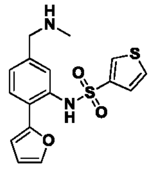 , 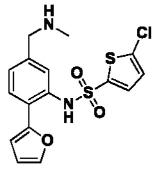 , 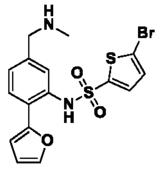 , 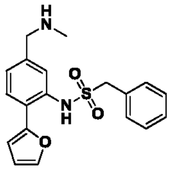 , 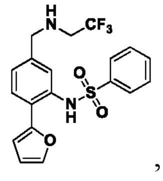 ,

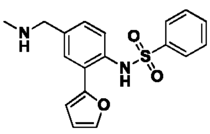 , 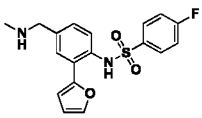 , 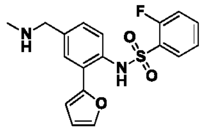 , 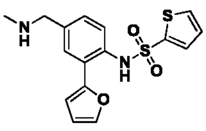 , 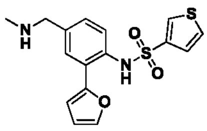 ,

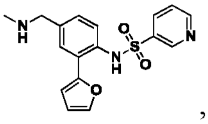 , 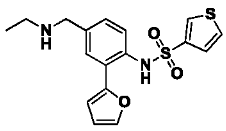 , 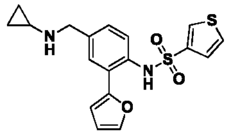 , 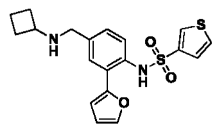 , 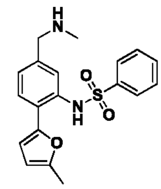 ,

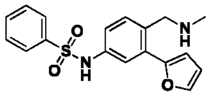 , 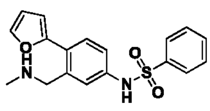 , 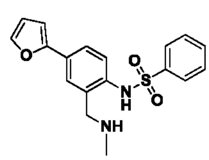 , 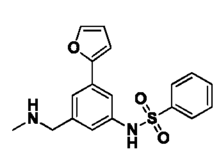 , 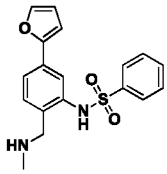 ,

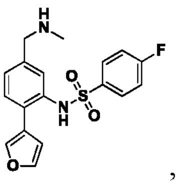 , 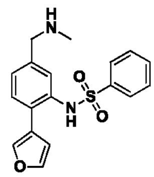 , 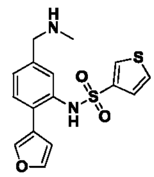 , 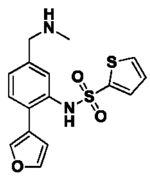 , 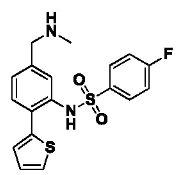 ,

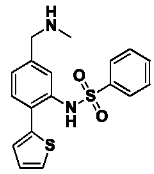 , 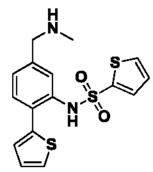 , 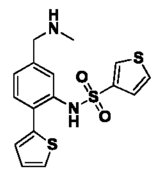 , 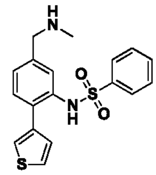 , 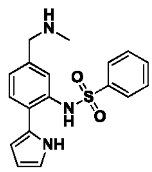 ,

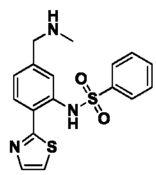 , 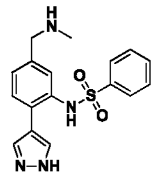 , 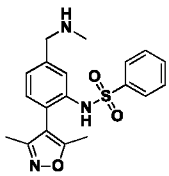 , 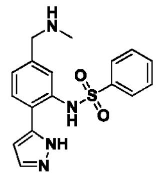 , 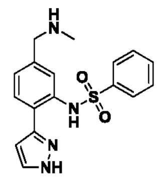 ,

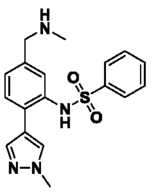 , 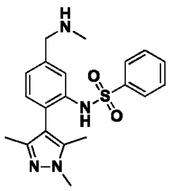 , 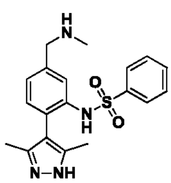 , 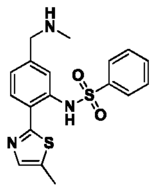 , 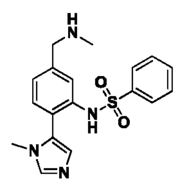 ,

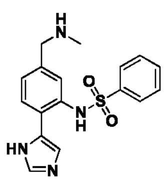 , 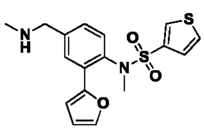 , 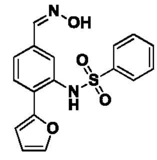 , 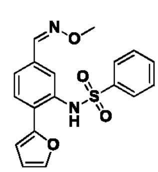 , 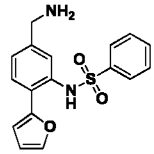 ,

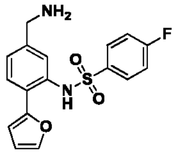, 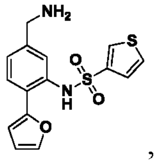, 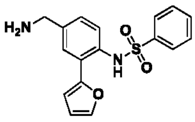, 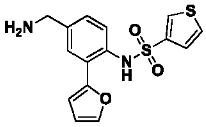, 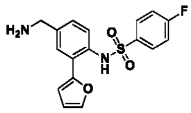,

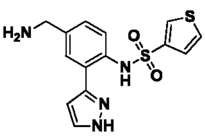, 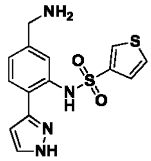, 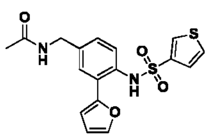, 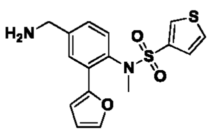, 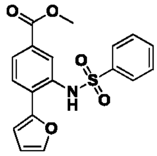,

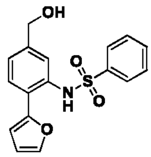, 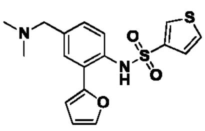, 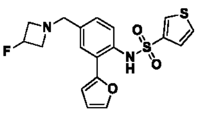, 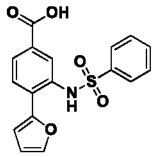, 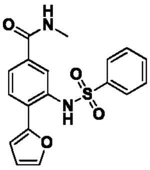,

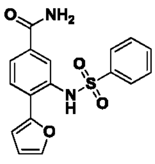, 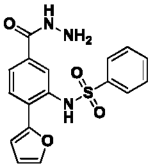, 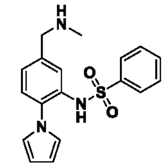, 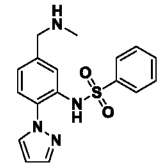, 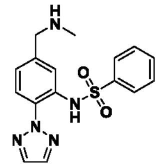,

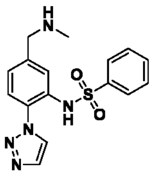, 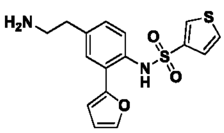, 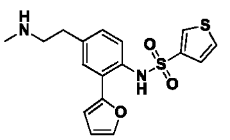, 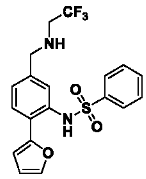, 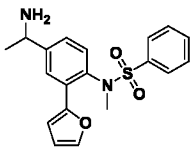

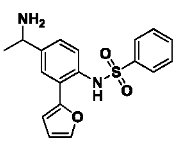, 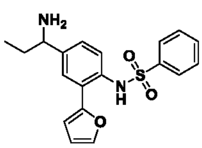, 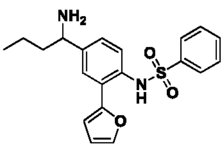, 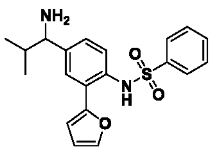, 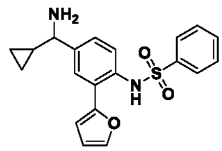,

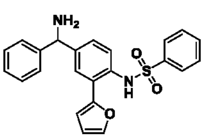, 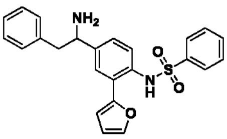, 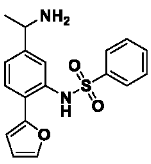, 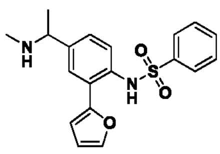, 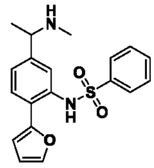,

and

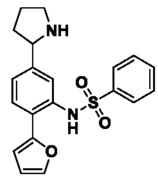

.

9. A pharmaceutical composition comprising a compound of formula I according to claim 1, or an optical isomer thereof, or a racemate thereof, or a pharmaceutically acceptable salt thereof, or a prodrug thereof; and pharmaceutically acceptable carriers.

10. A method for preparing a compound of formula I according to claim 1, or an optical isomer thereof, or a racemate thereof, or a pharmaceutically acceptable salt thereof, or a prodrug thereof, wherein the method comprises one of the method 1 to method 4:

method 1:

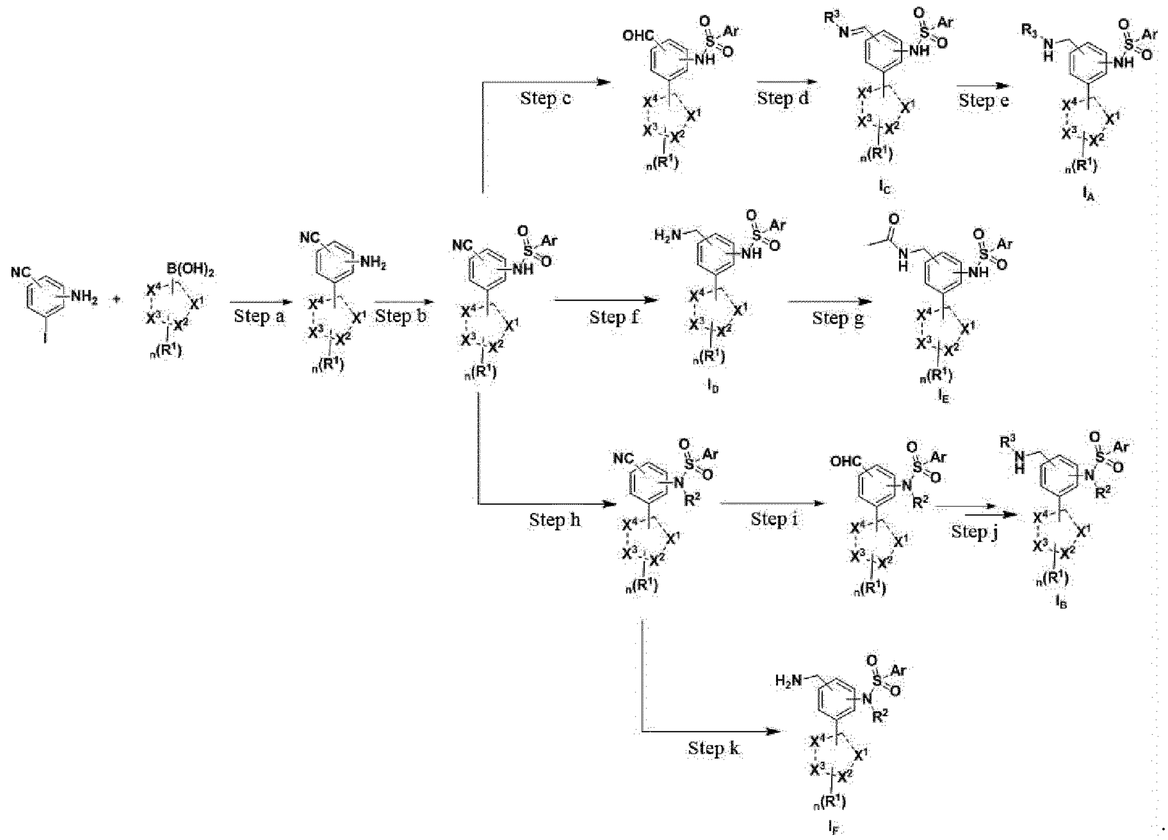


;

or

method 2:

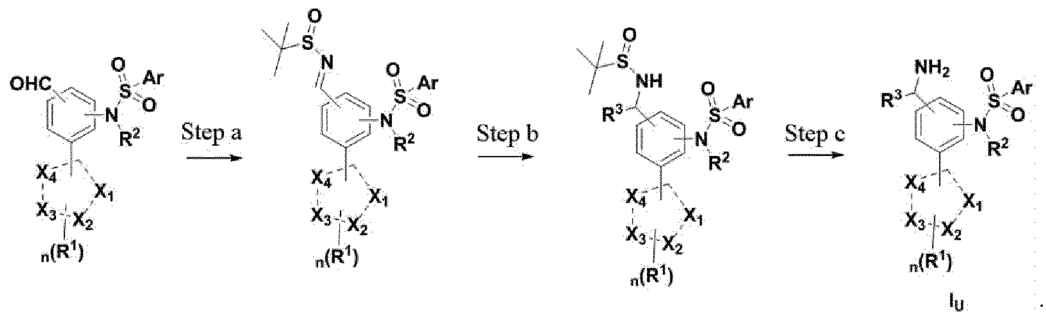


$I_U$ ;

or
method 3:

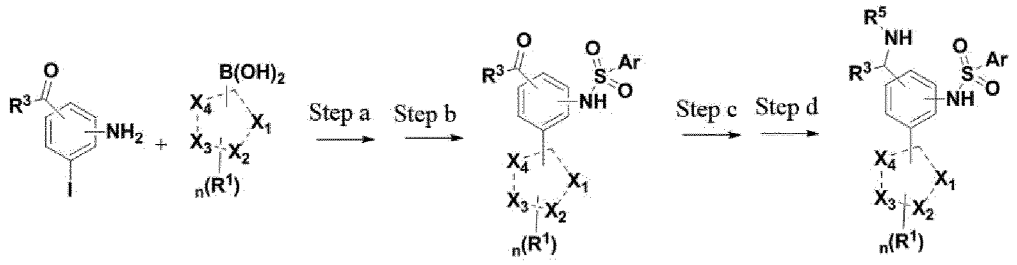


$I_V$ ;

or
method 4:

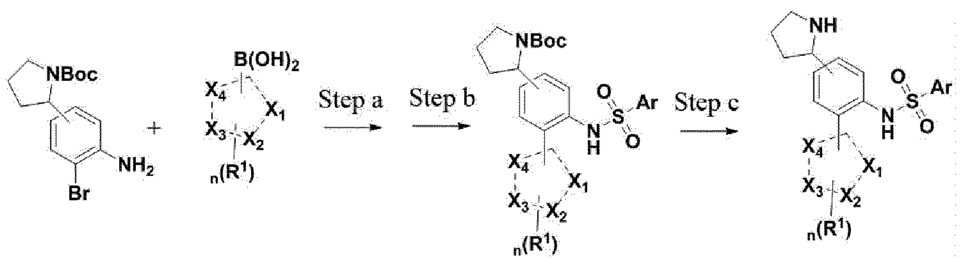


$I_W$ ;

wherein, $X^1$, $X^2$, $X^3$, $X^4$, $R^1$, $R^2$, n, Ar, and $R^3$ are defined as claim 1.

**11.** A use of a compound of formula I according to claim 1, or an optical isomer thereof, or a racemate thereof, or a pharmaceutically acceptable salt thereof, or a prodrug thereof, or a pharmaceutical composition according to claim 9 in (a) preparing an inhibitor of transient receptor potential channel protein TRPA1; and/or (b) preparing drugs for preventing and/or treating disease related to transient receptor potential channel protein TRPA1.

**12.** The use according to claim 11, wherein the disease related to transient receptor potential channel protein TRPA1 is selected from the group consisting of inflammatory bowel disease, irritable bowel syndrome, pain, inflammation, and combinations thereof.

**13.** The use according to claim 12, wherein the inflammatory bowel disease includes Crohn's disease and/or ulcerative colitis.

**14.** The use according to claim 12, wherein the pain includes visceral pain, acute inflammatory pain, chronic inflammatory pain, neurogenic pain, fibromyalgia, headache, neuralgia, or pain caused by cancer.

**15.** A method for *in vitro* non-therapeutically and non-diagnostically inhibiting the activity of transient receptor potential

channel protein, comprising the steps of contacting the transient receptor potential channel protein or cells expressing the protein with a compound of formula I according to claim 1, or an optical isomer thereof, or a racemate thereof, or a pharmaceutically acceptable salt thereof, or a prodrug thereof, thereby inhibiting the activity of transient receptor potential channel proteins.

**16.** A method for inhibiting transient receptor potential channel protein, or preventing and/or treating disease related to transient receptor potential channel protein TRPA1, comprising the steps of administering to a subject in need thereof a compound of formula I according to claim 1, or an optical isomer thereof, or a racemate thereof, or a pharmaceutically acceptable salt thereof, or a prodrug thereof, or a pharmaceutical composition according to claim 9.

**17.** A compound shown in one of the following formulas II-1 to II-6:

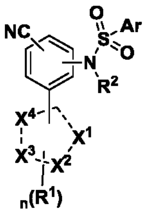
II-1

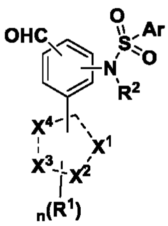
II-2

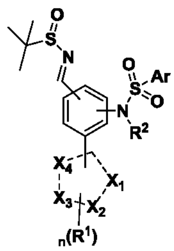
II-3

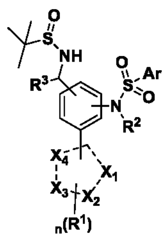
II-4

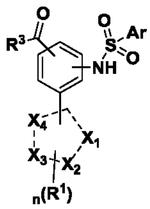
II-5

, or

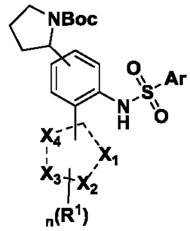
II-6

wherein,

Ar is substituted or unsubstituted C6-C12 aryl, substituted or unsubstituted 3-12 membered heteroaryl, 3-12 membered heterocycloalkyl ring fused C6-C12 aryl, substituted or unsubstituted C6-C12 aryl-substituted or unsubstituted C1-C8 alkyl-, or substituted or unsubstituted 3-12 membered heteroaryl-substituted or unsubstituted C1-C8 alkyl-;

$X^1$, $X^2$, $X^3$, and $X^4$ are each independently C, O, S, or N;

"------" labeled as a, b, c, d, and e are single bond or double bond;

$R^1$ is hydrogen, substituted or unsubstituted C1-C6 alkyl, substituted or unsubstituted C3-C7 cycloalkyl, or halogen;

$R^2$ is hydrogen, substituted or unsubstituted C1-C10 alkyl, or substituted or unsubstituted C3-C10 cycloalkyl;

n is 0, 1, 2, 3, 4, or 5;

wherein, any one of "substituted" refers to one or more (preferably 1, 2, 3, 4, 5, or 6) hydrogen atoms on the ring or group being substituted by substituents selected from the group consisting of C1-C8 alkyl, C3-C8 cycloalkyl, C1-C8 haloalkyl, C3-C8 halocycloalkyl, halogen, nitro, -CN, hydroxyl, thiol, amino, C1-C4 carboxyl, C2-C4 ester group, C2-C4 amido, C1-C8 alkoxy, C1-C8 alkylthio, C1-C8 haloalkoxy, C1-C8 haloalkylthio, C6-C12 aryl, 5-10 membered heteroaryl, and 5-10 membered heterocycloalkyl; and

the heterocycles in the heteroaryl, heterocycloalkyl ring, and heterocycloalkyl each independently contain 1-4 (preferably 1, 2, 3, or 4) heteroatoms selected from N, O, and S.

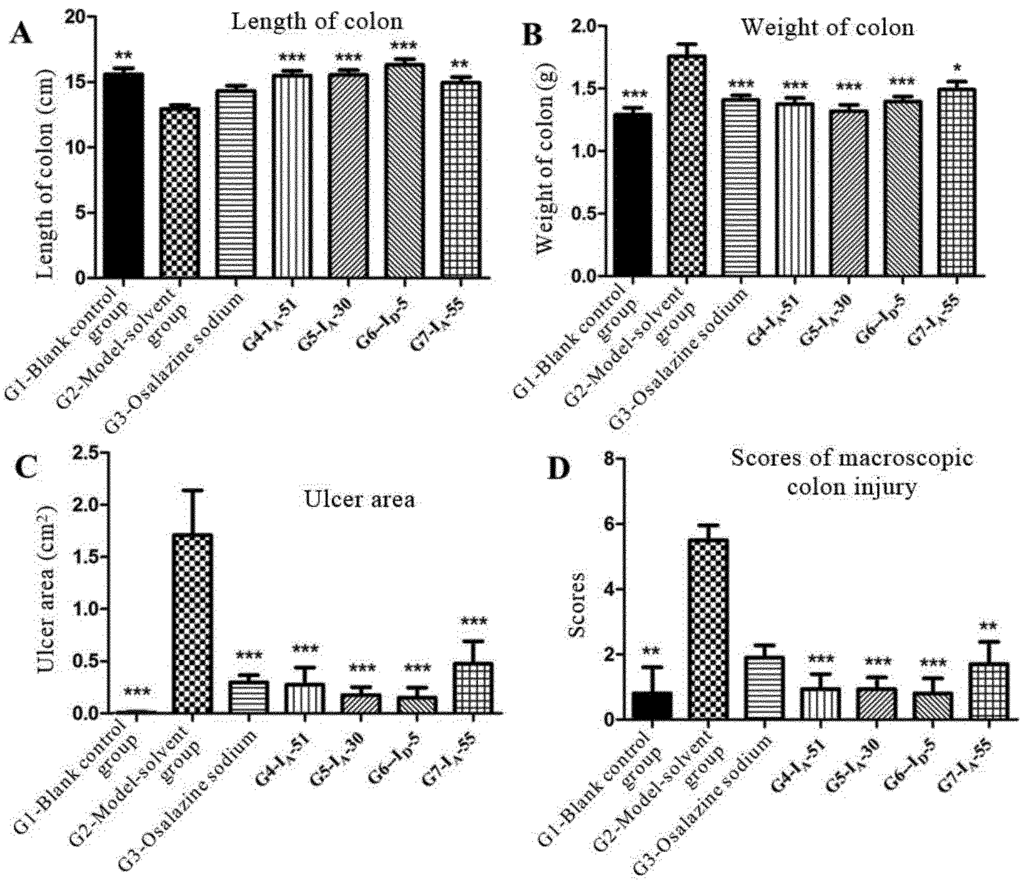


Figure 1

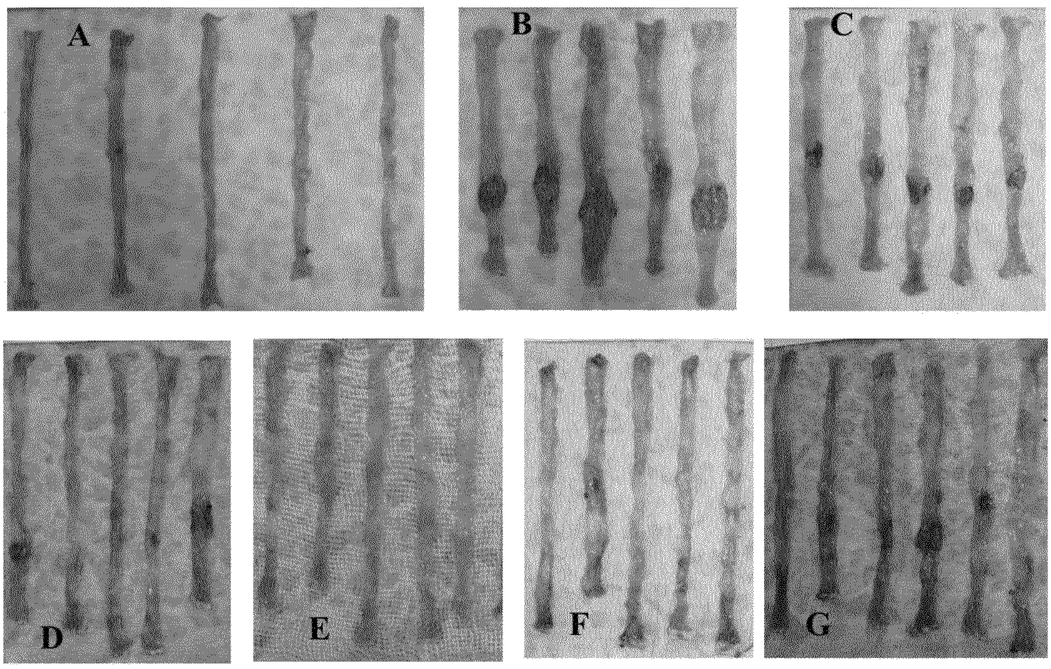


Figure 2

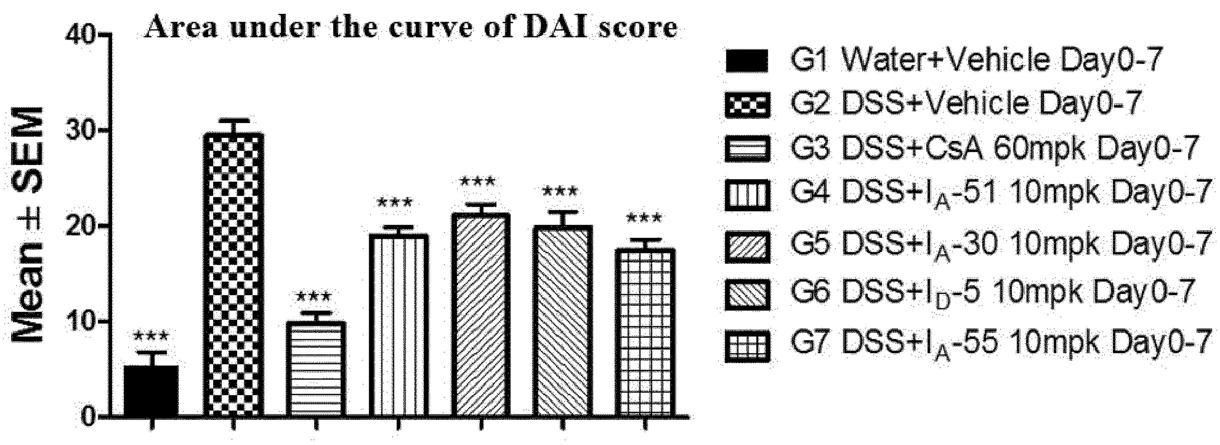


Figure 3

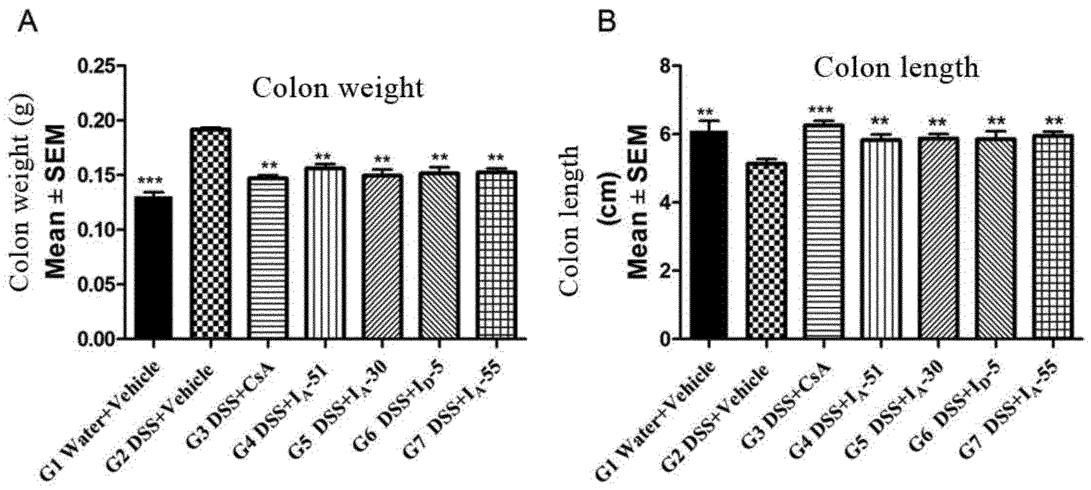


Figure 4

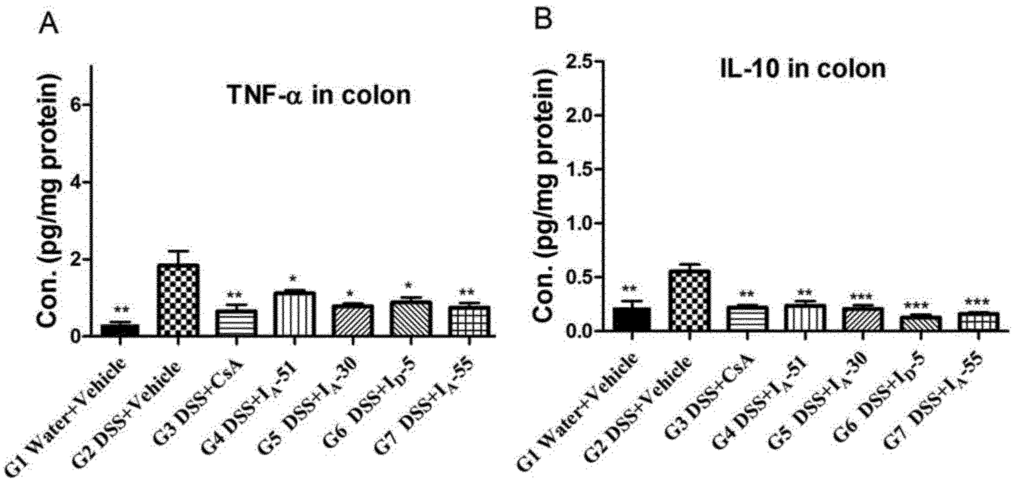


Figure 5

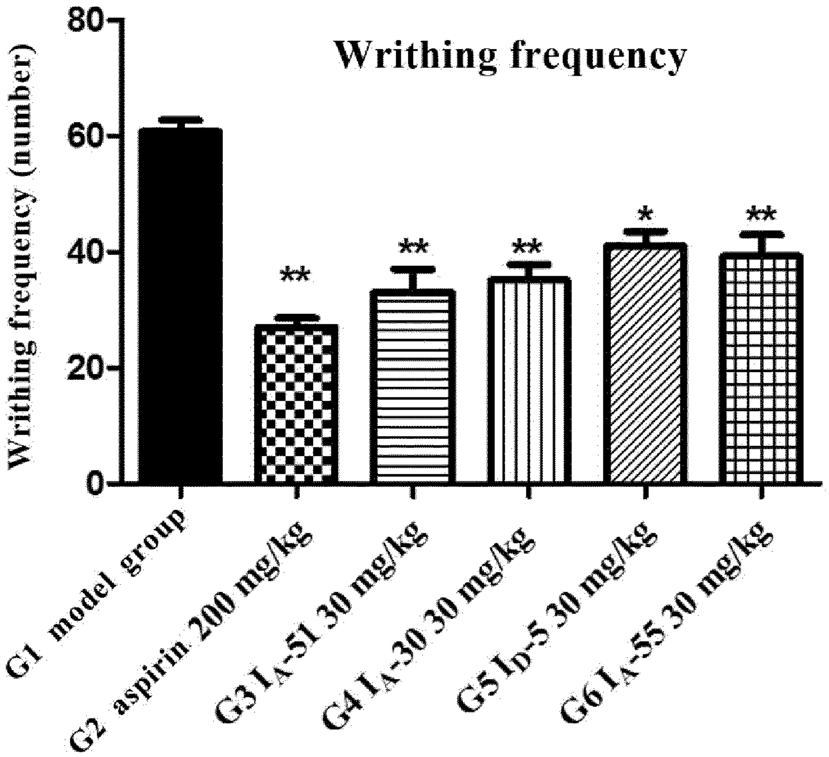
Writhing frequency
Writhing frequency (number)
80
60
40
20
0
**
**
**
*
**
G1 model group
G2 aspirin 200 mg/kg
G3 IA-51 30 mg/kg
G4 IA-30 30 mg/kg
G5 ID-5 30 mg/kg
G6 IA-55 30 mg/kg

Figure 6

**INTERNATIONAL SEARCH REPORT**

International application No.
**PCT/CN2022/098183**

**A. CLASSIFICATION OF SUBJECT MATTER**

C07D 307/52(2006.01)i; C07D 307/79(2006.01)i; C07D 417/12(2006.01)i; C07D 405/12(2006.01)i; C07D 409/12(2006.01)i; C07D 333/20(2006.01)i; C07D 333/34(2006.01)i; C07D 207/335(2006.01)i; C07D 277/28(2006.01)i; C07D 231/12(2006.01)i; C07D 261/08(2006.01)i; C07D 233/64(2006.01)i; C07D 307/54(2006.01)i; C07D 409/14(2006.01)i; C07D 249/06(2006.01)i; A61P 29/00(2006.01)i; A61P 1/00(2006.01)i; A61P 1/04(2006.01)i; A61P 25/00(2006.01)i; A61K 31/341(2006.01)i; A61K 31/428(2006.01)i; A61K 31/443(2006.01)i; A61K 31/4178(2006.01)i; A61K 31/381(2006.01)i; A61K 31/40(2006.01)i; A61K 31/426(2006.01)i; A61K 31/415(2006.01)i; A61K 31/4164(2006.01)i; A61K 31/402(2006.01)i; A61K 31/4192(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; DWPI; CJFD; CNKI; CNTXT; USTXT; WOTXT; EPTXT; STN-REGISTRY; STN-CAPLUS; PATENTICS; 超星读秀, DUXIU; ISI-Web of Science: 磺酰胺, 苯, 呋喃, 噻吩, 吡咯, 噻唑, 吡唑, 异恶唑, 瞬时受体电位通道蛋白, TRPA1, ANKTM1, 炎症性肠病, IBD, 溃疡性结肠炎, 克罗恩氏病, 肠易激综合征, 炎症, 疼痛, +sulfonamid+, +phenyl+, +furan+, +thiophen+, +pyrrol+, +thiazol+, +pyrazol+, +isoxazol+, transient receptor potential ankyrin, inflammatory bowel disease, ulcerative colitis, Crohn's disease, irritable bowel syndrome, inflammat+, +ache, pain, sore

**C. DOCUMENTS CONSIDERED TO BE RELEVANT**

| Category* | Citation of document, with indication, where appropriate, of the relevant passages | Relevant to claim No. |
|---|---|---|
| X | CN 101511800 A (CHEMOCENTRYX, INC.) 19 August 2009 (2009-08-19)<br>description, paragraphs [00531] and [00631]-[00680], and claims 1 and 18 | 1-9, 11-15, 17 |
| X | WO 2020097408 A1 (NIMBUS ARTEMIS INC) 14 May 2020 (2020-05-14)<br>description, pp. 61 and 72, and embodiments 330, 331, 334, and 335 | 1-6, 9 |
| X | WANG, Xiaoyu et al. "Ruthenium-catalyzed 1, 2, 3-triazole Directed Intermolecular C–H Amidation of Arenes with Sulfonyl Azides"<br>*RSC Advances,* Vol. 6, No. 73, 15 July 2016 (2016-07-15),<br>ISSN: 2046-2069,<br>p. 68931, table 2 | 1-6, 17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 July 2022** | **05 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No. **PCT/CN2022/098183**

**C. DOCUMENTS CONSIDERED TO BE RELEVANT**

| Category* | Citation of document, with indication, where appropriate, of the relevant passages | Relevant to claim No. |
|---|---|---|
| X | BOOMINATHAN, S. S. K. et al. "A Palladium- and Copper-Catalyzed Synthesis of Dihydro[1, 2-b]indenoindole-9-ol and Benzofuro[3, 2-b]indolines: Metal-Controlled Intramolecular C-C and C-O Bond-Forming Reactions" *CHEMISTRY-A EUROPEAN JOURNAL,* Vol. 21, No. 47, 07 October 2015 (2015-10-07), ISSN: 1521-3765, Scheme 1 in support information, and Table S1 | 1-6, 17 |
| X | MONTANGE, G. P. et al. "Observed Bromodomain Flexibility Reveals Histone Peptide- and Small Molecule Ligand-compatible Forms of ATAD2A" *BIOCHEMICAL JOURNAL,* Vol. vol. 466, No. 2, 01 March 2015 (2015-03-01), ISSN: 1470-8728, p. 6, paragraphs 2 and 3 | 1-6 |
| X | WO 2010126002 A1 (SHIONOGI & CO.) 04 November 2010 (2010-11-04) description, paragraph [0104] | 1-6 |
| X | WO 2008038955 A1 (CHONG KUN DANG PHARM CORP) 03 April 2008 (2008-04-03) description, table 4 | 1-6 |
| X | WO 2014163162 A1 (TAKEDA PHARMACEUTICAL COMPANY LIMITED) 09 October 2014 (2014-10-09) description, tables 1-1 | 17 |
| X | KR 20150144121 A (SFC CO., LTD.) 24 December 2015 (2015-12-24) description, paragraph [0413] | 17 |
| A | US 2021130380 A1 (NOCION THERAPEUTICS INC) 06 May 2021 (2021-05-06) entire document | 1-15, 17 |

Form PCT/ISA/210 (second sheet) (January 2015)

**INTERNATIONAL SEARCH REPORT**

International application No.
**PCT/CN2022/098183**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

[1] Claim 16 relates to a method for inhibiting transient receptor potential ankyrin or preventing and/or treating diseases associated with transient receptor potential ankyrin 1 (TRPA1), belonging to the scope of a method for treating a human body, i.e., belonging to the cases listed in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/098183**

| Patent document cited in search report | Publication date (day/month/year) | Patent family member(s) | Publication date (day/month/year) |
|---|---|---|---|
| CN 101511800 A | 19 August 2009 | CA 2657776 A1 | 24 January 2008 |
| | | PH 12009500093 A | 24 January 2008 |
| | | WO 2008010934 A2 | 24 January 2008 |
| | | US 2008039465 A1 | 14 February 2008 |
| | | WO 2008010934 A3 | 27 March 2008 |
| | | MX 2009000395 A1 | 31 January 2009 |
| | | AU 2007275873 A1 | 12 February 2009 |
| | | SG 149223 A1 | 27 February 2009 |
| | | NO 20090662 A | 26 March 2009 |
| | | EP 2046762 A2 | 15 April 2009 |
| | | KR 20090053779 A | 27 May 2009 |
| | | IN 200900221 P4 | 05 June 2009 |
| | | ZA 200900281 A | 24 February 2010 |
| | | US 7718683 B2 | 18 May 2010 |
| | | NZ 574759 A | 29 October 2010 |
| | | MX 283382 B | 26 January 2011 |
| | | EP 2046762 B1 | 16 February 2011 |
| | | DE 602007012552 E | 31 March 2011 |
| | | ES 2360741 T3 | 08 June 2011 |
| | | SG 149223 B | 31 August 2011 |
| | | VN 10009516 B | 27 September 2011 |
| | | EA 015710 B1 | 31 October 2011 |
| | | AU 2007275873 B2 | 14 June 2012 |
| | | US 2012165303 A1 | 28 June 2012 |
| | | IL 196409 A | 31 October 2012 |
| | | CN 101511800 B | 27 February 2013 |
| | | BR PI0714407 A2 | 05 March 2013 |
| | | US 8481579 B2 | 09 July 2013 |
| | | JP 5266219 B2 | 21 August 2013 |
| | | CA 2657776 C | 27 August 2013 |
| | | HK 1135091 A1 | 22 November 2013 |
| | | KR 101433392 B1 | 29 August 2014 |
| | | PH 12009500093 B1 | 27 July 2015 |
| | | IN 275729 B | 23 September 2016 |
| | | NO 341949 B1 | 26 February 2018 |
| | | JP 2010504908 A | 18 February 2010 |
| WO 2008038955 A1 | 03 April 2008 | KR 20080028763 A | 01 April 2008 |
| | | EP 2066632 A1 | 10 June 2009 |
| | | US 2009275575 A1 | 05 November 2009 |
| | | KR 932093 B1 | 16 December 2009 |
| | | EP 2066632 A4 | 27 April 2011 |
| | | US 8053439 B2 | 08 November 2011 |
| | | EP 2066632 B1 | 04 September 2013 |
| | | ES 2437079 T3 | 08 January 2014 |
| WO 2014163162 A1 | 09 October 2014 | EP 2982670 A1 | 10 February 2016 |
| | | US 2016052897 A1 | 25 February 2016 |
| | | EP 2982670 A4 | 30 November 2016 |
| | | US 9624184 B2 | 18 April 2017 |
| | | JP 6272833 B2 | 31 January 2018 |
| | | EP 2982670 B1 | 07 November 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010075353 A